# EUROPEAN PATENT APPLICATION

(11) **EP 4 495 111 A1**
(43) Date of publication of application: **22.01.2025**
(21) Application number: 23771112.2
(22) Date of filing: 16.03.2023
(51) Int. Cl.: C07D 401/14, A61K 31/497, A61P 35/00, C07D 487/04

(54) **NOVEL COMPOUND AND USE THEREOF FOR INHIBITING CHECKPOINT KINASE 2**

(30) Priority: 16.03.2022 KR 20220033007
(71) Applicant: Arum Therapeutics Inc, Seoul 07793 (KR)
(72) Inventor: KANG, Young Woo, Seoul 07803 (KR); BAIK, Tae Gon, Incheon 21111 (KR); LEE, Ha Young, Seoul 03453 (KR)
(74) Representative: HGF
(86) International application number: PCT/KR2023/003519
(87) International publication number: WO 2023/177233

(57) **Abstract**

The present invention provides a novel compound, a use thereof for inhibiting checkpoint kinase 2 (Chk2), and a use thereof for preventing or treating cancer. The compound of the present invention has high Chk2 selectivity, and thus it can overcome resistance and toxicity to a PARP inhibitor, and can be effectively used for the prevention or treatment of cancer.

## Description

### Technical Field

The present invention relates to a novel compound, a preparation method thereof, a pharmaceutical composition, a use thereof for inhibiting checkpoint kinase 2, and a method for preventing or treating a disease mediated by checkpoint kinase 2 activation, for example cancer, using the same.

### Background Art

In order to maintain genomic integrity, cells activate checkpoint pathways to stop the cell cycle and then repair DNA damage, and when the damaged portion is not properly repaired, apoptosis is usually induced. If genotoxic damage is not adequately dealt with, the accumulation of modified genes is caused, which may directly cause the development of cancer. Depending on the DNA damage caused, checkpoint kinase 1 or 2 (Chk1 or Chk2) is phosphorylated by ATM (ataxia telangiectasia mutated) or ATR (ATM/Rad3 related). In general, Chk1 is activated in the presence of DNA single-strand damage, and Chk2 is activated in the presence of double-strand damage. Upon phosphorylation by ATM, activated Chk2 kinase acts as a signal transducer and phosphorylates various substrates, including Cdc25 phosphatase, p53, PML, E2F-1, and BRCA1, involved in functions such as cell cycle arrest, DNA repair, and apoptosis after DNA damage.

Chk2 participates in DNA repair by regulating the function of BRCA1 (breast cancer 1), a tumor suppressor, through phosphorylation. It is known that activated BRCA1 coordinates the function of the HR (homologous recombination) DNA repair pathway, while it inhibits the NHEJ (non-homologous end joining) pathway (Nature, 2000, vol.404, pp.201-204; Cancer research, 2006, vol.66, pp.1401-1408). In order to facilitate DNA repair through HR, BRCA1 forms a complex with BRCA2 and directly interacts with Rad51 recombinase, a key protein in the HR DNA repair pathway. Regulation of BRCA1 by this Chk2 supports the transition from NHEJ to HR pathway. In addition, BRCA1 is related to Msh2, a DNA mismatch repair protein, and Chk2 interacts with Msh2, which means that Chk2 and BRCA1 are also involved in DNA mismatch repair (Mol. Cell. Bio, 2004, vol.24, no.2, pp.708-718).

On the other hand, poly(ADP-ribose) polymerase 1(PARP1) is a DNA damage sensor, and when activated, participates in DNA repair by recruiting downstream proteins by poly-ADP-ribosylation (PARylation). A recent study reported co-immunoprecipitation of PARP1 and Chk2, increased interaction between the two proteins after oxidative DNA damage, and decreased overall poly-ADP-ribosylation by Chk2 depletion (Oncogene, 2019, vol.38, no.8, pp.1166-1182). In addition, through the interaction of the Chk2 SCD domain and the PARP1 BRCT domain, Chk2 stimulates the poly-ADP-ribosylation activity of PARP1 through Chk2-dependent phosphorylation. In other words, Chk2 has a pleiotropic effect on PARP1 during oxidative stress in order to promote DNA repair and cell survival.

Olaparib, developed as a BRCA mutation-based synthetic lethality strategy, is a PARP1 inhibitor that has achieved clinical success in groups of patients with breast cancer, ovarian cancer, prostate cancer, and pancreatic cancer. However, despite this success, as resistance and toxicity issues have been raised due to the use of PARP inhibitors, various attempts have been made to overcome these issues. In particular, as it is known that Chk2 interacts with target proteins associated with resistance to a PARP inhibitor, Chk2 is recognized as a major target for overcoming resistance to a PARP inhibitor.

Therefore, there is an urgent need in the art for the development of Chk2 inhibitors with high selectivity.

### Detailed Description of Invention

### Technical Problem

An object of the present invention is to provide a novel compound having checkpoint kinase 2 inhibitory activity.

Another object of the present invention is to provide a pharmaceutical composition for preventing or treating cancer, comprising a novel compound having checkpoint kinase 2 inhibitory activity.

Another object of the present invention is to provide a method of inhibiting checkpoint kinase 2 using a novel compound having checkpoint kinase 2 inhibitory activity.

Another object of the present invention is to provide a method for preventing or treating cancer using a novel compound having checkpoint kinase 2 inhibitory activity.

### Solution to Problem

### Definition

It is to be understood that any feature, integer, property, compound, chemical moiety or group described in connection with a particular aspect, embodiment or example of the invention can be applied to any another aspect, embodiment or example described herein, where appropriate. That is, all combinations of the various elements disclosed herein, except for mutually exclusive combinations, fall within the scope of the present application. In addition, the scope of the present application is not to be construed as being limited by the specific embodiments set forth below.

Throughout the description and claims of the present specification, the words "comprise" and "contain" and variations thereof refer to "including but not limited to", which means that other structures, substituents, additives, components, integers or steps are not excluded. Throughout the description and claims of the present specification, a singular form includes a plural form unless the context requires otherwise. In particular, where the indefinite article is used, it is to be understood that the present specification contemplates the plural form as well as the singular form, unless the context requires otherwise.

As used herein, when referring to a numerical value or a range of numerical values, the term "about" means that the numerical value or range of numerical values referred to is within the range of experimental deviation (or statistical error) and, in some cases, can mean ±1 % to ±20%, ±5% to ±15%, or ±10% of the the numerical value or range of numerical values referred to.

As used herein, the following terms have the following meanings unless otherwise specified.

The term "alkyl" refers to a fully saturated branched or unbranched (or straight chain or linear) hydrocarbon. The alkyl may be a substituted or unsubstituted alkyl group. As used herein, the term "alkyl" may refer to, for example, C₁-C₈ alkyl, C₁-C₆ alkyl, C₁-C₅ alkyl, C₁-C₃ alkyl, and the like. The C₁-C₆ alkyl may be a C₁ to C₅, C₁ to C₄, C₁ to C₃, or C₁ to C₂ alkyl group. Non-limiting examples of said C₁-C₆ alkyl may be methyl, ethyl, n-propyl, isopropyl, n-butyl, isobutyl, sec-butyl, n-pentyl, isopentyl, neopentyl, iso-amyl, or n-hexyl.

The term "cycloalkyl" or "cyclic ring" refers to a saturated or partially unsaturated, non-aromatic monocyclic or bicyclic hydrocarbon group. A bicyclic cycloalkyl may be a fused, bridged and/or spirocyclic structure. A cyclic ring group may contain, for example, 3 to 10, 3 to 8, 3 to 6, or 3 to 5 carbon atoms. A monocyclic ring group may be, for example, cyclopentyl, cyclopentenyl, cyclohexyl, or cyclohexenyl, or the like. A bicyclic ring group include, for example, bornyl, decahydronaphthyl, bicyclo[2.1.1]hexyl, bicyclo[2.2.1]heptyl, bicyclo[2.2.1]heptenyl, or bicyclo[2.2.2]octylyl, or the like.

The term "halogen" atom refers to an atom belonging to group 17 of the periodic table. The halogen atom includes fluorine (F), chlorine (Cl), bromine (Br), iodine (I), and the like. The term "halogen" may be used interchangeably with the term "halo," which means a monovalent functional group composed of halogen.

The term "hydroxy" refers to a -OH functional group (hydroxyl group).

The term "amino" refers to -NH₂ in which hydrogen is bound to a nitrogen atom.

The term "nitro" refers to -NO₂.

The term "cyano" is -CN, and refers to a functional group consisting of a triple bond between a carbon atom and a nitrogen atom.

The term "oxo" refers to =O, and "substituted with oxo" means that the carbon atom has a =O substituent in the form of -C(=O)-.

Unless otherwise stated, the term "alkoxy" refers to a substituent in which a substituted or unsubstituted straight chain or branched chain alkyl moiety is linked to another chemical structure by oxygen. As used herein, the term "alkoxy" may refer to, for example, C₁-C₈ alkoxy, C₁-C₆ alkoxy, C₁-C₅ alkoxy, C₁-C₃ alkoxy, and the like. The alkoxy may include all possible isomers thereof such as, for example, methoxy, ethoxy, propoxy, and butoxy, or isopropoxy, isobutoxy, and t-butoxy, but is not limited thereto.

The term "alkenyl" refers to a branched or unbranched hydrocarbon having at least one carbon-carbon double bond. As used herein, the term "alkenyl" may include, for example, C₂-C₈ alkenyl, C₂-C₆ alkenyl, C₂-C₅ alkenyl, C₂-C₃ alkenyl, and the like. Non-limiting examples of the alkenyl may include ethenyl, 3-propenyl, 1-propenyl, isopropenyl, buten-4-yl, and the like.

The term "aryl" refers to an aromatic hydrocarbon ring group and also includes a group in which a ring is fused to one or more carbon rings. For example, the aryl may be a C₆ to C₁₀ aryl group. The aryl may be phenyl, naphthyl, or tetrahydronaphthyl.

The term "heteroaryl" refers to a monocyclic or bicyclic aromatic group that contains one or more heteroatoms (i.e., one or more ring heteroatoms selected from oxygen, nitrogen and sulfur) and the remaining ring atoms being carbon. The heteroaryl group may contain, for example, 1 to 5, 1 to 3, 1 or 2 heteroatoms and may contain 5 to 10 ring elements. The "heteroaryl" may be, for example, pyrrolyl, furanyl, thiophenyl, pyrazolyl, imidazolyl, isoxazolyl, oxazolyl, thiazolyl, isothiazolyl, pyridinyl, pyrimidinyl, pyrazinyl, pyridazinyl, indolyl, benzofuranyl, benzothiophenyl, benzopyrazolyl, benzoimidazolyl, benzoxazolyl, benzisoxazolyl, benzothiazolyl, or benzisothiazolyl, or the like.

The term "heterocycloalkyl", "heterocyclyl" or "heterocycle" refers to a saturated or partially unsaturated single non-aromatic ring or non-aromatic multi-ring system having one or more heteroatoms (i.e., one or more ring heteroatoms selected from oxygen, nitrogen and sulfur) in the ring. Unless otherwise specified, a heterocycloalkyl has about 3 to 12 ring atoms, 3 to 10 ring atoms, 3 to 8 ring atoms, 3 to 6 ring atoms, 3 to 5 ring atoms, 4 to 6 ring atoms, or 5 to 6 ring atoms. A multi-ring heterocycloalkyl may have a fused, bridged or spirocyclic structure when permitted by valence requirements. A heterocycloalkyl may includes, but is not limited to, azetidine, aziridine, imidazolidine, morpholine, oxirane (epoxide), oxetane, thietane, piperazine, piperidine, pyrazolidine, piperidine, pyrrolidine, 1,4-oxazepan-6-yl, 1,4-diazepan-6-yl, pyrrolidinone, tetrahydrofuran, tetrahydrothiophene, dihydropyridine, tetrahydropyridine, quinuclidine, 2-oxa-6-azaspiro[3.3]heptan-6-yl, 6-oxa-1-azaspiro[3.3]heptan-1-yl, 2-thia-6-azaspiro[3.3]heptan-6-yl, 2,6-diazaspiro[3.3]heptan-2-yl, 2-azabicyclo[3.1.0]hexan-2-yl, 3-azabicyclo[3.1.0]hexanyl, 2-azabicyclo[2.1.1]hexanyl, 2-azabicyclo[2.2.1]heptan-2-yl, 4-azaspiro[2.4]heptanyl, 5-azaspiro[2.4]heptanyl, 4-azaspiro[2.5]octan-6-yl, and the like. A heterocycloalkyl group may be unsubstituted or substituted.

As used herein, the term "substitution" in "optionally substituted" refers to introduction in place of a hydrogen atom when one or more hydrogen atoms in an organic compound are substituted with another atomic group to form a derivative, and "substituent" refers to an introduced atomic group. A chemical structure denoted herein as "substituted" assumes that the valences of the indicated substituents and atoms substituted therewith are satisfied and that a chemically stable structure is formed by substitution.

In the present specification, when a combination of substituents is mentioned as one group, for example, arylalkyl, cycloalkylalkyl, or the like, the last-mentioned group generally contains the atom attached to the remainder of the molecule.

In the present specification, "-" is used to indicate a position at which a substituent is bonded to the remaining moiety of the compound. For example, if - is indicated at the end of a substituent, it means that the end is attached to the remaining moiety of the compound. In addition, when two or more substituents are linked by "-," it means that the substituent immediately before "-" is bonded to a substitutable atom of the substituent immediately after

At various positions in the present specification, substituents of compounds are designated as groups or ranges. It is specifically intended that the description include each and every individual subcombination of the above group and range elements. For example, it is specifically intended that the term "C₁-C₆ alkyl" individually denotes C₁, C₂, C₃, C₄, C₅, C₆, C₁-C₆, C₁-C₅, C₁-C₄, C₁-C₃, C₁-C₂, C₂-C₆, C₂-C₅, C₂-C₄, C₂-C₃, C₃-C₆, C₃-C₅, C₃-C₄, C₄-C₆, C₄-C₅ and C₅-C₆ alkyls.

As used herein, the term "treating" or "treatment" refers to inhibiting a disease, for example, inhibiting a disease, condition or disorder in a subject who experiences or exhibits the pathology or signs of the disease, condition or disorder, i.e., preventing further development of the pathology and/or signs, or ameliorating the disease, for example, ameliorating the disease, condition or disorder in a subject who experiences or exhibits the pathology or signs of the disease, condition or disorder, i.e., reversing the pathology and/or signs, for example, reducing the severity of the disease. A subject in need of treatment include a subject who already exhibits the symptom or sign, a subject who is more likely to have the symptom or sign, or a subject who is in need of prevention.

As used herein, the term "preventing" or "prevention" refers to preventing a disease, for example, preventing a disease, condition or disorder in a subject who may be predisposed to the disease, condition or disorder but has not yet experienced or exhibited the pathology or signs of the disease.

The term "cancer" refers to a physiological symptom, disorder or disease in mammals that is typically characterized by abnormal or uncontrolled growth of cells.

The term "pharmnaceutically acceptable" means being chemically and/or toxicologically compatible with other ingredients that constitute a compound or composition.

The term "therapeutically effective amount" or "effective amount" refers to an amount sufficient to produce a beneficial or desired clinical result, for example, an amount sufficient to alleviate, ameliorate, stabilize, reverse, slow or delay the progression of a disease.

### Compound

In one aspect of the present invention, there is provided a compound of Formula A below, or a stereoisomer, hydrate, solvate or pharmaceutically acceptable salt thereof: 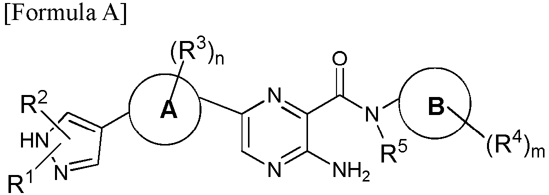 in which,
ring A is a C₆-C₁₀ aryl or a 5 to 10-membered heteroaryl containing 1 to 3 nitrogen atoms;
ring B is a C₃-C₁₀ monocyclic or bicyclic cycloalkyl or a monocyclic or bicyclic 3 to 10-membered heterocycloalkyl, wherein the heterocycloalkyl contains 1 to 3 heteroatoms selected from N, O and S;
R¹ and R² are each independently halogen, -OH, -CN, amino, nitro, oxo, C₁-C₈ alkyl, C₁-C₈ alkoxy, a straight chain or branched chain C₁-C₈ alkyl optionally substituted with one or more R', or a C₃-C₈ cycloalkyl optionally substituted with one or more R', or
when R¹ and R² are attached to adjacent ring elements, they may be taken together with the carbon atom and the nitrogen atom to which they are attached to form a pyrrolidine ring, wherein the pyrrolidine ring is optionally substituted with one or more R';
R' is one or more substituents independently selected from the group consisting of halogen, -OH, -CN, amino, nitro, oxo, C₁-C₈ alkyl and C₁-C₈ alkoxy;
R³ is selected from the group consisting of halogen, -OH, -CN, amino, nitro, oxo, C₁-C₈ alkyl, C₃-C₈ cycloalkyl and C₁-C₈ alkoxy;
R⁴ is selected from the group consisting of halogen, -OH, -CN, amino, nitro, oxo, C₁-C₈ alkyl, C₁-C₈ alkoxy, -CO-(C₁-C₈ alkyl), -CO-(C₂-C₈ alkenyl), -COOH, and -COO-C₁-C₈ alkyl, wherein said C₁-C₈ alkyl, C₁-C₈ alkoxy and C₂-C₈ alkenyl may be optionally substituted with one or more halogen, -OH or -CN;
R⁵ is H or a straight chain or branched chain C₁-C₈ alkyl;
n is an integer from 0 to 2, and wherein when n is 2, R³ may each be bound to the same or different ring atoms; and
m is an integer from 0 to 3, and wherein when m is 2 or more, R⁴ may each be bound to the same or different ring atoms.

In Formula A above, ring A may be phenyl. In addition, ring A may be a 5- or 6-membered heteroaryl containing 1 to 2 nitrogen atoms. In one embodiment, ring A may be pyridinyl or pyrimidinyl.

In Formula A above, ring B may be a monocyclic 3 to 10-membered heterocycloalkyl. In addition, ring B may be a spirocyclic 3 to 10-membered heterocycloalkyl. In one embodiment, ring B may be a monocyclic 3 to 8-membered heterocycloalkyl. In one embodiment, ring B may be a spirocyclic 3 to 8-membered heterocycloalkyl. The monocyclic or spirocyclic heterocycloalkyl may contain one nitrogen atom (N). In addition, the monocyclic or spirocyclic heterocycloalkyl may optionally contain, in addition to one N, 1 or 2 heteroatoms selected from N, O and S. For example, the heterocycloalkyl may contain, without limitation, 2 N, 1 N and 1 O, or 1 N and 1 S.

In addition, in one aspect of the present invention, there is provided a compound of Formula I below, or a stereoisomer, hydrate, solvate or pharmaceutically acceptable salt thereof:

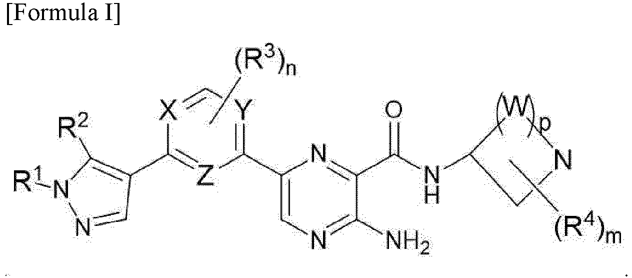

What is applicable in the embodiments related to Formula I described below can also be applied to Formula A above.

In Formula I above, X, Y and Z may be each independently C or N. X may be C or N. Y may be C or N. Z may be C or N. At least one of X, Y and Z may be C. For example, X and Z may be N, and Y may be C. Y and Z may be N, and X may be C. X may be N, and Y and Z may be C. Y may be N, and both X and Z may be C. X, Y and Z may be all C.

In Formula I above, R¹ may be a straight chain or branched chain C₁-C₈ alkyl optionally substituted with one or more R' or a C₃-C₈ cycloalkyl optionally substituted with one or more R'. Here, R' may be one or more substituents independently selected from the group consisting of halogen, -OH, CN, amino, nitro, oxo, C₁-C₈ alkyl and C₁-C₈ alkoxy. R¹ may be a straight chain or branched chain C₁-C₅ alkyl optionally substituted with one or more halogen, -OH or -CN, or a C₃-C₅ cycloalkyl optionally substituted with one or more halogen, - OH or -CN. R¹ may be H, methyl, ethyl, -CH₂-CF₃, -CH₂-CN, -CH₂-C(CH₃)₂OH, or cyclopropyl.

In Formula I above, R² may be H or a straight chain or branched chain C₁-C₈ alkyl. R² may be H.

In Formula I above, R¹ may be -CH₂-CF₃, -CH₂-CN, -CH₃, -CH₂-C(CH₃)₂OH, or cyclopropyl, and R² may be H.

In Formula I above, R¹ and R² may be taken together with the nitrogen atom and the carbon atom to which they are each attached to form a pyrrolidine ring, wherein the pyrrolidine ring may be optionally substituted with one or more R'. Here, R' may be one or more substituents independently selected from the group consisting of halogen, -OH, CN, amino, nitro, C₁-C₈ alkyl and C₁-C₈ alkoxy. R¹ and R² may be taken together with the nitrogen atom and the carbon atom to which they are each attached to form a pyrrolidine ring, wherein the pyrrolidine ring may be optionally substituted with one or more C₁-C₅ alkyl. 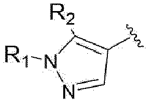 may have a structure of

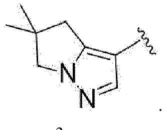

In Formula I above, R³ may be selected from the group consisting of halogen, -OH, - CN, amino, nitro, oxo, C₁-C₈ alkyl, C₃-C₈ cycloalkyl and C₁-C₈ alkoxy. R³ may be selected from the group consisting of halogen, -OH, -CN, C₁-C₅ alkyl and C₁-C₅ alkoxy. R³ may be selected from the group consisting of F, -OH, and methyl.

In Formula I above, R⁴ may be selected from the group consisting of halogen, -OH, - CN, amino, nitro, oxo, C₁-C₈ alkyl, C₁-C₈ alkoxy, -CO-C₁-C₈ alkyl, -CO-C₂-C₈ alkenyl, - COOH, and -COO-C₁-C₈ alkyl, wherein said C₁-C₈ alkyl, C₁-C₈ alkoxy and C₂-C₈ alkenyl may be optionally substituted with one or more halogen, -OH or -CN. R⁴ may be selected from the group consisting of halogen, -OH, -CN, C₁-C₅ alkyl and C₁-C₅ alkoxy. R⁴ may be F or -OH.

In Formula I above, R³ may be selected from the group consisting of halogen, -OH, - CN, C₁-C₅ alkyl and C₁-C₅ alkoxy, and R⁴ may be selected from the group consisting of halogen, -OH, -CN, C₁-C₅ alkyl and C₁-C₅ alkoxy.

In Formula I above, n may be an integer from 0 to 2, and wherein when n is 2, two R³ may each be bound to the same or different ring atoms.

In Formula I above, m may be an integer from 0 to 2, and wherein when m is 2, two R⁴ may each be bound to the same or different ring atoms.

In Formula I above, p may be an integer from 1 to 5, from 2 to 4, or from 3 to 4.

In Formula I above, W may be C, and wherein when p is 2 or more, one of W may be optionally replaced by a heteroatom selected from N, O or S. In this case, one of W may be taken together with an additional carbon atom to form a C₃-C₅ cycloalkyl ring. W may be all C, or one of W may be replaced by O, or one of W may be taken together with an additional carbon atom to form a cyclopropyl ring. The heteroatom may be any one or more selected from the group consisting of N, O and S.

In Formula I above, p may be an integer from 2 to 4, and W may be all C, or one of W may be replaced by O, or one of W may be taken together with an additional carbon atom to form a cyclopropyl ring.

In Formula I above, 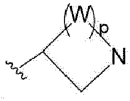 may have a structure selected from the group consisting of

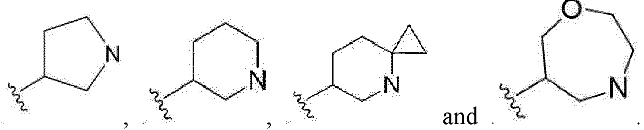

The compound of Formula I may be a compound selected from the group consisting of the following compounds:

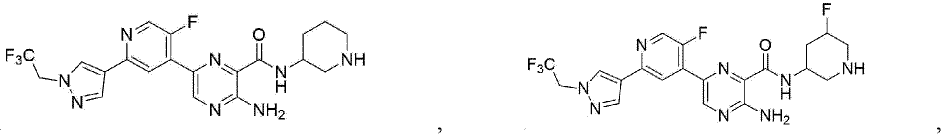

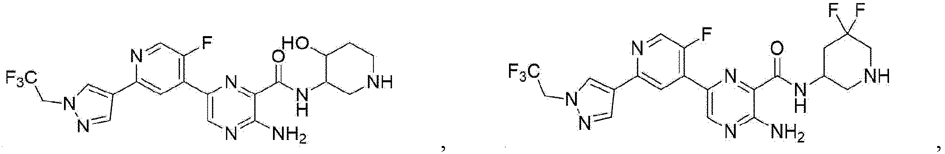

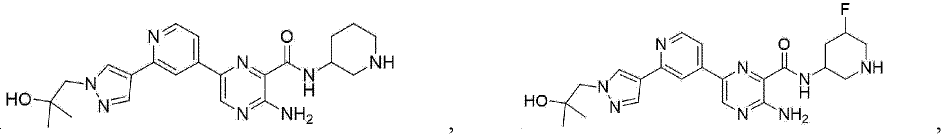

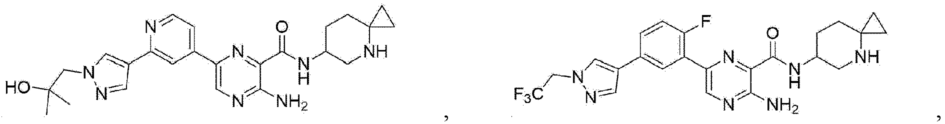

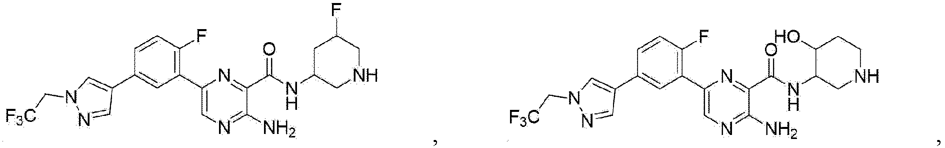

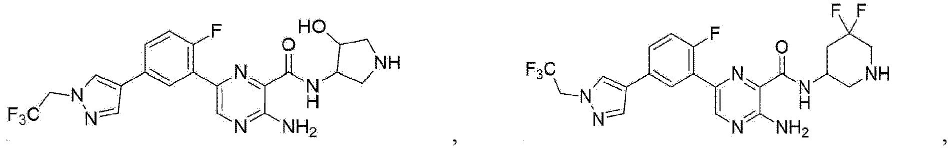

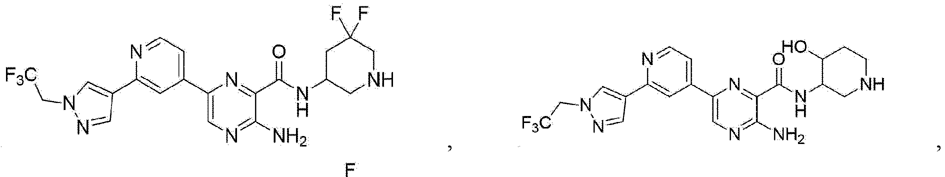

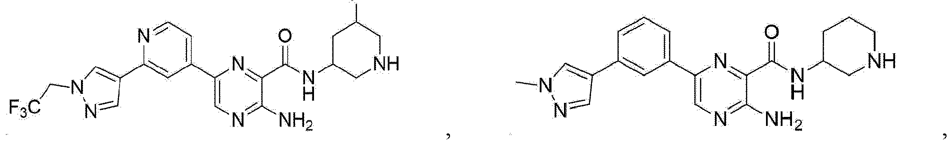

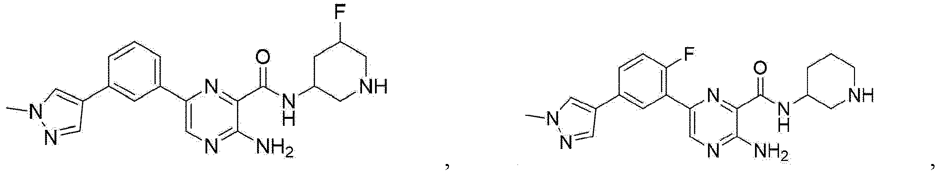

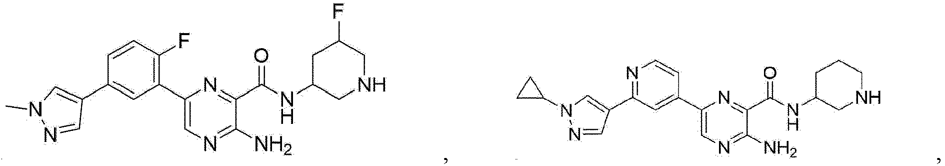

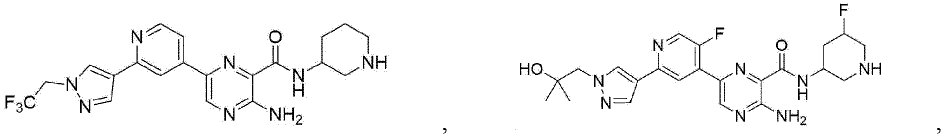

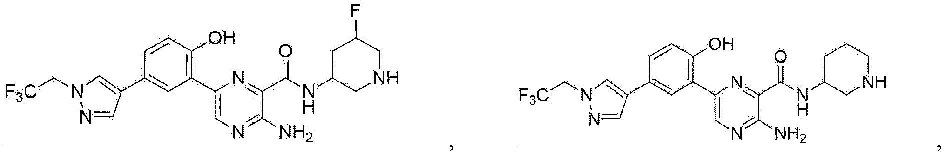

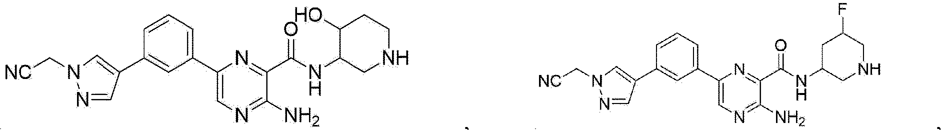

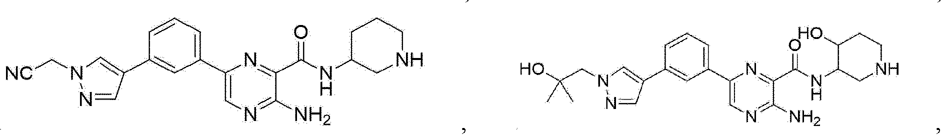

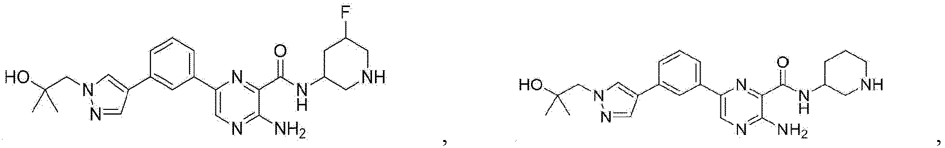

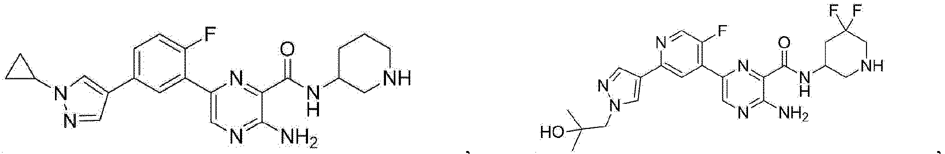

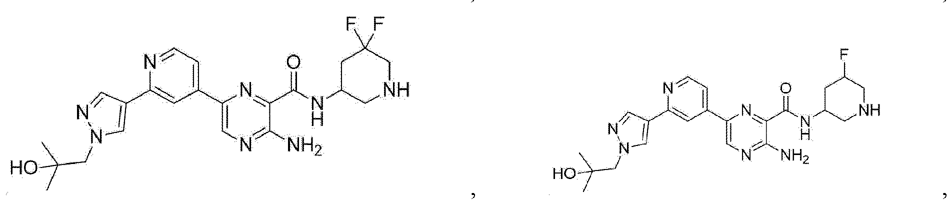

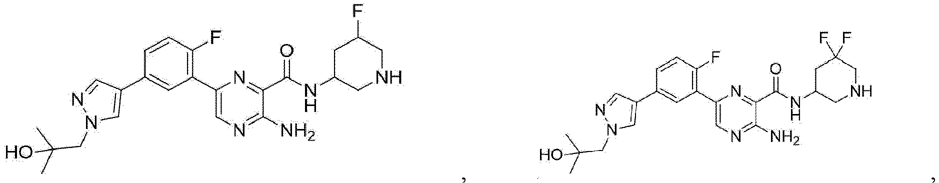

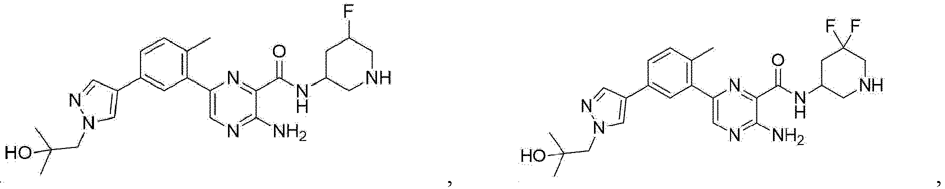

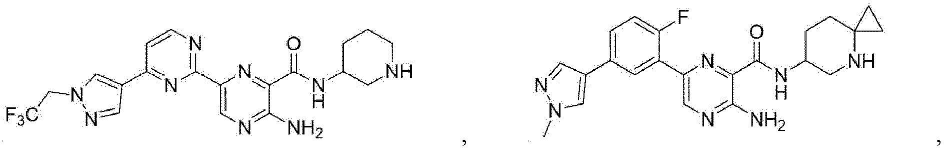

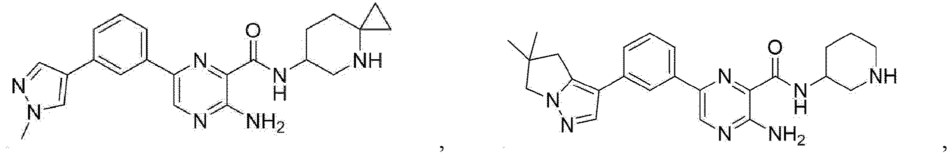

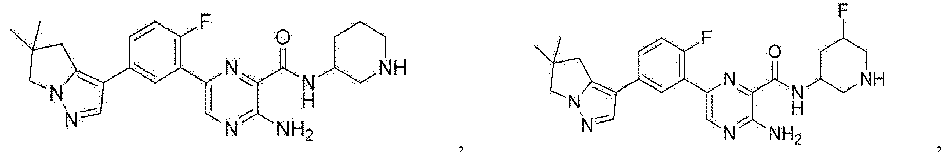

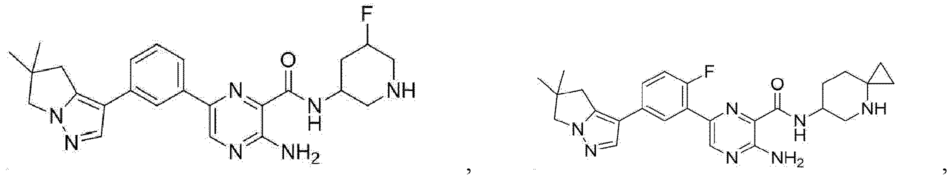

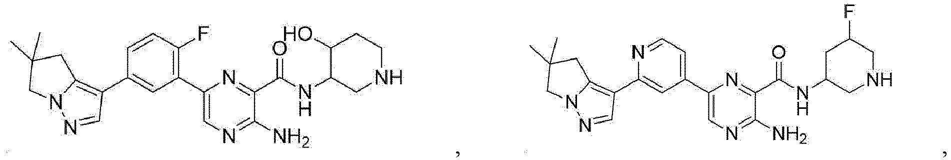

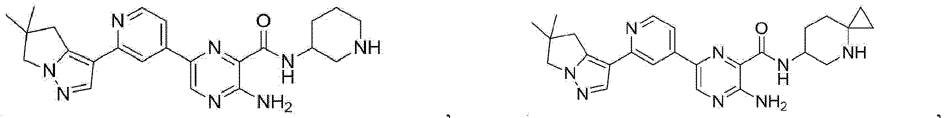

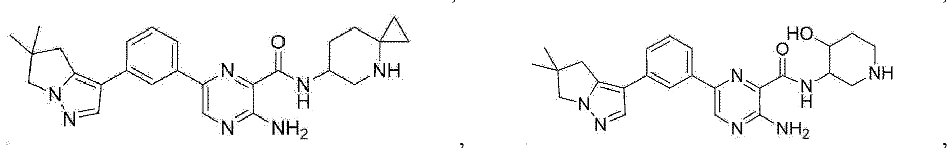

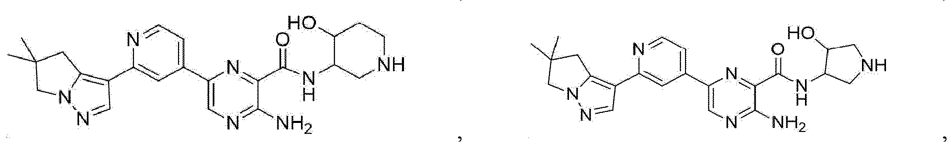

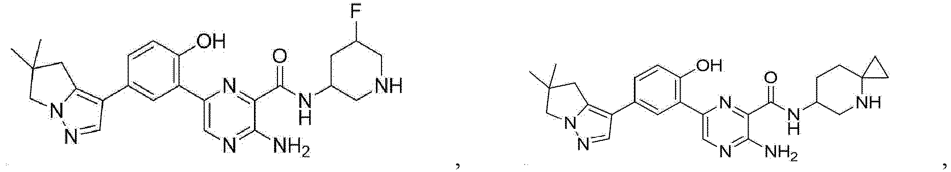

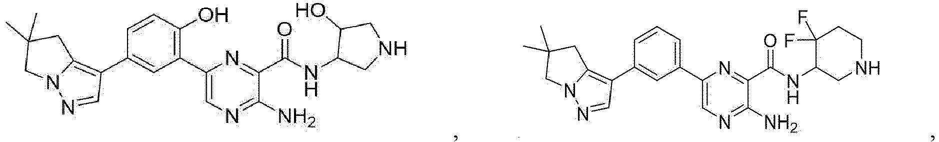

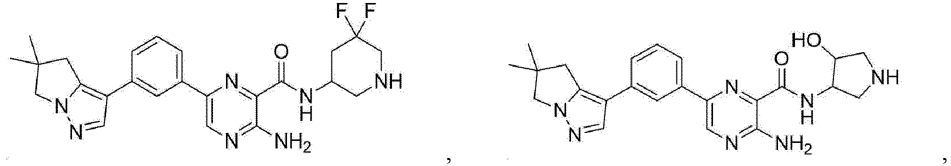

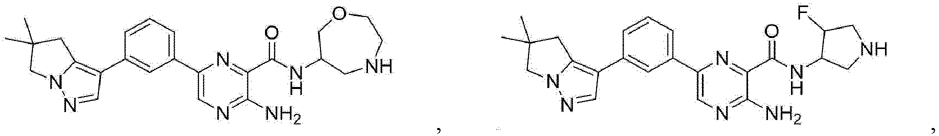

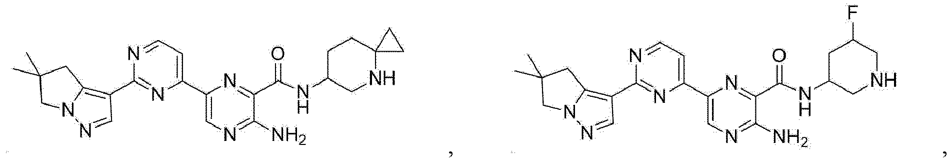

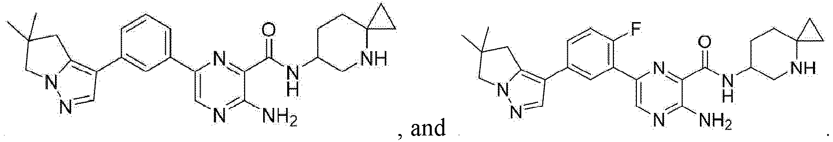

### Stereoisomer, hydrate, solvate and pharmaceutically acceptable salt

The term "isomer" in "stereoisomer" refers to a compound that has the same molecular formula but not the same linking method or spatial arrangement of constituent atoms in the molecule. An isomer includes, for example, a structural isomer and a stereoisomer. The stereoisomer may be a diastereomer or an enantiomer. The enantiomer refers to an isomer that does not overlap with its mirror image, such as the relationship between left and right hands, and is also referred to as an optical isomer. The enantiomer is classified as R (Rectus: clockwise) and S (Sinister: counterclockwise) when four or more substituents on the chiral central carbon differ from each other. A diastereomer is a stereoisomer that is not related as mirror image, and is an isomer that results from differences in the spatial arrangement of atoms. The diastereomers may be divided into cis-trans isomers and conformational isomers or conformers.

When the compound of the present invention contains a chiral center, it may exist in different stereoisomeric forms. The compound of the present invention may be, but is not limited to, an enantiomer, a diastereomer, a racemic mixture, and the like. When the stereochemistry of any particular chiral atom in a structure depicted herein is not specified, all stereoisomers are included as compounds of the present invention.

In addition, when a compound of the present invention contains a group capable of tautomerization, all tautomeric forms are included within the scope of the present invention. For example, 2-hydroxy pyridine may include 2-pyridone, and all such isomeric forms are included in the present invention.

As used herein, the term "solvate" may refer to a compound of the present invention or a salt thereof comprising a stoichiometric or non-stoichiometric amount of a solvent bound by non-covalent intermolecular forces. A preferred solvent in this regard may be any solvent that is volatile, non-toxic, and/or suitable for administration to humans. The solvate is, for example, a hydrate. The compound of the present invention may exist in the form of an unsolvated form, a hydrate solvated with water, a solvate solvated with a pharmaceutically acceptable solvent such as ethanol, etc., and all such forms are intended to be included in the scope of the present invention.

As used herein, the term "salt" may include inorganic and organic acid addition salts or base addition salts of the parent compound. A pharmaceutically acceptable salt herein may be a salt that does not cause serious irritation to the organism to which the compound is administered and does not impair the biological activity and physical properties of the compound. It may include, but is not limited to, inorganic or organic acid salts of a basic moiety such as amine, alkali (earth) metal or organic salts of an acid moiety such as carboxylic acid, and the like. The inorganic acid salt may be hydrochloride, bromate, phosphate, sulfate, or bisulfate. The organic acid salt may be formate, acetate, propionate, lactate, oxalate, tartrate, malate, maleate, citrate, fumarate, besylate, camsylate, edisylate, trichloroacetate, trifluoroacetate, benzoate, gluconate, methanesulfonate, glycolate, succinate, 4-toluenesulfonate, galacturonate, embonate, glutamate, ethanesulfonate, benzenesulfonate, p-toluenesulfonate, or aspartate. The metal salt may be a calcium salt, a sodium salt, a magnesium salt, a strontium salt, or a potassium salt.

### Preparation method of compound

The compound of the present invention, including a stereoisomer, a solvate and a salt, can be prepared by known organic synthetic methods and can be synthesized through a number of synthetic routes.

The reaction for preparing the compound of the present invention can be carried out in a suitable solvent that can be appropriately selected by one of ordinary skill in the art of organic synthesis. A suitable solvent is one that is substantially non-reactive with the starting materials (reactants), intermediates or target materials at the temperature at which the reaction takes place. One of ordinary skill in the art will be able to properly select a suitable solvent for a particular reaction step.

Protection and deprotection of various functional groups can be performed during the synthesis of the compound of the present invention. The need for protection and deprotection and appropriate protecting groups will be readily selectable by one of ordinary skill in the art.

Each reaction can be monitored by appropriate methods known in the art. For example, the synthesis of the target compound can be monitored by spectroscopic means, such as NMR (for example, ¹H or ¹³C), mass spectroscopy, or chromatography (HPLC or TLC), etc.

If necessary, the compound of the present invention can be purified by, for example, chromatography, crystallization from a solvent or a solvent mixture, distillation, extraction, and the like. Chromatography may be, for example, reverse phase, normal phase, size exclusion, ion exchange, preparative, or flash chromatography, but is not limited thereto. One of ordinary skill in the art will readily be able to select a technique that is optimal for the purification of a target material.

When the compound of the present invention is a stereoisomer, it can be isolated, if desired, from a racemic mixture by any appropriate method. For example, formation of ionic and diastereomeric salts using chiral compounds and separation by fractional crystallization, etc., formation of diastereomers using chiral derivatization reagents and conversion to pure stereoisomers after separation, and a method for separating substantially pure stereoisomers directly under chiral conditions, and the like can be used.

The compound of the present invention can be synthesized according to the synthetic process described in the following examples, and based on these examples, any target compound can be prepared by appropriately changing reactants and reaction conditions depending on the structure of the target compound.

In general, the compound of Formula I can be prepared through the synthetic process of Reaction Scheme 1 below. The compound of Formula A of the present invention can also be easily prepared by one of ordinary skill in the art by appropriately modifying Reaction Scheme 1 below and the examples herein.

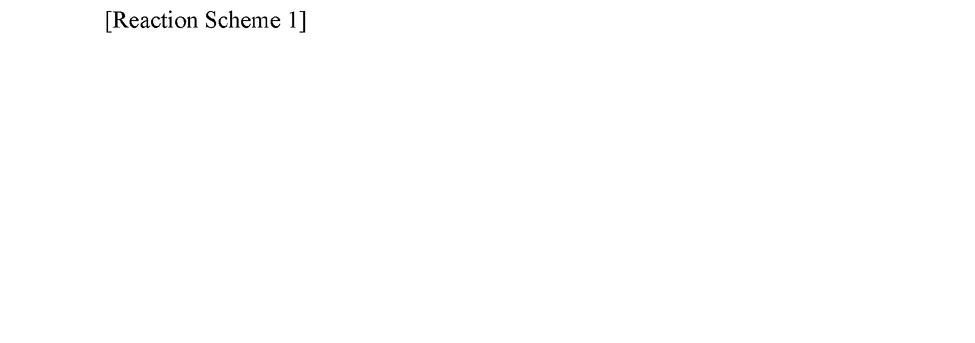

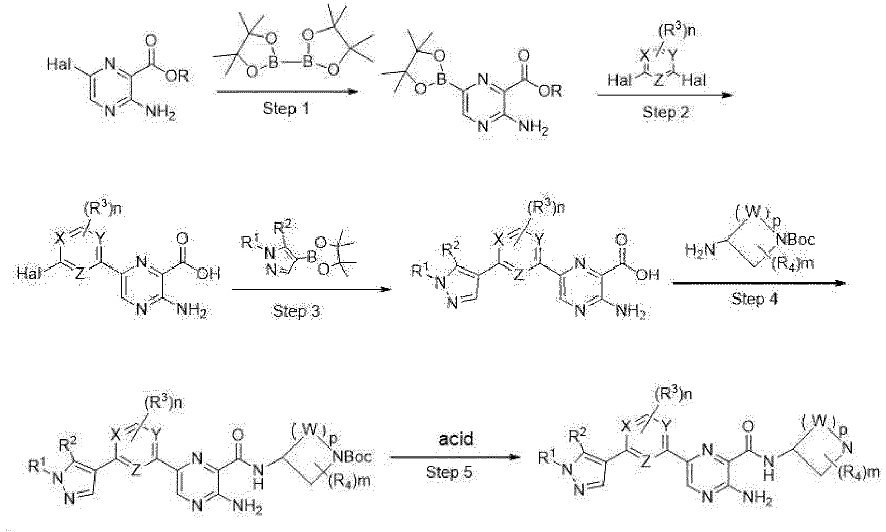

(In Reaction Scheme 1 above, Hal is a halogen, such as, Br, I, Cl or F, and R represents an alkyl group.)

In step 1, a pyrazine carboxylate compound is esterified to a pinacol boronic acid ester using an appropriate reagent (for example, bis(pinacolato)diboron) in the presence of an appropriate base (for example, KOAc) and an appropriate catalyst (for example, PdCl₂(dppf)·DCM). The reaction of step 1 may be performed at 90 °C for 15 hours in an appropriate organic solvent such as dioxane.

In step 2, a Suzuki-Miyaura coupling reaction is performed through a pinacol boronic acid ester compound using an appropriate reagent (for example, 4-bromo-2-chloro-5-fluoropyridine, 4-bromo-2-chloropyridine, 2,4-dibromo-1-fluorobenzene, 1,3-dibromobenzene, 2-bromo-4-chlorophenol, 4-bromo-2-iodo-1-methylbenzene, 2,4-dibromopyrimidine) in the presence of an appropriate base (for example, 1M sodium carbonate aqueous solution) and an appropriate catalyst (for example, PdCl₂(dppf)·DCM). The reaction of step 2 may be performed at 90 °C for 15 hours in an appropriate organic solvent such as dioxane.

In step 3, a Suzuki-Miyaura coupling reaction is performed using an appropriate reagent (for example, 4-(4,4,5,5-tetramethyl-1,3,2-dioxaborolan-2-yl)-1-(2,2,2-trifluoroethyl)-1H-pyrazole, [1-(2-hydroxy-2-methyl-propyl)pyrazol-4-yl]boronic acid pinacol ester, 1-methyl-4-(4,4,5,5-tetramethyl-1,3,2-dioxaborolan-2-yl)pyrazole, 1-cyclo-4-(4,4,5,5-tetramethyl-1,3,2-dioxaborolan-2-yl)-1H-pyrazole, 2-(4-(4,4,5,5-tetramethyl-1,3,2-dioxaborolan-2-yl)-1H-pyrazol-1-yl)acetonitrile, 5,5-dimethyl-3-(4,4,5,5-tetramethyl-1,3,2-dioxaborolan-2-yl)-5,6-dihydro-4H-pyrrolo[1,2-b]pyrazole) in the presence of an appropriate base (for example, 1M sodium carbonate aqueous solution) and an appropriate catalyst (for example, PdCl₂(dppf)·DCM). The reaction of step 3 may be performed at 90 °C for 15 hours in an appropriate organic solvent such as dioxane.

In step 4, an amide coupling reaction is performed through a carboxylic acid compound using an appropriate reagent (for example, tert-butyl (*S*)-3-aminopiperidine-1-carboxylate, tert-butyl (3S,5S)-3-amino-5-fluoropiperidine-1-carboxylate, tert-butyl (5S)-5-amino-3,3-difluoropiperidine-1-carboxylate, tert-butyl trans-3-amino-4-hydroxy-piperidine-1-carboxylate, 4-azaspiro[2.5]octan-6-amine, tert-butyl (3R,4R)-3-amino-4-hydroxypyrrolidine-1-carboxylate, tert-butyl 6-amino-4-azaspiro[2.5]octane-carboxylate) in the presence of an appropriate base (for example, triethylamine) and an appropriate catalyst (for example, HBTU). The reaction of step 4 may be performed at 25 °C for 15 hours in an appropriate organic solvent such as N,N-dimethylformamide.

In step 5, the tert-butoxycarbonyl (Boc) group of the amine is deprotected using an appropriate acid (for example, 4M HCl). The reaction of step 5 may be performed at 25 °C for about 1 hour to 15 hours in an appropriate organic solvent such as dichloromethane.

### Pharmaceutical composition and medicinal use

In another aspect, there is provided a pharmaceutical composition for preventing or treating a disease mediated by checkpoint kinase 2 activation, comprising the compound according to one aspect, a stereoisomer, hydrate, solvate or pharmaceutically acceptable salt thereof.

The compound, stereoisomer, hydrate, solvate and salt are the same as described above.

Checkpoint kinase 2 (Chk2) can be a tumor suppressor protein that encodes Chk2, a serine-threonine kinase. Chk2 can be involved in DNA repair, cell cycle arrest, or apoptosis in response to DNA damage. Whereas normal cells can activate other checkpoints upon Chk2 inhibition, cancer cells often lack one or more genome integrity checkpoints. Therefore, the compound or composition of the present invention having Chk2 inhibitory activity can further increase tumor cell selectivity of anticancer therapies such as γ-radiation or a DNA damaging agent. In addition, the compound or composition of the present invention having Chk2 inhibitory activity can enhance the activity of various anticancer agents when they stimulate Chk2 dependent cell cycle checkpoints. In addition, the compound or composition of the present invention having the Chk2 inhibitory activity can overcome resistance to a PARP inhibitor and reduce toxicity.

The disease mediated by checkpoint kinase 2 activation may be cancer. Since a Chk2 inhibitor can increase the activity of various anticancer agents, various types of cancer will be able to be treated by the compound, composition and method of the present invention. The cancer may be a solid cancer or a non-solid cancer. Solid cancer refers to a cancerous tumor that has occurred in organs, for example, liver, lung, breast, and skin. Non-solid cancer is a cancer that has occurred in the blood, and is also referred to as hematological cancer. The cancer may be carcinoma, sarcoma, hematopoietic cell-derived cancer, germ cell tumor, or blastoma.

The disease mediated by checkpoint kinase 2 activation may be a cancer having an aberration of ATM, CHK2, and/or TP53 gene encoding p53 protein. For example, the above aberration of the gene may include homozygous germ cell mutations of ATM (for example, ataxia telangiectasia), somatic cell mutations of ATM (for example, lymphatic malignancy), loss of expression of ATM (for example, colorectal cancer, breast cancer, non-small cell lung cancer, adenocarcinoma of the lung, or pancreatic cancer), loss of heterozygoxity of ATM due to 11q deletion (for example, breast cancer, chronic lymphocytic leukemia and other lymphatic malignancies, childhood neuroblastoma), CHK2 mutations (for example, colon cancer, kidney cancer, breast cancer, prostate cancer), homozygous germ cell CHK2 mutations, TP53 gene mutations (for example, Li-Fraumeni syndrome), loss of heterozygoxity of TP53 through 17p allelic deletion, and the like.

Cancers that can be prevented or treated by the compound of the present disclosure, a stereoisomer, hydrate, solvate or pharmaceutically acceptable salt thereof, may be selected from the group consisting of, for example, ovarian cancer, cervical cancer, endometrial cancer, uterine sarcoma, vulvar cancer, breast cancer, skin cancer, head and neck cancer, pancreatic cancer, lung cancer (for example, small cell lung cancer, non-small cell lung cancer, adenocarcinoma of the lung, squamous cell cancer or large cell cancer), large intestine cancer, colorectal cancer, colon cancer, gastric cancer, prostate cancer, bladder cancer, urethral cancer, liver cancer, kidney cancer, skin cancer, cerebrospinal tumor, brain cancer, thymoma, mesothelioma, bronchial cancer, nasopharyngeal cancer, laryngeal cancer, esophageal cancer, biliary tract cancer, testicular cancer, germ cell tumor, thyroid cancer, parathyroid cancer, lymphoma, myelodysplastic syndrome (MDS), myelofibrosis, acute myeloid leukemia (AML), acute lymphocytic leukemia (ALL), chronic myeloid leukemia (CML), chronic lymphocytic leukemia (CLL), multiple myeloma, endocrine cancer, sarcoma, ataxia telangiectasia, childhood neuroblastoma, and Li-Fraumeni syndrome.

The pharmaceutical composition may further comprise a known active ingredient having anticancer activity. The pharmaceutical composition may be a single composition or a separate composition. For example, the pharmaceutical composition according to one aspect may be a composition of an oral dosage form, and the known active ingredient having anticancer activity may be a composition of a parenteral dosage form.

The pharmaceutical composition may comprise a pharmaceutically acceptable carrier. The carrier is used as a meaning that includes an excipient, a diluent, or an auxiliary agent. The carrier may be selected from the group consisting of, for example, lactose, dextrose, sucrose, sorbitol, mannitol, xylitol, erythritol, maltitol, starch, gum acacia, alginate, gelatin, calcium phosphate, calcium silicate, cellulose, methyl cellulose, polyvinyl pyrrolidone, water, physiological saline, a buffer such as PBS, methylhydroxy benzoate, propylhydroxy benzoate, talc, magnesium stearate, and mineral oil. The composition may comprise a filler, an anti-agglomerating agent, a lubricant, a wetting agent, a flavoring agent, an emulsifying agent, a preservative, or a combination thereof.

The pharmaceutical composition may be prepared in any dosage form according to a conventional method. The composition may be formulated into, for example, an oral dosage form (for example, a powder, a tablet, a capsule, a syrup, a pill, or a granule), or a parenteral dosage form (for example, an injection). In addition, the composition may be prepared as a systemic dosage form or a topical dosage form.

In the pharmaceutical composition, the solid preparation for oral administration may be a tablet, a pill, a powder, a granule, or a capsule. The solid preparation may further comprise an excipient. The excipient may be, for example, starch, calcium carbonate, sucrose, lactose, or gelatin. In addition, the solid preparation may further comprise a lubricant such as magnesium stearate or talc. In the pharmaceutical composition, the liquid preparation for oral administration may be a suspension, an internal solution, an emulsion, or a syrup. The liquid preparation may comprise water or liquid paraffin. The liquid preparation may comprise an excipient such as a wetting agent, a sweetening agent, a flavoring agent, or a preservative. In the pharmaceutical composition, the preparation for parenteral administration may be a sterilized aqueous solution, a non-aqueous solution, a suspension, an emulsion, a freeze-dried preparation and/or a suppository. A non-aqueous solution or a suspension may comprise a vegetable oil or an ester. The vegetable oil may be, for example, propylene glycol, polyethylene glycol, or olive oil. The ester may be, for example, ethyl oleate. The base of the suppository may be witepsol, macrogol, tween 61, cacao butter, laurin butter, or glycerogelatin.

The pharmaceutical composition comprises a therapeutically effective amount of the compound according to one aspect, a stereoisomer, hydrate, solvate or pharmaceutically acceptable salt thereof, as an active ingredient. "Active ingredient" refers to a physiologically active substance used to achieve pharmacological activity (for example, treatment of cancer).

The pharmaceutical composition may comprise an effective amount of the compound according to one aspect, a stereoisomer, hydrate, solvate or pharmaceutically acceptable salt thereof. The effective amount can be appropriately selected by one of ordinary skill in the art depending on the cell or subject to be selected. A preferred dosage of the pharmaceutical composition varies depending on the condition and body weight of the subject, the severity of the disease, the type of drug, the route and duration of administration, but can be appropriately selected by one of ordinary skill in the art. However, the compound, stereoisomer, hydrate, solvate or pharmaceutically acceptable salt thereof may be administered in an amount of, for example, about 0.0001 mg/kg to about 100 mg/kg, or about 0.001 mg/kg to about 100 mg/kg, which may be divided into 1 to 24 times a day, 1 to 7 times in 2 days to 1 week, or 1 to 24 times in 1 month to 12 months. For example, the administration may be performed once a day, multiple times a day, or once a week, once every 2 weeks, once every 3 weeks, or once every 4 weeks to once a year. In the pharmaceutical composition, the compound, stereoisomer, hydrate, solvate or pharmaceutically acceptable salt thereof may be included in an amount of about 0.0001% by weight to about 10% by weight, or about 0.001% by weight to about 1% by weight based on the total weight of the entire composition.

The method of administration may be oral or parenteral administration. The method of administration may be, for example, oral, transdermal, subcutaneous, rectal, intravenous, intraarterial, intraperitoneal, intramuscular, intrasternal, topical, intranasal, intratracheal, or intradermal route. The composition may be administered systemically or topically, and may be administered alone or in combination with other pharmaceutically active compounds.

### Administration in combination

In some embodiments, the compound according to one aspect, or a stereoisomer, hydrate, solvate or pharmaceutically acceptable salt thereof, may be administered alone or in combination with an additional therapeutic agent having anticancer activity. The additional therapeutic agent may be an agent that has complementary or synergistic pharmacological activity, for example, through different mechanisms, without alleviating the pharmacological activity of the compound of the present disclosure. The additional therapeutic agent may be administered together with the compound of the present disclosure as a single pharmaceutical composition, or may be administered separately. When administered separately, the compound of the present disclosure and the additional therapeutic agent may be administered simultaneously or sequentially in any order. The additional therapeutic agent may include surgical therapy and ionizing radiation.

For example, the additional therapeutic agent may be selected from the group consisting of surgical therapy, ionizing radiation, a chemotherapeutic agent (for example, an alkylating agent, an antimetabolite, a topoisomerase inhibitor, a microtubule inhibitor, an antibiotic or a plant alkaloid, a hormonal agent), a targeted anticancer agent, an immune checkpoint inhibitor, a cell therapeutic agent, an antibody therapeutic agent, an anticancer virus and a combination thereof, but is not limited thereto.

The alkylating agent may include, for example, cisplatin, carboplatin, oxaliplatin, busulfan, chlorambucil, cyclophosphamide, ifosfamide, melphalan, thiotepa, altretamine, procarbazine, dacarbazine, streptozotocin, carmustine, lomustine, uracil nitrogen mustard, triethylenemelamine, temozolomide, and 2-chloroethyl-3-sarcosinamide-1-nitrosourea, and the like.

The antimetabolite may include chlorodeoxyadenosine, cytarabine, capecitabine, 2'-deoxycoformycin, fludarabine phosphate, 5-fluorouracil (5-FU), 5-fluoro-2'-deoxyuridine, gemcitabine, camptothecin, 6-mercaptopurine, methotrexate, 4-methylthioamphetamine, pemetrexed and thioguanine and the like, but is not limited thereto.

The topoisomerase inhibitor may include topotecan, irinotecan, etoposide, bleomycin, adriamycin, daunorubicin, and the like, but is not limited thereto.

The microtubule inhibitor includes docetaxel, paclitaxel, vinblastine, vincristine, vinorelbine, and the like, but is not limited thereto.

The anticancer antibiotic or a plant alkaloid may include actinomycin-D, bleomycin, cryptophycins, daunorubicin, doxorubicin, idarubicin, irinotecan, L-asparaginase, mitomycin-C, mithramycin, navelbine, paclitaxel, docetaxel, topotecan, vinblastine, vincristine, teniposide (VM-26), and etoposide (VP-16), and the like, but is not limited thereto.

The hormonal agent includes a 5α-reductase inhibitor, aminoglutethimide, anastrozole, bicalutamide, chlorotrianisene, diethylstilbestrol (DES), dromostanolone, estramustine, ethinyl estradiol, flutamide, fluoxymesterone, goserelin, hydroxyprogesterone, letrozole, leuprolide, medroxyprogesterone acetate, megestrol acetate, methylprednisolone, methyltestosterone, mitotane, nilutamide, prednisolone, arzoxifene (SERM-3), tamoxifen, toremifene, fulvestrant, exemestane, testolactone, testosterone, triamcinolone, zoladex, and the like, but is not limited thereto.

The targeted anticancer agent is a therapeutic agent that specifically kills cancer cells by blocking signals involved in the growth and development of cancer by targeting changes in specific proteins or specific genes that appear only in cancer cells. It is classified into monoclonal antibodies that react outside cells and small molecule substances that act inside cells. Monoclonal antibodies are anticancer agents that block cancer cell-inducing signals transmitted to the outside of cells, and act on initiation signals related to proliferation, death and the like, and small molecule substances act on complex signal transduction occurring inside cells.

Specifically, the proteins to be targeted may be EGFR, VEGFR, CD20, CD38, RNAK-L, BTK, Bcr-abl, PDGFR/FGFR family, MEK/RAF/KRAS, HER2/Neu, ubiquitin, JAK, ALK, PARP, TGFβRI, proteasome, Bcl-2, C-Met, VR1, VR2, VR3, c-kit, AXL, RET, Braf, DNMT, CDK4/6, STING, and the like.

The term "epidermal growth factor receptor (EGFR)" is a cell membrane receptor that regulates cell growth, division, survival and death, and expression of EGFR is increased in tumor tissues of various cancers. Tumor tissues with increased EGFR are known to have high invasion, metastasis and resistance to anticancer agents. An EGFR inhibitor is a substance that inhibits the EGFR, and may be, in one embodiment, cetuximab, trastuzumab, pertuzumab, gefitinib, erlotinib, osimertinib or panitumumab.

The term "vascular endothelial growth factor receptor (VEGFR)" is a cell membrane receptor for vascular endothelial growth factor that induces angiogenesis, and a VEGFR inhibitor inhibits the angiogenesis to inhibit tumor growth and metastasis. In one embodiment, a VEGF inhibitor or a VEGFR inhibitor may be axitinib, lenvatinib, bevacizumab, ramucirumab or aflibercept.

The term "CD20 (B lymphocyte antigen CD20)" is a protein expressed on the surface of B cells and is used as a target protein for treatment of B cell lymphoma. An inhibitor targeting CD20 may be rituximab or obinutuzumab.

The term "CD38 (cluster of differentiation 38)" is a protein that regulates cell proliferation and death while acting as a signal transduction system receptor in immune cells, and an inhibitor targeting this protein may be daratumumab.

The term "RNAK-L (receptor activator of nuclear factor kappa-B ligand)" is a RANK receptor expressed on the surface of osteoclasts, and when activated by binding to the ligand, it acts to cause bone destruction. A RANK-L inhibitor is mainly used for cancer patients suffering from bone metastasis or osteoporosis, and may be, for example, denosumab.

The term "BTK (Bruton's tyrosine kinase)" is an enzyme involved in the proliferation of B cells, and when overexpressed, it can develop into hematological cancer. In one embodiment, an inhibitor targeting BTK may be ibrutinib.

The term "Bcr-abl" is a fusion protein that is highly expressed in patients with chronic myeloid leukemia, and is known to induce abnormal proliferation of blood cells. Specifically, the inhibitor of this protein may be dasatinib, nilotinib, imatinib or bosutinib.

The term "tumor growth factor β receptor (TGFβR)" is a cell membrane receptor for tumor growth factor, and regulates the growth, migration, differentiation and death and the like of epithelial cells and hematopoietic cells. An inhibitor targeting TGFβR may include galunisertib or vactosertib, but is not limited thereto.

The term "PDGFR (platelet derived growth factor receptor)" is a cell membrane receptor for PDGF that is frequently expressed in cancer cells, and is known to be involved in angiogenesis to regulate cancer growth, metastasis, and drug resistance. FGFR (fibroblast growth factor receptor) is a receptor for fibroblast growth factor (FGF) and regulates various biological processes including cell growth, differentiation and migration and the like. The FGFR gene is frequently mutated, and these mutants are commonly observed in breast cancer, uterine cancer, ovarian cancer, cervical cancer, and the like. An inhibitor targeting PDGFR or FGFR may be nintedanib, sunitinib, sorafenib, cabozantinib, lenvatinib, regorafenib, masitinib, semaxanib, tivozanib, vandetanib, axitinib or pazopanib.

The term "MEK/RAF/KRAS" is an intracellular signal transduction mediator involved in cell proliferation, cell cycle regulation, cell survival, angiogenesis, cell migration, and the like, and is overactivated in cancer cells. An inhibitor targeting MEK/RAF/KRAS may be trametinib, dabrafenib or sotorasib.

The term "HER-2/neu (human epidermal growth factor receptor 2) regulates cell proliferation by the activation of PI3K/AkT. It is overexpressed in metastatic breast cancer and ovarian cancer and the like, and is known to induce resistance to anticancer agents. The Her2/neu targeting anticancer agent may be trastuzumab, afatinib, lapatinib or neratinib.

The term "ubiquitin" maintains cellular homeostasis by binding to other proteins and inducing proteolysis by proteasome, a proteolytic enzyme (ubiquitin-proteasome system, UPS). Abnormal expression or activity of the UPS is observed in various tumors, and the inhibitor of UPS exhibits anticancer activity. Specifically, an inhibitor targeting ubiquitin or proteasome may be lenalidomide or ixazomib.

The term "JAK (Janus kinase)" is an upstream protein to STAT, which is a transcription factor that regulates cell proliferation, cell survival, cell migration and immune response, and an inhibitor of JAK is known to reduce cell proliferation and induce cell death through inhibition of STAT activity. An inhibitor targeting JAK may be ruxolitinib, lestaurtinib or pacritinib.

The term "MAP2K (mitogen-activated protein kinase kinase)" is an intracellular signal transduction mediator involved in cell proliferation, cell cycle regulation, cell survival, angiogenesis, cell migration, and the like by phosphorylation of MAPK, and is overactivated in cancer cells. An inhibitor targeting MAP2K may be cobimetinib, selumetinib, trametinib or binimetinib.

The term "ALK (anaplastic lymphoma kinase)" is a signal transduction mediator that promotes cell proliferation, cell migration and angiogenesis and inhibits cell death, and is overactivated in various cancer tissues. An inhibitor targeting ALK may be alectinib or crizotinib.

The term "Bcl-2" is a protein that inhibits cell death, and is overexpressed or overactivated in various cancer tissues. An inhibitor targeting Bcl-2 may be venetoclax.

The term "C-Met" is a receptor for hepatocyte growth factor (HGF), and activates signal transduction related to cell growth, formation, motility, survival and angiogenesis and the like. A C-Met targeting anticancer agent may be crizotinib or cabozantinib.

The term "VR (vanilloid receptor)" is also known as TRPV (transient receptor potential vanilloid), and exists in the form of VR1, VR2, VR3, VR4, VR5 and VR6. VR is known to regulate proliferation, death, migration, infiltration and angiogenesis of cancer cells at each stage in the cancer progression process.

The term "c-kit" is also known as CD117, and induces signal transduction that activates cell survival, proliferation and differentiation. c-kit is a proto-oncogene, and overexpression or mutation of this gene is associated with cancer development.

The term "AXL (tyrosine-protein kinase receptor UFO)" is a tyrosine kinase receptor present on the cell surface and mediates signal transduction involved in cell proliferation and survival. It is known to be involved in resistance to anticancer agents in anticancer treatment. In one embodiment, an AXL targeting anticancer agent may be bemcentinib or gilteritinib.

The term "RET (rearranged during transfection)" is a receptor that mediates signals involved in cell proliferation, cell death and survival, and mutations in RET are known to be involved in cancer development. An inhibitor targeting RET may be selpercatinib or pralsetinib, but is not limited thereto.

The term "Braf" is a MAPK signal transduction mediator involved in cell proliferation, cell cycle regulation, cell survival, angiogenesis, cell migration, and the like, and its genetic mutations are observed in cancer cells. An inhibitor targeting Braf may be vemurafenib.

The term "PARP (poly[ADP-ribose] polymerase)" is a protein that is activated by recognition of damaged DNA in the nucleus and then activates DNA repair-related proteins. An inhibitor targeting PARP inhibits the proliferation of cancer cells by inhibiting DNA repair in cancer cells. In one embodiment, an inhibitor targeting PARP may be olaparib, talazoparib, niraparib or rucaparib.

The term "DNA methyltransferase (DNMT)" is an enzyme that attaches a methyl group to a histone protein wrapped around DNA, and through this process, the expression of the gene is inhibited. An inhibitor targeting DMNT exhibits anticancer activity by inhibiting hypermethylation of cancer suppressor genes and inducing normal expression of cancer suppressor genes. In one embodiment, an inhibitor targeting DNMT may be azacitidine, decitabine, or guadecitabine.

The term "CDK (cyclin dependent kinase) 4/6" is a protein that promotes cell growth by regulating the cell cycle, and is overactivated during the development and progression of various malignancies. An inhibitor targeting CDK4/6 exhibits anticancer activity by inhibiting the cell cycle of cancer cells, inhibiting cell proliferation and inducing cell death. An inhibitor targeting CDK4/6 may be abemaciclib, ribociclib or palbociclib.

The term "STING (stimulator of interferon genes)" is an in vivo sensor that recognizes DNA fragments from cancer cells, and activates immune cells in the body, such as dendritic cells, by stimulating interferon genes. A STING agonist exhibits an immune enhancing effect and a cancer angiogenesis inhibitory effect. For example, a STING agonist may be CDNs, SB11285, DMXAA, and the like.

The targeted anticancer agent includes trastuzumab, ramucirumab, bismodegib, sonidegib, bevacizumab, lapatinib, neratinib, pertuzumab, trastuzumab emtansine, palbociclib, cetuximab, panitumumab, aflibercept, regorafenib, imatinib mesylate, nintedanib, sunitinib, cabozantinib, regorafenib, masitinib, semaxanib, tivozanib, vandetanib, denosumab, alitretinoin, axitinib, lenvatinib, pazopanib, trametinib, dabrafenib, temsirolimus, everolimus, tretinoin, dasatinib, nilotinib, imatinib, bosutinib, galunisertib, vactosertib, rituximab, alemtuzumab, ofatumumab, obinutuzumab, daratumumab, ibrutinib, idelalisib, blinatumomab, sorafenib, crizotinib, erlotinib, gefitinib, sotorasib, afatinib, ceritinib, lenalidomide, ixazomib, ruxolitinib, lestaurtinib, pacritinib, cobimetinib, selumetinib, binimetinib, alectinib, venetoclax, bemcentinib, gilteritinib, selpercatinib, pralsetinib, vemurafenib, olaparib, talazoparib, niraparib, rucaparib, azacitidine, decitabine, guadecitabine, abemaciclib, ribociclib , palbociclib, osimertinib, and necitumumab, and the like, but is not limited thereto.

The additional therapeutic agent may be an immune checkpoint inhibitor. The term "immune checkpoint inhibitor" is a substance that inhibits the activity of an immune checkpoint protein, which inhibits the differentiation, proliferation, and activity of immune cells, and is known to eliminate cancer cells by preventing them from exerting functions to evade the immune system. The immune checkpoint inhibitor may be any one selected from the group consisting of an anti-CTLA-4 antibody, an anti-PD-1 antibody, an anti-PD-L1 antibody, an anti-PD-L2 antibody, an anti-B7-H4 antibody, an anti-HVEM antibody, an anti-TIM3 antibody, an anti-GAL9 antibody, an anti-LAG3 antibody, an anti-VISTA antibody, an anti-KIR antibody, an anti-BTLA antibody and an anti-TIGIT antibody. In one embodiment, the immune checkpoint inhibitor may be ipilimumab, pembrolizumab, nivolumab, cemiplimab, atezolizumab, avelumab and durvalumab and the like, but is not limited thereto.

The additional therapeutic agent may be a cell therapeutic agent. The term "cell therapeutic agent" is a therapeutic agent that exhibits an anticancer effect by activating an immune response in the body using immune cells such as dendritic cells, natural killer cells, T cells, and the like. An immune cell therapeutic agent is used by extracting immune cells in the body, and enhancing or genetically modifying them and then injecting them back into the body. Representative immune cell therapeutic agents may include T cell receptor-modified T cells (TCR-T), chimeric antigen receptor-modified T cells (CAR-T), and the like. Specifically, it may be tisagenlecleucel or axicabtagene ciloleucel, but is not limited thereto.

The additional therapeutic agent may be an antibody therapeutic agent. The term "antibody therapeutic agent" is a therapeutic agent that exhibits an anticancer effect using an antibody that recognizes a specific protein of cancer cells as an antigen. An antibody therapeutic agent may be cetuximab, trastuzumab, rituximab, ibritumomab, tositumomab, brentuximab, ofatumumab, obinutuzumab, necitumumab, bevacizumab, ramucirumab, nivolumab, pembrolizumab, atezolizumab, durvalumab, ipilimumab, and the like.

The antibody therapeutic agent may include an antibody drug conjugate (ADC). The term "ADC (antibody drug conjugate)" is a therapeutic agent that exhibits high anticancer effects through targeted delivery by chemical binding of an antibody and a cytotoxic drug. It may be gemtuzumab-ozogamicin, brentuximab-vedotin, trastuzumab-emtansine, inotuzumab-ozogamicin, and the like.

The additional therapeutic agent may be an anticancer virus therapeutic agent. The term "anticancer virus therapeutic agent" is a therapeutic agent that kills cancer by inserting a specific gene targeting cancer cells into a virus capable of proliferation and having infectivity. The anticancer virus therapeutic agent may be Talimogene Laherparepvec.

In one embodiment, the additional therapeutic agent may be a DNA damaging agent such as an alkylating agent, an antimetabolite, a topoisomerase inhibitor, a microtubule inhibitor, an antibiotic or a plant alkaloid. It is known that cancers caused by DNA repair defects can be sensitive to a DNA damaging agent. Therefore, the compound of the present invention can enhance the anticancer activity of a DNA damaging agent. For example, a DNA damaging agent may include radiation, cisplatin, oxaliplatin, carboplatin, adriamycin or doxorubicin, daunorubicin, irinotecan, gemcitabine, temozolomide, capecitabine, topotecan, camptothecin, cytarabine, 5-FU (fluorouracil), cyclophosphamide, etoposide or etoposide phosphate, teniposide, daunorubicin, pemetrexed, and the like. In certain embodiments, a DNA damaging agent may be selected from the group consisting of radiation, cisplatin, doxorubicin, irinotecan, temozolomide, capecitabine, camptothecin, Ara-C, and 5-FU. In certain embodiments, a DNA damaging agent may be radiation, cisplatin, or doxorubicin.

In one embodiment, the additional therapeutic agent may be a PARP inhibitor. In one embodiment, the PARP inhibitor may be olaparib, talazoparib, niraparib or rucaparib.

"PARP (poly[ADP-ribose] polymerase)" is a protein that is activated by recognition of damaged DNA in the nucleus and activates DNA repair-related proteins. A PARP inhibitor inhibits the proliferation of cancer cells by inhibiting DNA repair in cancer cells. In particular, it is known that cells in which homologous recombination repair (HRR) function is lost are highly dependent on PARP activity. In patients with mutations in BRCA1/2, which play an important role in HRR, the anticancer effect of a PARP inhibitor has been clinically confirmed (N. Engl. J. Med. 361, 123, 2009). However, when a PARP inhibitor is administered, hematological toxicity such as reduction of reticulocytes, B-cell precursor cells, and immature thymocytes and myelosuppression is problematic. In addition, a PARP inhibitor has also raised the issue of resistance expression through the following mechanisms: recovery of homologous recombination (HR), stabilization of replication fork, increase in PARylation activity, or elimination of drugs by efflux transporters (Cancer Drug Resist., 2, 665-79, 2019).

In this context, Chk2 is known to interact with proteins involved in resistance to a PARP inhibitor. For example, Chk2 has been reported to be involved in activation of HR function through interaction with BRCA1, activation of PARylation through interaction with PARP1, and replication stabilization through interaction with Msh2. In addition, it has been reported that combination with a CHK2 inhibitor reduces the hematological toxicity of a PARK inhibitor. Therefore, the compound of the present invention having Chk2 inhibitory activity can exhibit a more advantageous anticancer effect when combined with a PARP inhibitor.

The additional therapeutic agent may include one or more anticancer agents. Specifically, the compound according to one aspect, or a stereoisomer, hydrate, solvate or pharmaceutically acceptable salt thereof, may be combined with two anticancer agents. For example, two anticancer agents may be a chemotherapeutic agent and a targeted anticancer agent; a chemotherapeutic agent and an anticancer virus; a targeted anticancer agent and an antibody therapeutic agent; a chemotherapeutic agent and a cell therapeutic agent; and a chemotherapeutic agent and an immune checkpoint inhibitor. In addition, two anticancer agents may be a targeted anticancer agent and an anticancer virus; a targeted anticancer agent and an antibody therapeutic agent; a targeted anticancer agent and a cell therapeutic agent; a targeted anticancer agent and an immune checkpoint inhibitor. In addition, two anticancer agents may be an anticancer virus and an antibody therapeutic agent; an anticancer virus and a cell therapeutic agent; and an anticancer virus and an immune checkpoint inhibitor. In addition, two anticancer agents may be an antibody therapeutic agent and a cell therapeutic agent; and an antibody therapeutic agent and an immune checkpoint inhibitor.

The compound, solvate, stereoisomer or pharmaceutically acceptable salt thereof may be combined with three anticancer agents. In addition to the aforementioned combination of two anticancer agents, a different anticancer agent may be further included.

According to one embodiment, the compound, solvate, stereoisomer or pharmaceutically acceptable salt thereof may be used together with two or more chemotherapeutic agents and immune checkpoint inhibitors. For example, the two or more chemotherapeutic agents may include an alkylating agent and an antimetabolite.

The compound, solvate, stereoisomer or pharmaceutically acceptable salt thereof may be used together with four, five or six anticancer agents. In addition to the aforementioned combination of three anticancer agents, a different anticancer agent may be further included.

### Method of inhibiting Chk2, treatment method, etc.

In another aspect, there is provided a method of inhibiting checkpoint kinase 2 (Chk2), comprising adding the compound according to one aspect, or a stereoisomer, hydrate, solvate or pharmaceutically acceptable salt thereof, to a cell in vitro.

The method may be performed in vitro. In the method, the compound according to one aspect, or a stereoisomer, hydrate, solvate or pharmaceutically acceptable salt thereof, may exhibit an IC₅₀ of 1000 nM or less, 100 nM or less, or 10 nM or less.

In another aspect, there is provided a method of inhibiting checkpoint kinase 2 activation, comprising administering the compound according to one aspect, or a stereoisomer, hydrate, solvate or pharmaceutically acceptable salt thereof, to a subject.

The compound, stereoisomer, hydrate, solvate, salt and checkpoint kinase 2 are the same as described above.

In another aspect, there is provided a method for preventing or treating cancer, comprising administering the compound according to one aspect, or a stereoisomer, hydrate, solvate or pharmaceutically acceptable salt thereof, to a subject. The compound, stereoisomer, hydrate, solvate, salt, cancer, prevention and treatment are the same as described above.

The subject may be a mammal, such as a human, mouse, rat, cow, horse, pig, dog, monkey, sheep, goat, ape, or cat. The subject may be a subject suffering from, or highly likely to suffer from, symptoms associated with cancer.

The method may further comprise administering an additional therapeutic agent having anticancer activity to the subject. The additional therapeutic agent may be administered to the subject simultaneously, separately, or sequentially in combination with the compound according to one aspect, a stereoisomer, hydrate, solvate or pharmaceutically acceptable salt thereof.

The dosage, administration method, administration cycle, etc. in the above method are the same as described above with respect to the pharmaceutical composition.

In another aspect, there is provided the compound according to one aspect, or a stereoisomer, hydrate, solvate or pharmaceutically acceptable salt thereof, for use in the prevention or treatment of cancer.

In another aspect, there is provided a pharmaceutical composition comprising the compound according to one aspect, or a stereoisomer, hydrate, solvate or pharmaceutically acceptable salt thereof, for use in the prevention or treatment of cancer.

In another aspect, there is provided a use of the compound according to one aspect, or a stereoisomer, hydrate, solvate or pharmaceutically acceptable salt thereof, for the manufacture of a medicament for preventing or treating cancer.

The compound, stereoisomer, hydrate, solvate, salt, cancer, prevention and treatment are the same as described above.

### Effects of Invention

According to the compound according to one aspect, a use thereof for inhibiting checkpoint kinase 2 (Chk2), and a use thereof for preventing or treating cancer, the compound of the present invention has high Chk2 selectivity, and thus it can overcome resistance to a PARP inhibitor and toxicity associated with the use thereof, and can be effectively used for the prevention or treatment of cancer.

### Brief Description of Drawings

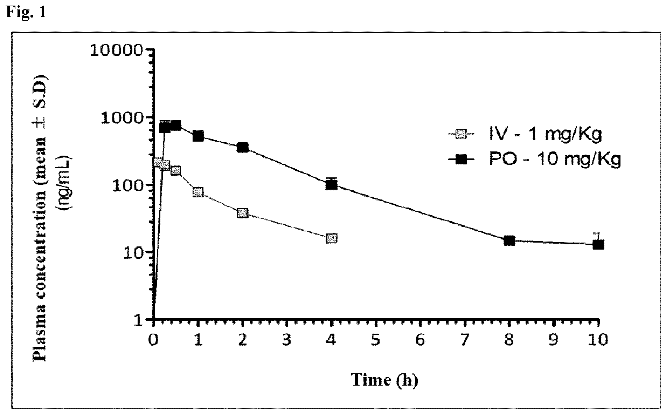

Fig. 1 is a graph showing the average blood concentration (ng/mL) of the compound of Example 9 over time (h) upon intravenous administration (IV) or oral administration (PO) in mice.

### Best Mode for Carrying out the Invention

Hereinafter, the present invention will be described in more detail by way of examples. However, the following examples are only for illustrating the present invention, and the scope of the present invention is not limited to the following examples.

### Preparation Example 1: Preparation of (S)-4-azaspiro[2.5]octan-6-amine

### Step 1: Preparation of tert-butyl (S)-benzyl(1-benzyl-6-oxopiperidin-3-yl)carbamate

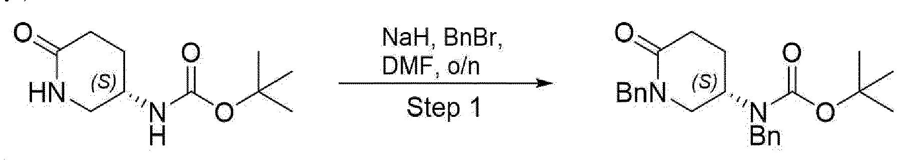

Tert-butyl (S)-(6-oxopiperidin-3-yl)carbamate (9 g, 42 mmol) was dissolved in DMF (90 mL), and then sodium hydride (1.5 g, 63 mmol) was added in small portions at 0 °C. The mixture was stirred for 30 minutes, and then benzyl bromide (8 mL, 67 mmol) was added, and the mixture was stirred at room temperature for 15 hours. Water (500 mL) and ethyl acetate (EA) (500 mL) were added to the reaction solution, and then the reaction solution was neutralized to pH 7 by the addition of a 1 M hydrochloric acid aqueous solution. The organic layers were combined, dried over anhydrous magnesium sulfate, and filtered, and then the solvent was removed under reduced pressure. The residue was purified by silica gel column chromatography (EA) to obtain tert-butyl (S)-benzyl(1-benzyl-6-oxopiperidin-3-yl)carbamate (3.3 g, 20%). ¹H NMR (400 MHz, CDCl₃) δ 7.31 - 7.20 (m, 7H), 7.17 - 7.11 (m, 4H), 4.69 - 3.82 (m, 5H), 3.13 (d, *J =* 10.0 Hz, 2H), 2.55 (ddd, *J =* 17.6, 5.8, 2.8 Hz, 1H), 2.49 - 2.38 (m, 1H), 1.98 (td, *J =* 12.2, 5.9 Hz, 1H), 1.86 - 1.76 (m, 1H), 1.37 (s, 9H); MS (ESI) m/z = 215 [M+H]⁺.

### Step 2: Preparation of tert-butyl (S)-benzyl(4-benzyl-4-azaspiro[2.5]octan-6-yl) carbamate

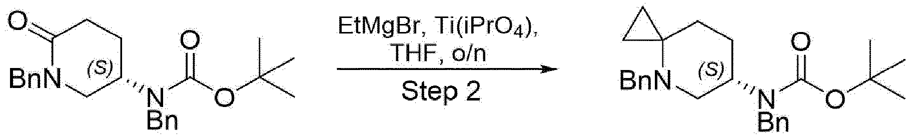

Ethylmagnesium bromide (8.3 mL, 25.1 mmol) was dissolved in THF (100 mL) and then cooled to -78 °C. Titanium isopropoxide (3.22 mL, 10.8 mmol) was added and then stirred for 10 minutes, and then tert-butyl (S)-benzyl(1-benzyl-6-oxopiperidin-3-yl)carbamate (3.3 g, 8.37 mmol) dissolved in THF (10 mL) was added dropwise. After slowly raising the temperature to room temperature, the mixture was refluxed at 70 °C for 15 hours. The reaction was terminated by the addition of a 10% (v/v) sodium hydroxide aqueous solution. The mixture was extracted by the addition of THF (30 mL), dried over anhydrous magnesium sulfate, and filtered, and then the solvent was removed under reduced pressure. The residue was purified by silica gel column chromatography (EA) to obtain tert-butyl (S)-benzyl(4-benzyl-4-azaspiro[2.5]octan-6-yl)carbamate (2.38 g, 70%). MS (ESI) m/z = 407 [M+H]⁺.

### Step 3: Preparation of (S)-4-azaspiro[2.5]octan-6-amine

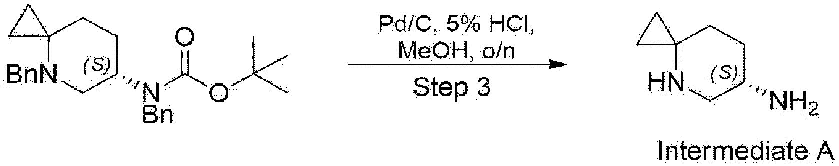

tert-butyl (S)-benzyl(4-benzyl-4-azaspiro[2.5]octan-6-yl)carbamate (2.38 g, 5.86 mmol) was dissolved in MeOH (30 mL), and then a 5% hydrochloric acid aqueous solution (57.3 mL, 0.12 mmol) was added. Pd/C (238 mg, 2.24 mmol) was added, and then the mixture was stirred under a hydrogen balloon at room temperature for 15 hours. After the reaction was completed, it was filtered through Celite, and then the filtrate was concentrated under reduced pressure to obtain Intermediate A (628 mg, 85%). ¹H NMR (400 MHz, DMSO-*D*₆) δ 8.49 (s, 2H), 3.46 (d, *J =* 9.9 Hz, 2H), 2.93 (d, *J =* 11.9 Hz, 1H), 2.04 (dt, *J =* 12.0, 7.3 Hz, 2H), 1.68 (qd, *J =* 12.4, 3.9 Hz, 1H), 1.40 - 1.31 (m, 1H), 1.01 (ddt, *J* = 22.5, 11.5, 5.6 Hz, 2H), 0.69 (ddt, *J =* 51.5, 10.4, 5.7 Hz, 2H); MS (ESI) m/z = 127 [M+H]⁺.

### Example 1: Preparation of (S)-3-amino-6-(5-fluoro-2-(1-(2,2,2-trifluoroethyl)-1H- pyrazol-4-yl)pyridin-4-yl)-N-(piperidin-3-yl)pyrazine-2-carboxamide

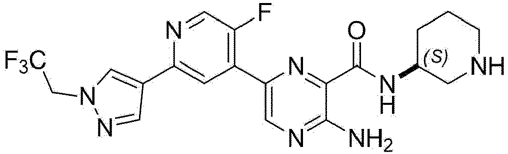

### Step 1: Preparation of methyl 3-amino-6-(4,4,5,5-tetramethyl-1,3,2-dioxaborolan-2-yl) pyrazine-2-carboxylate

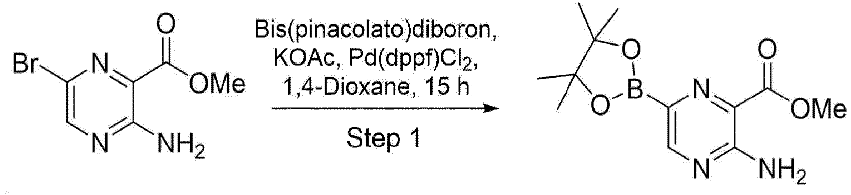

Methyl 3-amino-bromopyrazine-2-carboxylate (5 g, 21.5 mmol) was dissolved in dioxane (70 mL), and then PdCl₂(dppf)·DCM (176 mg, 0.21 mmol), bis(pinacolato)diboron (7.11 g, 28.0 mmol), and potassium acetate (3.81 g, 38.8 mmol) were added, and argon bubbling was performed for 10 minutes. Thereafter, it was refluxed for 15 hours. The mixture was filtered through a Celite filter, and then the residue was washed with n-hexane (200 mL). The filtrate was concentrated under reduced pressure to obtain a residue. After adding ethyl acetate (7 mL) to the residue, and then the solid was filtered to obtain methyl 3-amino-6-(4,4,5,5-tetramethyl-1,3,2-dioxaborolan-2-yl)pyrazine-2-carboxylate (4.15 g, 69%) as a brown powder. ¹H NMR (400 MHz, DMSO-*d*₆): δ 8.31 (s, 1H), 7.44 (s, 2H), 3.82 (s, 3H), 1.20 (s, 12H); MS (ESI) m/z = 197 [M+H]⁺.

### Step 2: Preparation of 3-amino-6-(2-chloro-5-fluoropyridin-4-yl)pyrazine-2-carboxylic acid

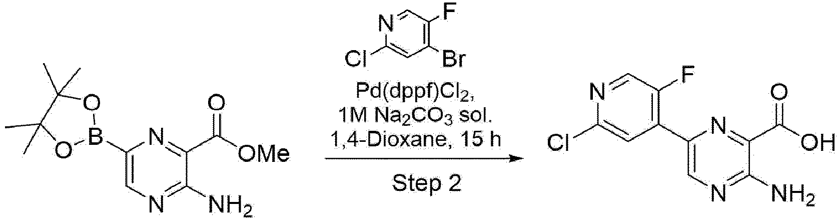

Methyl 3-amino-6-(4,4,5,5-tetramethyl-1,3,2-dioxaborolan-2-yl)pyrazine-2-carboxylate (219 mg, 0.78 mmol) was dissolved in dioxane (5 mL), and then PdCl₂(dppf)·DCM (8.4 mg, 0.05 mmol), 4-bromo-2-chloro-5-fluoropyridine (150 mg, 0.71 mmol), a 1M sodium carbonate aqueous solution (2.14 mL, 2.14 mmol) were added, and argon bubbling was performed for 10 minutes. Thereafter, it was refluxed for 15 hours. The mixture was concentrated under reduced pressure to obtain a residue. After adding water (40 mL) to the residue, it was extracted with EA (40 mL). The water layers were combined and acidified to pH 3 by the addition of a 1 M hydrochloric acid aqueous solution and then extracted with EA (40 mL*2). The organic layer was washed with a sodium chloride aqueous solution (40 mL), then dried over anhydrous magnesium sulfate, and filtered, and then the solvent was removed under reduced pressure and used in step 3 without purification process (yield 89%). ¹H NMR (400 MHz, METHANOL-*D*₄) δ 8.80 - 8.79 (d, *J=* 1.8 Hz, 1H), 8.35 - 8.34 (d, *J=* 3.0 Hz, 1H), 8.23 - 8.22 (d, *J =* 5.7 Hz, 1H), 8.20 - 8.19 (d, *J* = 2.4 Hz, 1H), 7.85 - 7.84 (d, *J =* 2.4 Hz, 1H); MS (ESI) m/z = 269 [M+H]⁺.

### Step 3: Preparation of 3-amino-6-(5-fluoro-2-(1-(2,2,2-trifluoroethyl)-1H-pyrazol-4-yl)pyridin-4-yl)pyrazine-2-carboxylic acid

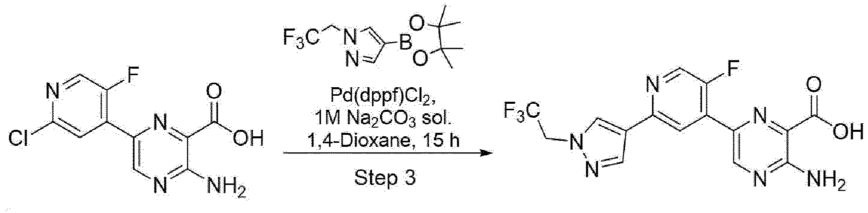

3-amino-6-(2-chloro-5-fluoropyridin-4-yl)pyrazine-2-carboxylic acid (168 mg, 0.63 mmol) was dissolved in dioxane (3 mL), and then PdCl₂(dppf)·DCM (33.7 mg, 0.04 mmol), 4-(4,4,5,5-tetramethyl-1,3,2-dioxaborolan-2-yl)-1-(2,2,2-trifluoroethyl)-1H-pyrazole (190 mg, 0.69 mmol), and a 1 M sodium carbonate aqueous solution (1.88 mL, 1.88 mmol) were added, and argon bubbling was performed for 10 minutes. Thereafter, it was refluxed for 15 hours. The mixture was concentrated under reduced pressure to obtain a residue. After adding water (30 mL) to the residue, it was extracted with EA (30 mL). The water layers were combined and acidified to pH 3 by the addition of a 1 M hydrochloric acid aqueous solution and then extracted with EA (30 mL*2). The organic layer was washed with a sodium chloride aqueous solution (30 mL), and then dried over anhydrous magnesium sulfate, and filtered, and then the solvent was removed under reduced pressure and used in step 4 without purification process (yield 58%). ¹H NMR (400 MHz, DMSO-*d*₆) δ 8.76 (s, 1H), 8.58 (s, 1H), 8.40 (s, 1H), 8.22 (m, 1H), 8.14 (s, 1H), 7.85 (s, 1H), 7.80 (bs, 2H), 5.22 - 5.15 (q, *J =* 9.1 Hz, 2H); MS (ESI) m/z = 393 [M+H]⁺.

### Step 4: Preparation of tert-butyl (S)-3-(3-amino-6-(5-fluoro-2-(1-(2,2,2-trifluoroethyl)-1H-pyrazol-4-yl)pyridin-4-yl)pyrazine-2-carboxamido)piperidine-1-carboxylate

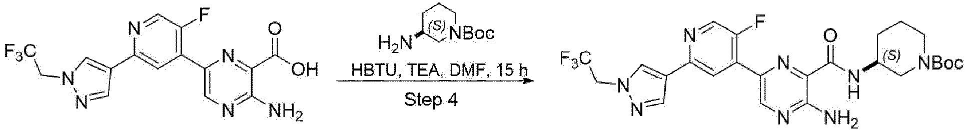

3-amino-6-(5-fluoro-2-(1-(2,2,2-trifluoroethyl)-1H-pyrazol-4-yl)pyridin-4-yl)pyrazine-2-carboxylic acid (33.5 mg, 0.09 mmol) was dissolved in*N*,*N-*dimethylformamide (1 mL), and triethylamine (14.7 µL, 0.11 mmol), HBTU (39.9 mg, 0.11 mmol), and tert-butyl (S)-3-aminopiperidine-I-carboxylate (17.6 mg, 0.09 mmol) were added and stirred at room temperature for 15 hours. After the reaction was completed, the mixture was concentrated under reduced pressure to obtain a residue. After adding water (20 mL) to the residue, it was extracted with EA (20 mL*2), and then dried over anhydrous sodium sulfate, and filtered. The solvent was removed under reduced pressure, and then the residue was purified by silica gel column chromatography (50% EA in n-hexane) to obtain tert-butyl (S)-3-(3-amino-6-(5-fluoro-2-(1-(2,2,2-trifluoroethyl)-1H-pyrazol-4-yl)pyridin-4-yl)pyrazine-2-carboxamido)piperidine-1-carboxylate (38 mg, 77%). ¹H NMR (400 MHz, CDCl₃) δ 8.79 (d, *J =* 1.5 Hz, 1H), 8.48 (d, *J* = 2.9 Hz, 1H), 8.28 (s, 1H), 8.16 (s, 1H), 8.10 (d, *J =* 2.4 Hz, 1H), 7.75 (d, *J =* 2.4 Hz, 1H), 4.81 (s, 2H), 3.85 - 3.80 (m, 1H), 3.40 (d, *J=* 13.8 Hz, 1H), 3.16 (t, *J=* 11.2 Hz, 1H), 1.88 (q, *J= 4.3* Hz, 2H), 1.76 - 1.52 (m, 4H), 1.22 (d, *J =* 2.0 Hz, 9H); MS (ESI) m/z = 565 [M+H]⁺.

### Step 5: Preparation of (S)-3-amino-6-(5-fluoro-2-(1-(2,2,2-trifluoroethyl)-1H-pyrazol-4-yl)pyridin-4-yl)-N-(piperidin-3-yl)pyrazine-2-carboxamide

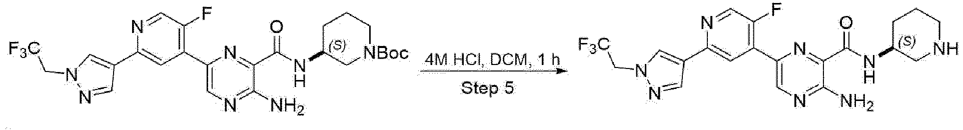

tert-butyl (S)-3-(3-amino-6-(5-fluoro-2-(1-(2,2,2-trifluoroethyl)-1H-pyrazol-4-yl)pyridin-4-yl)pyrazine-2-carboxamido)piperidine-1-carboxylate (37 mg, 0.07 mmol) was dissolved in dichloromethane (1 mL), and then 4 M HCl (0.33 mL, 1.3 mmol) was added, and the mixture was reacted at room temperature for 1 hour. After the reaction was completed, the mixture was concentrated under reduced pressure to obtain a residue, which was purified by preparative column chromatography to obtain the compound of Example 1 (20 mg, 66%) as a yellow powder. ¹H NMR (400 MHz, METHANOL-*D*₄) δ 8.75 (s, 1H), 8.64 (s, 1H), 8.48 (s, 1H), 8.32 (s, 1H), 8.28 - 8.26 (d, *J =* 6.3 Hz, 1H), 8.16 (s, 1H), 5.04 - 4.98 (q, *J =* 8.7 Hz, 2H), 4.28 (bs, 1H), 3.50 (m, 1H), 3.34 (m, 1H), 3.05 (m, 1H), 2.96 (m, 1H), 2.10 (m, 2H), 1.86 (m, 2H); MS (ESI) m/z = 465 [M+H]⁺.

### Examples 2 to 41

The compounds of Examples 2 to 41 were prepared in the same manner as in Example 1 using starting materials corresponding to structures of target compounds shown in Table 3 below. However, when Intermediate A was used in step 4, it was purified by preparative column chromatography in step 4, and the target compound was obtained directly without step 5.

**[Table 1]**

| Example No. | Chemical structure | Compound name |
|---|---|---|
| 2 | 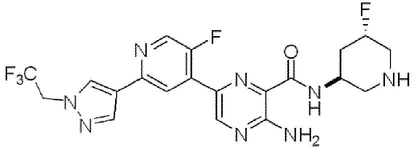 | 3-amino-6-(5-fluoro-2-(1-(2,2,2-trifluoroethyl)-1H-pyrazol-4-yl)pyridin-4-yl)-N-((3 S,5 S)-5-fluoropiperidin-3-yl)pyrazine-2-carboxamide |
| 3 | 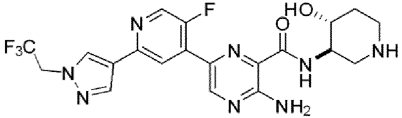 | 3-amino-6-(5-fluoro-2-(1-(2,2,2-trifluoroethyl)-1H-pyrazol-4-yl)pyridin-4-yl)-N-((3R,4R)-4-hydroxypiperidin-3-yl)pyrazine-2-carboxamide |
| 4 | 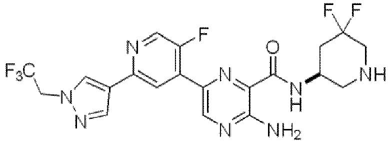 | (S)-3-amino-N-(5,5-difluoropiperidin-3-yl)-6-(5-fluoro-2-(1-(2,2,2-trifluoroethyl)-1H-pyrazol-4-yl)pyridin-4-yl)pyrazine-2-carboxamide |
| 5 | 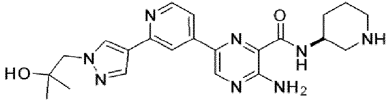 | (S)-3-amino-6-(2-(1-(2-hydroxy-2-methylpropyl)-1H-pyrazol-4-yl)pyridin-4-yl)-N-(piperidin-3-yl) pyrazine-2-carboxamide |
| 6 | 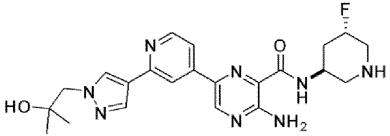 | 3-amino-N-((3S,5S)-5-fluoropiperidin-3-yl)-6-(2-(1-(2-hydroxy-2-methylpropyl)-1H-pyrazol-4-yl)pyridin-4-yl)pyrazine-2-carboxamide |
| 7 | 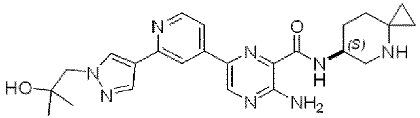 | (S)-3-amino-6-(2-(1-(2-hydroxy-2-methylpropyl)-1H-pyrazol-4-yl)pyridin-4-yl)-N-(4-azaspiro[2.5] octan-6-yl)pyrazine-2-carboxamide |
| 8 | 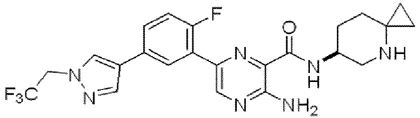 | (S)-3-amino-6-(2-fluoro-5-(1-(2,2,2-trifluoroethyl)-1H-pyrazol-4-yl)phenyl)-N-(4-azaspiro[2.5]octan-6-yl)pyrazine-2-carboxamide |
| 9 | 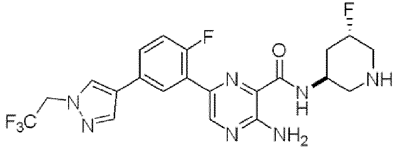 | 3-amino-6-(2-fluoro-5-(1-(2,2,2-trifluoroethyl)-1H-pyrazol-4-yl)phenyl)-N-((3 S,5 S)-5-fluoropiperidin-3-yl)pyrazine-2-carboxamide |
| 10 | 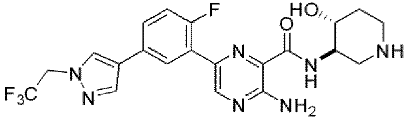 | 3-amino-6-(2-fluoro-5-(1-(2,2,2-trifluoroethyl)-1H-pyrazol-4-yl)phenyl)-N-((3R,4R)-4-hydroxypiperidin-3-yl)pyrazine-2-carboxamide |
| 11 | 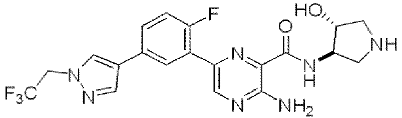 | 3-amino-6-(2-fluoro-5-(1-(2,2,2-trifluoroethyl)-1H-pyrazol-4-yl)phenyl)-N-((3R,4R)-4-hydroxypyrrolidin-3-yl)pyrazine-2-carboxamide |
| 12 | 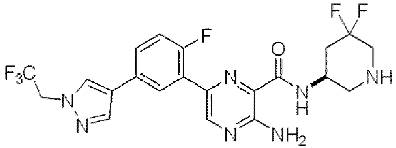 | (S)-3-amino-N-(5,5-difluoropiperidin-3-yl)-6-(2-fluoro-5-(1-(2,2,2-trifluoroethyl)-1H-pyrazol-4-yl)phenyl)pyrazine-2-carboxamide |
| 13 | 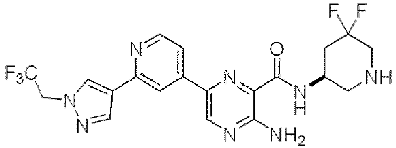 | (S)-3-amino-N-(5,5-difluoropiperidin-3-yl)-6-(2-(1-(2,2,2-trifluoroethyl)-1H-pyrazol-4-yl)pyridin-4-yl)pyrazine-2-carboxamide |
| 14 | 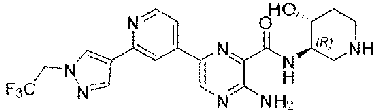 | 3-amino-N-((3R,4R)-4-hydroxypiperidin-3-yl)-6-(2-(1-(2,2,2-trifluoroethyl)-1H-pyrazol-4-yl)pyridin-4-yl)pyrazine-2-carboxamide |
| 15 | 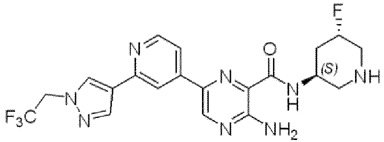 | 3-amino-N-((3S,5S)-5-fluoropiperidin-3-yl)-6-(2-(1-(2,2,2-trifluoroethyl)-1H-pyrazol-4-yl)pyridin-4-yl)pyrazine-2-carboxamide |
| 16 | 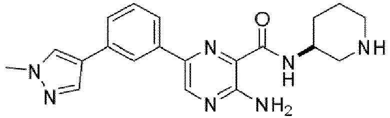 | (S)-3-amino-6-(3-(1-methyl-1H-pyrazol-4-yl)phenyl)-N-(piperidin-3-yl)pyrazine-2-carboxamide |
| 17 | 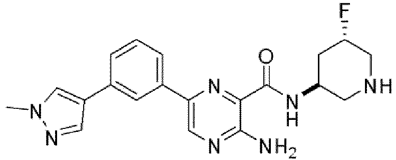 | 3-amino-N-((3S,5S)-5-fluoropiperidin-3-yl)-6-(3-(1-methyl-1H-pyrazol-4-yl)phenyl)pyrazine-2-carboxamide |
| 18 | 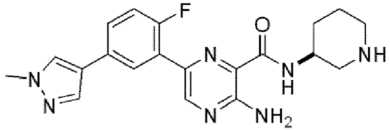 | (S)-3-amino-6-(2-fluoro-5-(1-methyl-1H-pyrazol-4-yl)phenyl)-N-(piperidin-3-yl)pyrazine-2-carboxamide |
| 19 | 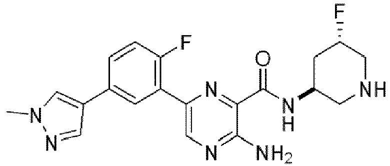 | 3-amino-6-(2-fluoro-5-(1-methyl-1H-pyrazol-4-yl)phenyl)-N-((3 S,5 S)-5-fluoropiperidin-3-yl)pyrazine-2-carboxamide |
| 20 | 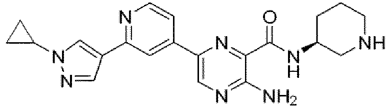 | (S)-3-amino-6-(2-(1-cyclopropyl-1H-pyrazol-4-yl)pyridin-4-yl)-N-(piperidin-3-yl)pyrazine-2-carboxamide |
| 21 | 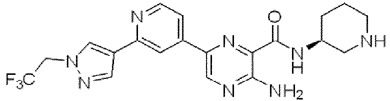 | (S)-3-amino-N-(piperidin-3-yl)-6-(2-(1-(2,2,2-trifluoroethyl)-1H-pyrazol-4-yl)pyridin-4-yl)pyrazine-2-carboxamide |
| 22 | 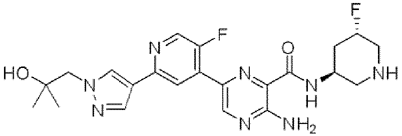 | 3-amino-6-(5-fluoro-2-(1-(2-hydroxy-2-methylpropyl)-1H-pyrazol-4-yl)pyridin-4-yl)-N-((3 S,5 S)-5-fluoropiperidin-3-yl)pyrazine-2-carboxamide |
| 23 | 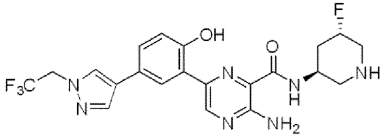 | 3-amino-N-((3S,5S)-5-fluoropiperidin-3-yl)-6-(2-hydroxy-5-(1-(2,2,2-trifluoroethyl)-1H-pyrazol-4-yl)phenyl)pyrazine-2-carboxamide |
| 24 | 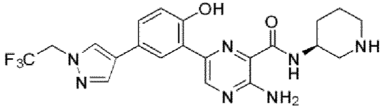 | (S)-3-amino-6-(2-hydroxy-5-(1-(2,2,2-trifluoroethyl)-1H-pyrazol-4-yl)phenyl)-N-(piperidin-3-yl)pyrazine-2-carboxamide |
| 25 | 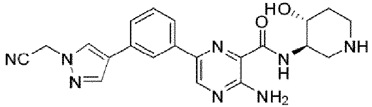 | 3-amino-6-(3-(1-(cyanomethyl)-1H-pyrazol-4-yl)phenyl)-N-((3R,4R)-4-hydroxypiperidin-3-yl)pyrazine-2-carboxamide |
| 26 | 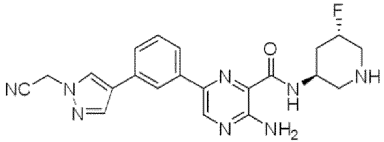 | 3-amino-6-(3-(1-(cyanomethyl)-1H-pyrazol-4-yl)phenyl)-N-((3 S,5 S)-5-fluoropiperidin-3-yl)pyrazine-2-carboxamide |
| 27 | 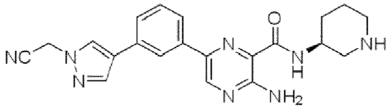 | (S)-3-amino-6-(3-(1-(cyanomethyl)-1H-pyrazol-4-yl)phenyl)-N-(piperidin-3-yl)pyrazine-2-carboxamide |
| 28 | 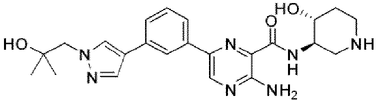 | 3-amino-6-(3-(1-(2-hydroxy-2-methylpropyl)-1H-pyrazol-4-yl)phenyl)-N-((3R,4R)-4-hydroxypiperidin-3-yl)pyrazine-2-carboxamide |
| 29 | 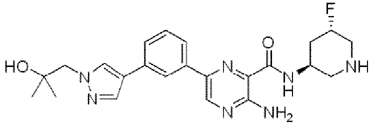 | 3-amino-N-((3S,5S)-5-fluoropiperidin-3-yl)-6-(3-(1-(2-hydroxy-2-methylpropyl)-1H-pyrazol-4-yl)phenyl)pyrazine-2-carboxamide |
| 30 | 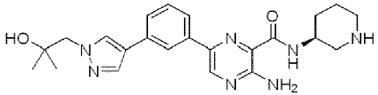 | (S)-3-amino-6-(3-(1-(2-hydroxy-2-methylpropyl)-1H-pyrazol-4-yl)phenyl)-N-(piperidin-3-yl)pyrazine-2-carboxamide |
| 31 | 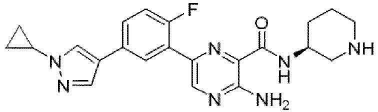 | (S)-3-amino-6-(5-(1-cyclopropyl-1H-pyrazol-4-yl)-2-fluorophenyl)-N-(piperidin-3-yl)pyrazine-2-carboxamide |
| 32 | 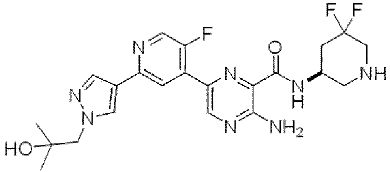 | (S)-3-amino-N-(5,5-difluoropiperidin-3-yl)-6-(5-fluoro-2-(1-(2-hydroxy-2-methylpropyl)-1H-pyrazol-4-yl)pyridin-4-yl)pyrazine-2-carboxamide |
| 33 | 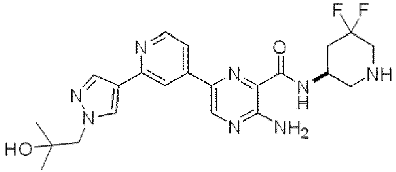 | (S)-3-amino-N-(5,5-difluoropiperidin-3-yl)-6-(2-(1-(2-hydroxy-2-methylpropyl)-1H-pyrazol-4-yl)pyridin-4-yl)pyrazine-2-carboxamide |
| 34 | 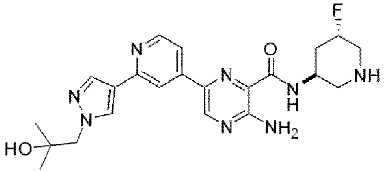 | 3-amino-N-((3S,5S)-5-fluoropiperidin-3-yl)-6-(2-(1-(2-hydroxy-2-methylpropyl)-1H-pyrazol-4-yl)pyridin-4-yl)pyrazine-2-carboxamide |
| 35 | 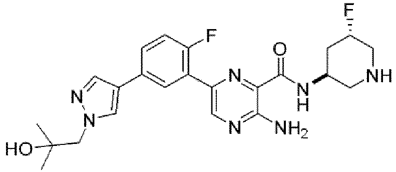 | 3-amino-6-(2-fluoro-5-(1-(2-hydroxy-2-methylpropyl)-1H-pyrazol-4-yl)phenyl)-N-((3 S,5 S)-5-fluoropiperidin-3-yl)pyrazine-2-carboxamide |
| 36 | 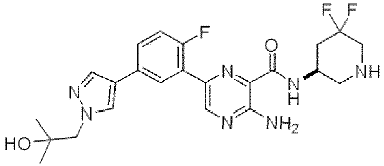 | (S)-3-amino-N-(5,5-difluoropiperidin-3-yl)-6-(2-fluoro-5-(1-(2-hydroxy-2-methylpropyl)-1H-pyrazol-4-yl)phenyl)pyrazine-2-carboxamide |
| 37 | 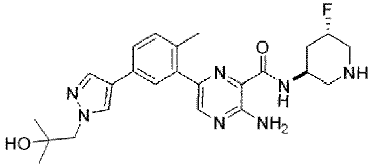 | 3-amino-N-((3 S,5 S)-5-fluoropiperidin-3-yl)-6-(5-(1-(2-hydroxy-2-methylpropyl)-1H-pyrazol-4-yl)-2-methylphenyl)pyrazine-2-carboxamide |
| 38 | 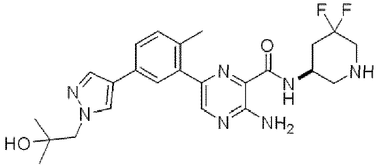 | (S)-3-amino-N-(5,5-difluoropiperidin-3-yl)-6-(5-(1-(2-hydroxy-2-methylpropyl)-1H-pyrazol-4-yl)-2-methylphenyl)pyrazine-2-carboxamide |
| 39 | 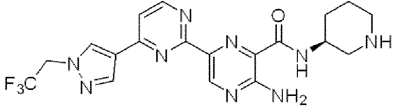 | (S)-3-amino-N-(piperidin-3-yl)-6-(4-(1-(2,2,2-trifluoroethyl)-1H-pyrazol-4-yl)pyrimidin-2-yl)pyrazine-2-carboxamide |
| 40 | 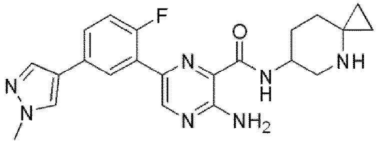 | 3-amino-6-(2-fluoro-5-(1-methyl-1H-pyrazol-4-yl)phenyl)-N-(4-azaspiro[2.5]octan-6-yl)pyrazine-2-carboxamide |
| 41 | 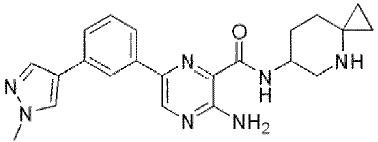 | 3-amino-6-(3-(1-methyl-1H-pyrazol-4-yl)phenyl)-N-(4-azaspiro[2.5]octan-6-yl)pyrazine-2-carboxamide |

**[Table 2]**

| Example No. | LC/MS (ESI) m/z: [M+H]⁺ | NMR |
|---|---|---|
| 2 | 483 | ¹H NMR (400 MHz, METHANOL-*D*₄) δ 8.75 (s, 1H), 8.73 - 8.71 (d, *J =* 8.2 Hz, 1H), 8.48 (s, 1H), 8.31 (s, 1H), 8.27 - 8.25 (d, *J =* 6.2 Hz, 1H), 8.17 (s, 1H), 5.29 - 5.18 (d, *J =* 45.0 Hz, 1H), 5.04 - 4.98 (q, *J =* 8.7, 2H), 4.64 (m, 1H), 3.63 (m, 1H), 3.55 (m, 1H), 3.12 (m, 2H), 2.47 (bs, 1H), 2.13 (m, 1H). |
| 3 | 481 | ¹H NMR (400 MHz, METHANOL-*D*₄) δ 8.76 (s, 1H), 8.68 - 8.66 (d, *J =* 8.2 Hz, 1H), 8.48 (s, 1H), 8.32 (s, 1H), 8.30 - 8.28 (d, *J=* 6.2 Hz, 1H), 8.18 (s, 1H), 5.04 - 4.98 (q, *J =* 8.7 Hz, 2H), 4.12 (m, 1H), 3.98 (m, 1H), 3.59 (m, 1H), 3.46 (m, 2H), 3.09 (m, 1H), 2.26 (m, 1H), 1.82 (m, 1H). |
| 4 | 501 | ¹H NMR (400 MHz, METHANOL-*D*₄) δ 8.80 - 8.79 (d, *J =* 7.0 Hz, 1H), 8.79 (s, 1H), 8.49 (s, 1H), 8.31 (s, 1H), 8.28 - 8.26 (d, *J =* 6.3 Hz, 1H), 8.16 (s, 1H), 5.03 - 4.97 (q, *J =* 8.6 Hz, 2H), 4.63 (m, 1H), 3.72 (m, 1H), 3.57 (m, 2H), 3.35 (m, 1H), 2.59 (m, 2H). |
| 5 | 437 | ¹H NMR (400 MHz, DMSO-d₆) δ 9.17 (s, 1H), 8.81 - 8.79 (d, *J =* 8.4 Hz, 1H), 8.77 - 8.74 (d, *J =* 11.1 Hz, 1H), 8.64 - 8.61 (d, *J =* 10.8 Hz, 1H), 8.59 - 8.58 (d, *J =* 5.6 Hz, 1H), 8.46 (s, 1H), 8.42 (s, 1H), 8.21 (s, 1H), 8.11 (s, 1H), 7.99 (bs, 1H), 4.20 (m, 1H), 4.06 (s, 2H), 3.32 - 3.22 (dd, *J =* 29.3, 12.0 Hz, 2H), 3.06 - 2.98 (q, *J =* 10.8 Hz, 1H), 2.79 (m, 1H), 1.89 (m, 2H), 1.73 (m, 2H), 1.08 (s, 6H). |
| 6 | 455 | ¹H NMR (400 MHz, DMSO-d₆) δ 9.27 (bs, 1H), 9.17 (s, 1H), 9.08 (bs, 1H), 8.92 - 8.89 (d, *J =* 8.7 Hz, 1H), 8.59 - 8.57 (d, *J =* 5.6 Hz, 1H), 8.45 (s, 1H), 8.41 (s, 1H), 8.21 (s, 1H), 8.12 - 8.11 (d, *J =* 5.6 Hz, 1H), 7.98 (bs, 1H), 5.28 - 5.17 (d, *J =* 45.2 Hz, 1H), 4.47 (m, 1H), 4.06 (s, 2H), 3.57 - 3.51 (d, *J =* 13.1 Hz, 1H), 3.32 - 3.29 (d, *J =* 11.8 Hz, 1H), 3.23 - 3.20 (d, *J =* 11.8 Hz, 1H), 3.11 (m, 1H), 2.19 (m, 1H), 2.06 (m, 1H), 1.07 (s, 6H). |
| 7 | 463 | ¹H NMR (400 MHz, DMSO-d₆) δ 9.40 (bs, 1H), 9.21 (s, 1H), 8.94 - 8.91 (d, *J =* 11.0 Hz, 1H), 8.89 - 8.87 (d, *J =* 8.6 Hz, 1H), 8.62 - 8.61 (d, *J =* 5.8 Hz, 1H), 8.53 (s, 1H), 8.49 (s, 1H), 8.24 (m, 2H), 8.10 (bs, 1H), 4.29 (m, 1H), 4.08 (s, 2H), 3.34 (m, 1H), 3.16 (m, 1H), 2.09 (m, 1H), 1.94 (m, 1H), 1.42 (m, 1H), 1.03 (s, 6H), 0.97 (m, 2H), 0.78 (m, 2H). |
| 8 | 490 | ¹H NMR (400 MHz, DMSO-d₆) δ 9.27 (bs, 1H), 8.77 (m, 2H), 8.63 - 8.62 (d, *J =* 2.3 Hz, 1H), 8.31 (s, 1H), 8.14 (m, 1H), 8.09 (s, 1H), 7.74 (bs, 1H), 7.64 (m, 1H), 7.33 (m, 1H), 5.17 - 5.10 (q, *J =* 9.1, 2H), 4.28 (m, 1H), 3.33 (m, 1H), 3.14 (m, 1H), 2.04 (m, 1H), 1.91 (m, 2H), 1.43 (m, 1H), 0.83 (m, 4H). |
| 9 | 482 | ¹H NMR (400 MHz, DMSO-d₆) δ 9.20 (bs, 1H), 9.09 (bs, 1H), 8.80 - 8.78 (d, *J =* 8.6 Hz, 1H), 8.61 (s, 1H), 8.19 (m, 2H), 7.65 (m, 2H), 7.32 (m, 1H), 5.25 (bs, 1H), 5.16 - 5.09 (q, *J =* 9.1, 2H), 4.45 (m, 1H), 3.52 (m, 1H), 3.25 (m, 1H), 3.08 (m, 2H), 2.24 (m, 1H), 2.05 (m, 1H). |
| 10 | 480 | ¹H NMR (400 MHz, DMSO-d₆) δ 8.62 (m, 4H), 8.30 (s, 1H), 8.12 (m, 1H), 8.08 (s, 1H), 7.71 (bs, 1H), 7.63 (m, 1H), 7.33 (m, 1H), 5.16 - 5.09 (q, *J =* 9.2 Hz, 2H), 4.01 (m, 1H), 3.78 (m, 1H), 3.51 (m, 1H), 3.13 (m, 1H), 2.98 (m, 1H), 2.86 (m, 1H), 2.00 (m, 1H), 1.63 (m, 1H). |
| 11 | 466 | ¹H NMR (400 MHz, DMSO-d₆) δ 9.15 (bs, 1H), 8.80 (bs, 1H), 8.80 - 8.78 (d, *J =* 7.1 Hz, 1H), 8.63 - 8.62 (d, *J* = 2.3 Hz, 1H), 8.31 (s, 1H), 8.13 (m, 1H), 8.08 (s, 1H), 7.69 (bs, 1H), 7.63 (m, 1H), 7.32 (m, 1H), 5.16 - 5.09 (q, *J= 9.1,* 2H), 4.37 (m, 2H), 3.55 (m, 2H), 3.31 (m, 1H), 3.10 (m, 1H). |
| 12 | 500 | ¹H NMR (400 MHz, METHANOL-*D*₄) δ 8.75 - 8.73 (d, *J =* 7.9 Hz, 1H), 8.64 (m, 1H), 8.13 (s, 1H), 8.09 (m, 1H), 7.98 (s, 1H), 7.59 (m, 1H), 7.23 (m, 1H), 4.98 - 4.92 (q, *J =* 8.7 Hz, 2H), 4.62 (m, 1H), 3.68 (m, 1H), 3.52 (m, 1H), 3.32 (m, 1H), 2.60 (m, 1H), 2.48 (m, 1H). |
| 13 | 483 | ¹H NMR (400 MHz, METHANOL-*D*₄) δ 9.06 - 9.05 (d, *J =* 2.3 Hz, 1H), 8.91 - 8.89 (d, *J =* 8.0 Hz, 1H), 8.57 (m, 3H), 8.32 - 8.31 (d, *J =* 2.3 Hz, 1H), 8.23 (m, 1H), 5.11 - 5.05 (q, *J =* 8.6 Hz, 2H), 4.63 (m, 1H), 3.73 (m, 1H), 3.56 (m, 2H), 3.34 (m, 1H), 2.57 (m, 2H). |
| 14 | 463 | ¹H NMR (400 MHz, DMSO-*D*₆) δ 9.14 (s, 1H), 8.74 (s, 1H), 8.66 (t, *J* = 9.6 Hz, 2H), 8.60 (d, *J =* 5.4 Hz, 1H), 8.52 (s, 1H), 8.43 (d, *J =* 1.7 Hz, 1H), 8.31 (s, 1H), 8.08 (dd, *J =* 5.4, 1.7 Hz, 1H), 5.22 (q, *J =* 9.1 Hz, 2H), 4.04 (qd, *J* = 9.2, 4.3 Hz, 1H), 3.84 (dt, *J* = 9.7, 5.0 Hz, 1H), 3.33 - 3.25 (m, 2H), 3.03 (q, *J* = 10.9 Hz, 1H), 2.88 (q, *J* = 9.2, 6.3 Hz, 1H), 2.07 - 2.00 (m, 1H), 1.68 - 1.54 (m, 1H). |
| 15 | 465 | ¹H NMR (400 MHz, DMSO-*D*₆) δ 9.24 (bs, 1H), 9.13 (s, 1H), 9.08 (bs, 1H), 8.89 - 8.87 (d, *J* = 8.7 Hz, 1H), 8.60 - 8.59 (d, *J* = 5.4 Hz, 1H), 8.51 (s, 1H), 8.42 - 8.41 (d, *J =* 1.6 Hz, 1H), 8.31 (s, 1H), 8.08 (m, 1H), 7.93 (bs, 1H), 5.22 (m, 3H), 4.47 (m, 1H), 3.30 (m, 1H), 3.20 (m, 1H), 3.10 (m, 2H), 2.16 (m, 2H). |
| 16 | 378 | ¹H NMR (400 MHz, DMSO-*D*₆) δ 8.93 (s, 1H), 8.65 (m, 3H), 8.21 (m, 2H), 7.97 (s, 1H), 7.95 (m, 1H), 7.61 (bs, 1H), 7.54 (m, 1H), 7.42 (m, 1H), 4.20 (m, 1H), 3.85 (s, 3H), 3.31 - 3.20 (dd, *J =* 30.9, 12.0 Hz, 1H), 3.08 - 2.99 (q, *J* = 10.7 Hz, 1H), 2.77 (m, 1H), 1.88 (m, 2H), 1.73 (m, 2H). |
| 17 | 396 | ¹H NMR (400 MHz, DMSO-*D*₆) δ 9.24 (bs, 1H), 9.05 (bs, 1H), 8.94 (s, 1H), 8.79 - 8.77 (d, *J* = 8.7 Hz, 1H), 8.21 (s, 1H), 8.20 (s, 1H), 7.98 (s, 1H), 7.96 (m, 1H), 7.61 (bs, 1H), 7.54 (m, 1H), 7.42 (m, 1H), 5.27 - 5.16 (d, *J =* 45.3 Hz, 1H), 4.48 (m, 1H), 3.85 (s, 3H), 3.51 (m, 1H), 3.30 (m, 1H), 3.15 (m, 2H), 2.15 (m, 2H). |
| 18 | 396 | ¹H NMR (400 MHz, METHANOL-*D*₄) δ 8.58 - 8.57 (d, *J =* 2.3 Hz, 1H), 8.08 (m, 2H), 7.96 - 7.95 (d, *J =* 4.4 Hz, 1H), 7.58 (m, 1H), 7.22 (m, 1H), 4.28 (m, 1H), 3.95 (s, 3H), 3.48 (m, 1H), 3.32 (m, 1H), 3.06 (m, 1H), 2.97 (m, 1H), 2.05 (m, 2H), 1.85 (m, 2H). |
| 19 | 414 | ¹H NMR (400 MHz, DMSO-*D*₆) δ 9.19 (s, 1H), 9.04 (d, *J =* 10.7 Hz, 1H), 8.78 (d, *J* = 8.7 Hz, 1H), 8.60 (d, *J =* 2.4 Hz, 1H), 8.14 (s, 1H), 8.04 (dd, *J* = 7.6, 2.4 Hz, 1H), 7.90 (s, 1H), 7.69 (s, 1H), 7.57 (ddd, *J =* 8.5, 4.7, 2.4 Hz, 1H), 7.29 (dd, *J =* 11.1, 8.5 Hz, 1H), 5.20 (d, *J =* 45.2 Hz, 1H), 4.51 - 4.38 (m, 1H), 3.83 (s, 4H), 3.49 (d, *J =* 12.5 Hz, 1H), 3.32 - 3.01 (m, 3H), 2.24 (s, 1H), 2.14 - 1.93 (m, 1H). |
| 20 | 405 | ¹H NMR (400 MHz, DMSO-*D*₆) δ 9.14 (s, 1H), 8.78 (m, 2H), 8.64 - 8.62 (d, *J* = 10.1 Hz, 1H), 8.59 - 8.58 (d, *J =* 5.6 Hz, 1H), 8.52 (s, 1H), 8.43 (s, 1H), 8.16 (s, 1H), 8.12 - 8.10 (d, *J =* 5.6 Hz, 1H), 4.18 (m, 2H), 3.32 - 3.22 (dd, *J* = 27.7, 12.2 Hz, 2H), 3.02 (m, 1H), 2.79 (m, 1H), 1.90 (m, 2H), 1.80 (m, 2H), 1.06 (m, 2H), 1.01 (m, 2H). |
| 21 | 447 | ¹H NMR (400 MHz, DMSO-*D*₆) δ 9.14 (s, 1H), 8.79 (m, 2H), 8.63 (m, 2H), 8.53 (s, 1H), 8.45 (s, 1H), 8.33 (s, 1H), 8.11 (m, 1H), 5.26 - 5.20 (q, *J=* 9.1 Hz, 2H), 4.18 (m, 1H), 3.32 - 3.22 (dd, *J =* 28.5, 12.2 Hz, 2H), 3.03 (m, 1H), 2.78 (m, 1H), 1.86 (m, 2H), 1.72 (m, 2H). |
| 22 | 473 | ¹H NMR (400 MHz, METHANOL-*D*₄) δ 8.94 (s, 1H), 8.55 (dd, *J* = 5.4, 0.8 Hz, 1H), 8.35 - 8.28 (m, 1H), 8.17 (d, *J* = 0.8 Hz, 1H), 7.89 (dd, *J* = 5.4, 1.7 Hz, 1H), 4.40 (m 1H), 4.20 (s, 2H), 3.19 - 3.03 (m, 3H), 2.91 (dd, *J =* 13.2, 7.0 Hz, 1H), 2.56 - 2.21 (m, 3H), 1.26 (s, 6H). |
| 23 | 480 | ¹H NMR (400 MHz, METHANOL-*D*₄) δ 8.81 (s, 1H), 8.73 - 8.71 (d, *J =* 8.2 Hz, 2H), 8.56 (s, 1H), 8.31 (s, 1H), 7.78 - 7.71 (d, *J* = 6.1 Hz, 1H), 7.21 - 7.18 (d, *J* = 5.3 Hz, 1H), 4.94 - 4.81 (q, *J* = 7.6 Hz, 2H), 4.03 - 3.87 (m, 1H), 3.63 - 3.51 (m, 1H), 3.40 - 2.69 (m, 4H), 2.08 - 1.67 (m, 2H). |
| 24 | 462 | ¹H NMR (400 MHz, METHANOL-*D*₄) δ 8.82 (s, 1H), 8.74 - 8.71 (d, *J =* 8.2 Hz, 2H), 8.46 (s, 1H), 8.22 (s, 1H), 7.80 - 7.69 (d, *J=* 7.4 Hz, 1H), 7.25 - 7.19 (d, *J* = 6.2 Hz, 1H), 4.94 - 4.81 (m, 2H), 3.81 - 3.70 (m, 1H), 3.63 - 2.69 (m, 4H), 1.98 - 1.78 (m, 2H), 1.68 - 1.42 (m, 2H). |
| 25 | 419 | ¹H NMR (400 MHz, METHANOL-*D*₄) δ 8.86 (s, 1H), 8.50 (d, *J =* 6.4 Hz, 2H), 8.33 (s, 1H), 8.20 (s, 1H), 7.77 - 7.64 (m, 2H), 4.85 - 4.80 (m, 2H), 4.27 - 4.24 (m, 1H) 3.70 - 3.63 (m, 1H), 3.00 - 2.92 (m, 2H), 2.84 - 2.69 (m, 4H), 1.78 - 1.53 (m, 2H). |
| 26 | 421 | ¹H NMR (400 MHz, METHANOL-*D*₄) δ 8.86 (s, 1H), 8.51 - 8.49 (m, 2H), 8.32 (s, 1H), 8.19 (s, 1H), 7.78 - 7.71 (m, 2H), 4.85 - 4.80 (q, *J =* 9.6 Hz, 2H), 3.63 - 3.51 (m, 3H), 3.47 - 2.85 (m, 4H), 2.18 - 1.88 (m, 1H). |
| 27 | 403 | ¹H NMR (400 MHz, METHANOL-*D*₄) δ 8.81 (s, 1H), 8.54 - 8.48 (m, 2H), 8.33 (s, 1H), 8.20 (s, 1H), 7.75 - 7.70 (m, 2H), 4.86 - 4.82 (q, *J =* 7.7 Hz, 2H), 3.74 - 3.66 (m, 1H), 3.12 - 2.85 (m, 2H), 2.78 - 2.63 (m, 2H), 1.82 - 1.63 (m, 4H). |
| 28 | 452 | ¹H NMR (400 MHz, METHANOL-*D*₄) δ 8.90 (s, 1H), 8.53 (dd, *J* = 6.4, 1.3 Hz, 2H), 8.34 - 8.20 (m, 1H), 8.14 (d, *J* = 1.8 Hz, 1H), 7.89 - 7.63 (m, 1H), 4.42 (m 1H), 3.89 - 3.75 (m, 2H), 3.64 (tt, *J =* 13.1, 7.8 Hz, 1H), 3.16 - 3.03 (m, 4H), 1.86 - 1.64 (m, 4H), 1.25 (s, 6H). |
| 29 | 454 | ¹H NMR (400 MHz, METHANOL-*D*₄) δ 8.84 (s, 1H), 8.51 (dd, *J* = 5.4, 2.3 Hz, 2H), 8.24 - 8.21 (m, 1H), 8.06 (d, *J =* 1.9 Hz, 1H), 7.86 - 7.60 (m, 1H), 3.80 - 3.76 (m, 2H), 3.60 - 3.12 (m, 4H), 3.10 - 2.88 (m, 2H), 1.86 - 1.64 (m, 2H), 1.23 (s, 6H). |
| 30 | 436 | ¹H NMR (400 MHz, METHANOL-*D*₄) δ 8.81 (s, 1H), 8.57 - 8.50 (m, 2H), 8.20 - 8.18 (m, 1H), 8.04 (d, *J =* 2.0 Hz, 1H), 7.87 - 7.66 (m, 1H), 3.81 - 3.78 (m, 2H), 3.60 - 3.55 (m, 1H), 3.15 - 2.93 (m, 4H), 1.89 - 1.60 (m, 4H), 1.26 (s, 6H). |
| 31 | 422 | ¹H NMR (400 MHz, METHANOL-*D*₄) δ 9.10 (s, 1H), 8.73 - 8.61 (m, 1H), 8.37 (s, 1H), 8.24 (s, 1H), 7.97 - 7.83 (m, 2H), 3.72 - 3.64 (m, 1H), 3.60 - 3.13 (m, 4H), 2.76 - 2.55 (m, 1H), 1.90 - 1.54 (m, 4H), 1.28 - 1.12 (m, 4H). |
| 32 | 491 | ¹H NMR (400 MHz, DMSO-*D*₆) δ 8.79 (d, *J =* 1.6 Hz, 1H), 8.75-8.73 (m, 1H), 8.590 (d, *J =* 3.6 Hz, 1H), 8.259 (t, *J =* 6.4 Hz, 2H), 8.04 (s, 1H), 7.95 (bs, 2H), 4.74 (s, 1H), 4.21-4.18 (m, 1H), 4.078 (s, 2H), 2.99-2.91 (m, 4H), 2.83-2.66 (m, 1H), 2.35-2.20 (m, 1H), 1.11 (s, 6H). |
| 33 | 473 | ¹H NMR (400 MHz, DMSO-*D*₆) δ 9.09 (s, 1H), 8.72 (d, *J =* 9.2 Hz, 1H), 8.55 (d, *J =* 5.2 Hz, 1H), 8.30 (s, 2H), 8.11 (s, 1H), 7.92-7.91 (m, 2H), 4.75 (s, 1H), 4.18-4.15 (m, 1H), 4.05 (s, 2H), 3.15-2.82 (m, 3H), 2.71-2.65 (m, 2H), 2.33-2.19 (m, 2H), 1.09 (s, 6H). |
| 34 | 455 | ¹H NMR (400 MHz, DMSO-*D*₆) δ 9.08 (s, 1H), 8.58-8.54 (m, 2H), 8.31 (s, 2H), 8.12 (s, 1H), 7.95-7.89 (m, 2H), 4.86 (s, 1H), 4.75 (s, 1H), 4.17-4.13 (m, 1H), 4.06 (s, 2H), 2.93-2.91 (m, 2H), 2.76-2.61 (m, 3H), 2.10-1.97 (m, 2H), 1.08 (s, 6H). |
| 35 | 472 | ¹H NMR (400 MHz, DMSO-*D*₆) δ 8.64 (d, *J =* 2 Hz, 1H), 8.50 (d, *J =* 8.8 Hz, 1H), 8.149-8.121 (m,2H), 7.94 (s, 1H), 7.83 (s, 1H), 7.65-7.61 (m, 1H), 7.34-7.29 (m, 1H), 4.85 (s, 1H), 4.73 (s, 1H), 4.19-4.09 (m, 1H), 4.04 (s, 2H) 2.96-2.61 (m, 5H), 2.09-1.98 (m, 2H), 1.10 (s, 6H). |
| 36 | 490 | ¹H NMR (400 MHz, DMSO-*D*₆) δ 8.69-8.66 (m, 2H) , 8.14-8.11 (m, 2H), 7.93 (s, 1H), 7.65-7.61 (m, 2H), 7.35-7.30 (m, 1H), 4.73 (s, 1H), 4.19 (m, 1H), 4.04 (s, 2H), 2.95-2.89 (m, 3H), 2.69-2.67 (m, 2H), 2.33-2.25 (m, 2H), 1.10 (s, 6H). |
| 37 | 468 | ¹H NMR (400 MHz, DMSO-*D*₆) δ 8.44 (s, 1H), 8.33 (d, *J =* 9.2 Hz, 1H), 8.09 (s, 1H), 7.88 (s, 1H), 7.64-7.48 (m, 3H), 7.28-7.26 (m, 1H), 4.76-7.64 (m, 2H), 4.13-4.00 (m, 3H), 2.86-2.65 (m, 4H), 2.58-2.48 (m, 1H), 2.32 (s, 3H), 2.05-1.94 (m, 2H), 1.07 (s, 6H). |
| 38 | 486 | ¹H NMR (400 MHz, DMSO-*D*₆) δ 8.50 (d, *J =* 9.2 Hz, 1H), 8.45 (s, 1H) , 8.09 (s, 1H), 7.88 (s, 1H), 7.64-7.60 (m, 2H), 7.50-7.48 (m, 1H), 7.27 (d, *J =* 8 Hz 1H), 4.70 (s, 1H), 4.14 (s, 1H), 4.00 (s, 2H), 2.95-2.75 (m, 4H), 2.65-2.59 (m, 1H), 2.32 (s, 3H), 2.23-2.10 (m, 2H), 1.07 (s, 6H). |
| 39 | 448 | ¹H NMR (400 MHz, METHANOL-*D*₄) δ 9.59 (d, *J =* 7.4 Hz, 1H), 9.42 (s, 1H), 8.80 (d, *J =* 5.5 Hz, 1H), 8.67 (s, 1H), 8.35 (s, 1H), 7.69 (d, *J* = 5.5 Hz, 1H), 5.06 (q, *J =* 8.6 Hz, 2H), 4.26 (s, 1H), 3.52 (dd, *J* = 12.3, 4.1 Hz, 1H), 3.37 - 3.31 (m, 1H), 3.01 (t, *J =* 11.2 Hz, 2H), 2.12 (dd, *J* = 9.4, 5.3 Hz, 2H), 1.85 (q, *J =* 10.1, 9.6 Hz, 2H). |
| 40 | 422 | ¹H NMR (400 MHz, DMSO-*D*₆) δ 9.26 (t, *J =* 10.5 Hz, 1H), 8.95 (s, 1H), 8.74 (dd, *J =* 17.3, 10.0 Hz, 2H), 8.22 (d, *J =* 2.2 Hz, 2H), 8.02 - 7.95 (m, 2H), 7.69 - 7.56 (m, 1H), 7.54 (dt, *J* = 7.8, 1.4 Hz, 1H), 7.43 (t, *J* = 7.7 Hz, 1H), 4.29 (s, 2H), 3.85 (s, 3H), 3.34 (d, *J =* 11.9 Hz, 1H), 3.18 (q, *J =* 10.6 Hz, 1H), 2.11 - 2.03 (m, 1H), 1.93 (dd, *J* = 9.5, 3.8 Hz, 2H), 1.42 (d, *J=* 14.0 Hz, 1H), 0.95 (ddd, *J =* 25.8, 10.9, 5.2 Hz, 2H), 0.77 (ddd, *J =* 24.2, 9.7, 4.9 Hz, 2H). |
| 41 | 404 | ¹H NMR (400 MHz, DMSO-*D*₆) δ 9.27 (d, *J =* 10.8 Hz, 1H), 8.97 (s, 1H), 8.77 (d, *J =* 8.7 Hz, 2H), 8.34 (dd, *J =* 7.4, 2.4 Hz, 1H), 8.20 (d, *J* = 2.4 Hz, 1H), 8.08 - 8.02 (m, 2H), 7.64 (s, 1H), 7.33 (dd, *J* = 11.0, 8.7 Hz, 1H), 4.29 (s, 1H), 3.88 (s, 4H), 3.33 (d, *J =* 11.9 Hz, 1H), 3.17 (q, *J =* 10.7 Hz, 1H), 2.11 - 2.02 (m, 1H), 1.91 (d, *J* = 4.2 Hz, 2H), 1.42 (d, *J =* 14.1 Hz, 1H), 1.03 - 0.89 (m, 2H), 0.85 - 0.70 (m, 2H). |

**[Table 3]**

| Example No. | Starting material used |
|---|---|
| 2 | Step 4: tert-butyl (3S,5S)-3-amino-5-fluoropiperidine-1-carboxylate |
| 3 | Step 4: tert-butyl trans-3-amino-4-hydroxy-piperidine-1-carboxylate |
| 4 | Step 4: tert-butyl (5S)-5-amino-3,3-difluoropiperidine-1-carboxylate |
| 5 | Step 2: 4-bromo-2-chloropyridine |
| | Step 3: [1-(2-hydroxy-2-methyl-propyl)pyrazol-4-yl]boronic acid pinacol ester |
| 6 | Step 2: 4-bromo-2-chloropyridine |
| | Step 3: [1-(2-hydroxy-2-methyl-propyl)pyrazol-4-yl]boronic acid pinacol ester |
| | Step 4: tert-butyl (3S,5S)-3-amino-5-fluoropiperidine-1-carboxylate |
| 7 | Step 2: 4-bromo-2-chloropyridine |
| | Step 3: [1-(2-hydroxy-2-methyl-propyl)pyrazol-4-yl]boronic acid pinacol ester |
| | Step 4: Intermediate A |
| 8 | Step 2: 2,4-dibromo-1-fluorobenzene |
| | Step 4: Intermediate A |
| 9 | Step 2: 2,4-dibromo-1-fluorobenzene |
| | Step 4: tert-butyl (3S,5S)-3-amino-5-fluoropiperidine-1-carboxylate |
| 10 | Step 2: 2,4-dibromo-1-fluorobenzene |
| | Step 4: tert-butyl trans-3-amino-4-hydroxy-piperidine-1-carboxylate |
| 11 | Step 2: 2,4-dibromo-1-fluorobenzene |
| | Step 4: tert-butyl (3R,4R)-3-amino-4-hydroxypyrrolidine-1-carboxylate |
| 12 | Step 2: 2,4-dibromo-1-fluorobenzene |
| | Step 4: tert-butyl (5S)-5-amino-3,3-difluoropiperidine-1-carboxylate |
| 13 | Step 2: 4-bromo-2-chloropyridine |
| | Step 4: tert-butyl (5S)-5-amino-3,3-difluoropiperidine-1-carboxylate |
| 14 | Step 2: 4-bromo-2-chloropyridine |
| | Step 4: tert-butyl trans-3-amino-4-hydroxy-piperidine-1-carboxylate |
| 15 | Step 2: 4-bromo-2-chloropyridine |
| | Step 4: tert-butyl (3S,5S)-3-amino-5-fluoropiperidine-1-carboxylate |
| 16 | Step 2: 1,3-dibromobenzene |
| | Step 3: 1-methyl-4-(4,4,5,5-tetramethyl-1,3,2-dioxaborolan-2-yl)pyrazole |
| 17 | Step 2: 1,3-dibromobenzene |
| | Step 3: 1-methyl-4-(4,4,5,5-tetramethyl-1,3,2-dioxaborolan-2-yl)pyrazole |
| | Step 4: tert-butyl (3S,5S)-3-amino-5-fluoropiperidine-1-carboxylate |
| 18 | Step 2: 2,4-dibromo-1-fluorobenzene |
| | Step 3: 1-methyl-4-(4,4,5,5-tetramethyl-1,3,2-dioxaborolan-2-yl)pyrazole |
| 19 | Step 2: 2,4-dibromo-1-fluorobenzene |
| | Step 3: 1-methyl-4-(4,4,5,5-tetramethyl-1,3,2-dioxaborolan-2-yl)pyrazole |
| | Step 4: tert-butyl (3S,5S)-3-amino-5-fluoropiperidine-1-carboxylate |
| 20 | Step 2: 4-bromo-2-chloropyridine |
| | Step 3: 1-cyclo-4-(4,4,5,5-tetramethyl-1,3,2-dioxaborolan-2-yl)-1H-pyrazole |
| 21 | Step 2: 4-bromo-2-chloropyridine |
| 22 | Step 3: [1-(2-hydroxy-2-methyl-propyl)pyrazol-4-yl]boronic acid pinacol ester |
| | Step 4: tert-butyl (3S,5S)-3-amino-5-fluoropiperidine-1-carboxylate |
| 23 | Step 2: 2-bromo-4-chlorophenol |
| | Step 4: tert-butyl (3S,5S)-3-amino-5-fluoropiperidine-1-carboxylate |
| 24 | Step 2: 2-bromo-4-chlorophenol |
| 25 | Step 2: 1,3-dibromobenzene |
| | Step 3: 2-(4-(4,4,5,5-tetramethyl-1,3,2-dioxaborolan-2-yl)-1H-pyrazol-1-yl)acetonitrile |
| | Step 4: tert-butyl trans-3-amino-4-hydroxy-piperidine-1-carboxylate |
| 26 | Step 2: 1,3-dibromobenzene |
| | Step 3: 2-(4-(4,4,5,5-tetramethyl-1,3,2-dioxaborolan-2-yl)-1H-pyrazol-1-yl)acetonitrile |
| | Step 4: tert-butyl (3S,5S)-3-amino-5-fluoropiperidine-1-carboxylate |
| 27 | Step 2: 1,3-dibromobenzene |
| | Step 3: 2-(4-(4,4,5,5-tetramethyl-1,3,2-dioxaborolan-2-yl)-1H-pyrazol-1-yl)acetonitrile |
| 28 | Step 2: 1,3-dibromobenzene |
| | Step 3: [1-(2-hydroxy-2-methyl-propyl)pyrazol-4-yl]boronic acid pinacol ester |
| | Step 4: tert-butyl trans-3-amino-4-hydroxy-piperidine-1-carboxylate |
| 29 | Step 2: 1,3-dibromobenzene |
| | Step 3: [1-(2-hydroxy-2-methyl-propyl)pyrazol-4-yl]boronic acid pinacol ester |
| | Step 4: tert-butyl (3S,5S)-3-amino-5-fluoropiperidine-1-carboxylate |
| 30 | Step 2: 1,3-dibromobenzene |
| | Step 3: [1-(2-hydroxy-2-methyl-propyl)pyrazol-4-yl]boronic acid pinacol ester |
| 31 | Step 2: 2,4-dibromo-1-fluorobenzene |
| | Step 3: 1-cyclo-4-(4,4,5,5-tetramethyl-1,3,2-dioxaborolan-2-yl)-1H-pyrazole |
| 32 | Step 3: [1-(2-hydroxy-2-methyl-propyl)pyrazol-4-yl]boronic acid pinacol ester |
| | Step 4: tert-butyl (5S)-5-amino-3,3-difluoropiperidine-1-carboxylate |
| 33 | Step 2: 4-bromo-2-chloropyridine |
| | Step 3: [1-(2-hydroxy-2-methyl-propyl)pyrazol-4-yl]boronic acid pinacol ester |
| | Step 4: tert-butyl (5S)-5-amino-3,3-difluoropiperidine-1-carboxylate |
| 34 | Step 2: 4-bromo-2-chloropyridine |
| | Step 3: [1-(2-hydroxy-2-methyl-propyl)pyrazol-4-yl]boronic acid pinacol ester |
| | Step 4: tert-butyl (3S,5S)-3-amino-5-fluoropiperidine-1-carboxylate |
| 35 | Step 2: 2,4-dibromo-1-fluorobenzene |
| | Step 3: [1-(2-hydroxy-2-methyl-propyl)pyrazol-4-yl]boronic acid pinacol ester |
| | Step 4: tert-butyl (3S,5S)-3-amino-5-fluoropiperidine-1-carboxylate |
| 36 | Step 2: 2,4-dibromo-1-fluorobenzene |
| | Step 3: [1-(2-hydroxy-2-methyl-propyl)pyrazol-4-yl]boronic acid pinacol ester |
| | Step 4: tert-butyl (5S)-5-amino-3,3-difluoropiperidine-1-carboxylate |
| 37 | Step 2: 4-bromo-2-iodo-1-methylbenzene |
| | Step 3: [1-(2-hydroxy-2-methyl-propyl)pyrazol-4-yl]boronic acid pinacol ester |
| | Step 4: tert-butyl (3S,5S)-3-amino-5-fluoropiperidine-1-carboxylate |
| 38 | Step 2: 4-bromo-2-iodo-1-methylbenzene |
| | Step 3: [1-(2-hydroxy-2-methyl-propyl)pyrazol-4-yl]boronic acid pinacol ester |
| | Step 4: tert-butyl (5S)-5-amino-3,3-difluoropiperidine-1-carboxylate |
| 39 | Step 2: 2,4-dibromopyrimidine |
| 40 | Step 2: 2,4-dibromo-1-fluorobenzene |
| | Step 4: 4-azaspiro[2.5]octan-6-amine (using racemate) |
| 41 | Step 2: 1,3-dibromobenzene |
| | Step 4: 4-azaspiro[2.5]octan-6-amine (using racemate) |

### Example 42: Preparation of (S)-3-amino-6-(3-(5,5-dimethyl-5,6-dihydro-4H-pyrrolo[1,2-b]pyrazol-3-yl)phenyl)-N-(piperidin-3-yl)pyrazine-2-carboxamide

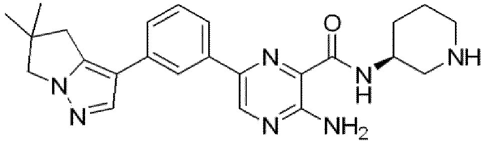

### Step 1: Preparation of methyl 3-amino-6-(3-bromophenyl)pyrazine-2-carboxylate

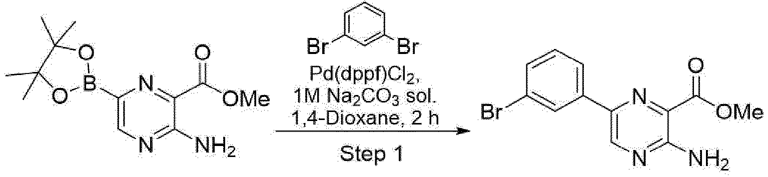

Methyl 3-amino-6-(4,4,5,5-tetramethyl-1,3,2-dioxaborolan-2-yl)pyrazine-2-carboxylate (500 mg, 1.79 mmol) was dissolved in dioxane (10 mL), and then PdCl₂(dppf)·DCM (97 mg, 0.12 mmol), 1,3-dibromobenzene (0.43 mL, 3.58 mmol), and a 1M sodium carbonate aqueous solution (5.37 mL, 5.37 mmol) were added, and argon bubbling was performed for 10 minutes. Thereafter, it was refluxed for 2 hours. The mixture was concentrated under reduced pressure to obtain a residue. After adding water (70 mL) to the residue, it was extracted with EA (70 mL*2). The organic layer was dried over anhydrous magnesium sulfate and filtered, and then the solvent was removed under reduced pressure. The residue was purified by silica gel column chromatography (30% EA in n-hexane) to obtain methyl 3-amino-6-(3-bromophenyl)pyrazine-2-carboxylate (86 mg, 16%). The water layers were combined and acidified to pH 3 by the addition of a 1 M hydrochloric acid aqueous solution and then extracted with EA (70 mL*2). The organic layer was dried over anhydrous magnesium sulfate and filtered, and then the solvent was removed under reduced pressure to obtain 3-amino-6-(3-bromophenyl)pyrazine-2-carboxylic acid (150 mg, 28%) without purification process. ¹H NMR (400 MHz, CDCl₃) δ 8.63 (s, 1H), 8.07 (t, *J =* 1.8 Hz, 1H), 7.81 (ddd, *J =* 7.8, 1.7, 1.0 Hz, 1H), 7.50 (ddd, *J =* 8.0, 2.0, 1.0 Hz, 1H), 7.32 (t, *J =* 7.9 Hz, 1H), 4.01 (s, 3H); MS (ESI) m/z = 309 [M+H]⁺.

### Step 2: Preparation of 3-amino-6-(3-bromophenyl)pyrazine-2-carboxylic acid

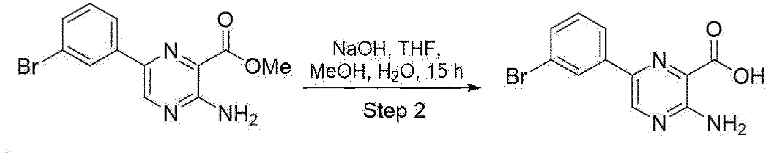

Methyl 3-amino-6-(3-bromophenyl)pyrazine-2-carboxylate (86 mg, 0.28 mmol) was dissolved in methanol (1 mL), tetrahydrofuran (1 mL), and water (0.5 mL), and then sodium hydroxide (33.5 mg, 0.84 mmol) was added and stirred at room temperature for 15 hours. The reaction solution was acidified to pH 2 by the addition of a 1M hydrochloric acid aqueous solution and then extracted with EA. The organic layer was dried over anhydrous magnesium sulfate and filtered, and then the solvent was removed under reduced pressure and used in step 3 without purification process (yield 90%). MS (ESI) m/z = 295 [M+H]⁺.

### Step 3: Preparation of 3-amino-6-(3-(5,5-dimethyl-5,6-dihydro-4H-pyrrolo[1,2-b] pyrazol-3-yl)phenyl)pyrazine-2-carboxylic acid

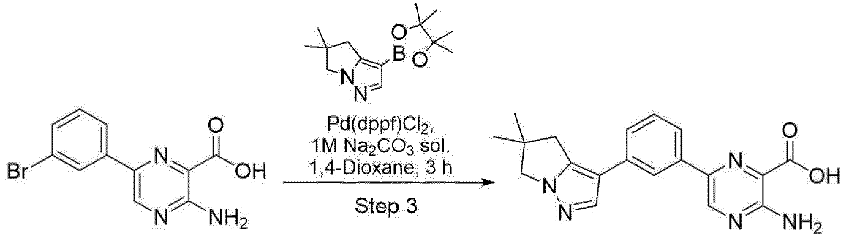

3-amino-6-(3-bromophenyl)pyrazine-2-carboxylic acid (100 mg, 0.34 mmol) was dissolved in dioxane (3 mL), and then PdCl₂(dppf)·DCM (18.3 mg, 0.02 mmol), 5,5-dimethyl-3-(4,4,5,5-tetramethyl-1,3,2-dioxaborolan-2-yl)-5,6-dihydro-4H-pyrrolo[1,2-b]pyrazole (89.1 mg, 0.34 mmol), and a 1 M sodium carbonate aqueous solution (1 mL, 1.00 mmol) were added, and argon bubbling was performed for 10 minutes. Thereafter, it was refluxed for 3 hours. The mixture was concentrated under reduced pressure to obtain a residue. After adding water (30 mL) to the residue, it was extracted with EA (30 mL). The water layers were combined and acidified to pH 3 by the addition of a 1M hydrochloric acid aqueous solution and then extracted with EA (30 mL*2). The organic layer was dried over anhydrous magnesium sulfate and filtered, and then the solvent was removed under reduced pressure and used in step 4 without purification process (yield 84%). ¹H NMR (400 MHz, DMSO-*D*₆) δ 8.91 (s, 1H), 8.09 (s, 1H), 7.96 (s, 1H), 7.86 - 7.80 (m, 1H), 7.56 - 7.38 (m, 5H), 3.85 (s, 2H), 2.94 (s, 2H), 1.24 (s, 6H); MS (ESI) m/z = 350 [M+H]⁺.

### Step 4: Preparation of tert-butyl (S)-3-(3-amino-6-(3-(5,5-dimethyl-5,6-dihydro-4H-pyrrolo[1,2-b]pyrazol-3-yl)phenyl)pyrazine-2-carboxamido)piperidine-1-carboxylate

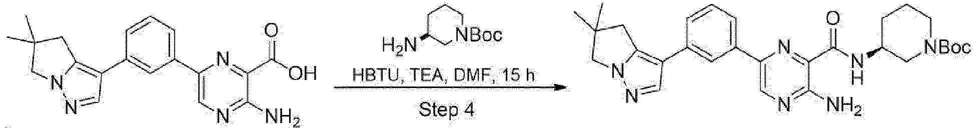

3-amino-6-(3-(5,5-dimethyl-5,6-dihydro-4H-pyrrolo[1,2-b]pyrazol-3-yl)phenyl)pyrazine-2-carboxylic acid (30 mg, 0.09 mmol) was dissolved in *N,N-*dimethylformamide (1 mL), and triethylamine (14 µL, 0.11 mmol), HBTU (39 mg, 0.11 mmol), and tert-butyl (*S*)-3-aminopiperidine-1-carboxylate (17.2 mg, 0.09 mmol) were added and stirred at room temperature for 15 hours. After the reaction was completed, the mixture was concentrated under reduced pressure to obtain a residue. After adding water (30 mL) to the residue, it was extracted with EA (30 mL*2), and then dried over anhydrous sodium sulfate, and filtered. The solvent was removed under reduced pressure, and then the residue was purified by silica gel column chromatography (60% EA in n-hexane) to obtain tert-butyl (S)-3-(3-amino-6-(3-(5,5-dimethyl-5,6-dihydro-4H-pyrrolo[1,2-b]pyrazol-3-yl)phenyl) pyrazine-2-carboxamido)piperidine-1-carboxylate (37 mg, 81%).

¹H NMR (400 MHz, CDCl₃) δ 8.63 (s, 1H), 8.01 (s, 2H), 7.89 (s, 1H), 7.85 (s, 1H), 7.65 (s, 1H), 7.46 - 7.43 (m, 2H), 4.11 - 4.02 (m, 1H), 3.95 (s, 2H), 3.79 (dd, *J =* 13.1, 3.6 Hz, 1H), 3.54 (s, 1H), 3.31 (dd, *J =* 13.0, 7.5 Hz, 2H), 1.73 (s, 4H), 1.62 (s, 2H), 1.36 (d, *J =* 4.6 Hz, 15H); MS (ESI) m/z = 532 [M+H]⁺.

### Step 5: Preparation of (S)-3-amino-6-(3-(5,5-dimethyl-5,6-dihydro-4H-pyrrolo [1,2-b] pyrazol-3-yl)phenyl)-N-(piperidin-3-yl)pyrazine-2-carboxamide

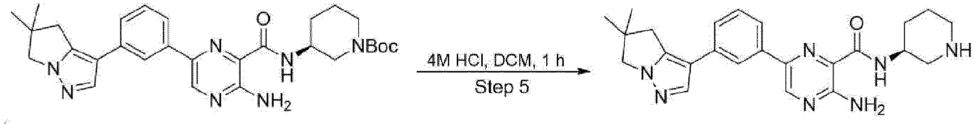

Tert-butyl (S)-3-(3-amino-6-(3-(5,5-dimethyl-5,6-dihydro-4H-pyrrolo[1,2-b]pyrazol-3-yl)phenyl)pyrazine-2-carboxamido)piperidine-1-carboxylate (35 mg, 0.065 mmol) was dissolved in dichloromethane (1 mL), and then 4 M HCl (0.35 mL, 1.3 mmol) was added, and the mixture was reacted at room temperature for 2 hours. After the reaction was completed, the mixture was concentrated under reduced pressure to obtain a residue, which was purified by preparative column chromatography to obtain the compound of Example 42 (10 mg, 33%). ¹H NMR (400 MHz, DMSO-*D*₆) δ 8.90 (s, 1H), 8.70 (d, *J =* 8.6 Hz, 2H), 8.60 (d, *J=* 11.0 Hz, 1H), 8.10 (t, *J =* 1.8 Hz, 1H), 7.99 (s, 1H), 7.89 (dt, *J =* 7.5, 1.6 Hz, 1H), 7.61 (s, 1H), 7.49 - 7.38 (m, 2H), 4.19 (dd, *J =* 10.0, 4.9 Hz, 1H), 3.86 (s, 2H), 3.26 (dd, *J =* 28.5, 11.6 Hz, 2H), 3.04 - 2.91 (m, 3H), 2.75 (d, *J =* 10.9 Hz, 1H), 1.97 - 1.82 (m, 2H), 1.73 (q, *J =* 12.5 Hz, 2H), 1.24 (d, *J=* 2.2 Hz, 6H); MS (ESI) m/z = 432 [M+H]⁺.

### Examples 43 to 66

The compounds of Examples 43 to 66 were prepared in the same manner as in Example 42 using starting materials corresponding to structures of target compounds shown in Table 6 below. However, when Intermediate A was used in step 4, it was purified by preparative column chromatography in step 4, and the target compound was obtained directly without step 5.

**[Table 4]**

| Example No. | Chemical structure | Compound name |
|---|---|---|
| 43 | 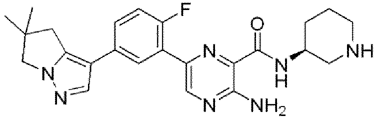 | (S)-3-amino-6-(5-(5,5-dimethyl-5,6-dihydro-4H-pyrrolo[1,2-b]pyrazol-3-yl)-2-fluorophenyl)-N-(piperidin-3-yl)pyrazine-2-carboxamide |
| 44 | 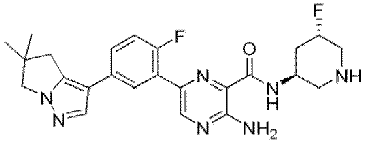 | 3-amino-6-(5-(5,5-dimethyl-5,6-dihydro-4H-pyrrolo[1,2-b]pyrazol-3-yl)-2-fluorophenyl)-N-((3 S,5 S)-5-fluoropiperidin-3-yl)pyrazine-2-carboxamide |
| 45 | 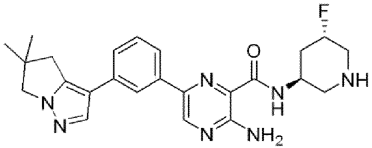 | 3-amino-6-(3-(5,5-dimethyl-5,6-dihydro-4H-pyrrolo[1,2-b]pyrazol-3-yl)phenyl)-N-((3 S,5 S)-5-fluoropiperidin-3-yl)pyrazine-2-carboxamide |
| 46 | 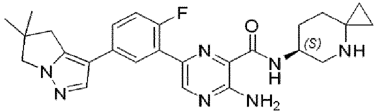 | (S)-3-amino-6-(5-(5,5-dimethyl-5,6-dihydro-4H-pyrrolo[1,2-b]pyrazol-3-yl)-2-fluorophenyl)-N-(4-azaspiro[2.5]octan-6-yl)pyrazine-2-carboxamide |
| 47 | 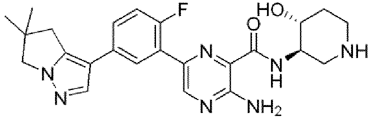 | 3-amino-6-(5-(5,5-dimethyl-5,6-dihydro-4H-pyrrolo[1,2-b]pyrazol-3-yl)-2-fluorophenyl)-N-((3R,4R)-4-hydroxypiperidin-3-yl)pyrazine-2-carboxamide |
| 48 | 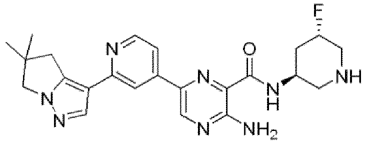 | 3-amino-6-(2-(5,5-dimethyl-5,6-dihydro-4H-pyrrolo[1,2-b]pyrazol-3-yl)pyridin-4-yl)-N-((3S,5S)-5-fluoropiperidin-3-yl)pyrazine-2-carboxamide |
| 49 | 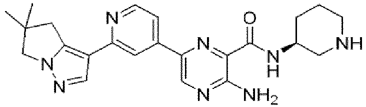 | (S)-3-amino-6-(2-(5,5-dimethyl-5,6-dihydro-4H-pyrrolo[1,2-b]pyrazol-3-yl)pyridin-4-yl)-N-(piperidin-3-yl)pyrazine-2-carboxamide |
| 50 | 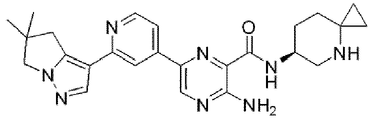 | (S)-3-amino-6-(2-(5,5-dimethyl-5,6-dihydro-4H-pyrrolo[1,2-b]pyrazol-3-yl)pyridin-4-yl)-N-(4-azaspiro[2.5]octan-6-yl)pyrazine-2-carboxamide |
| 51 | 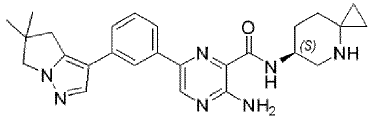 | (S)-3-amino-6-(3-(5,5-dimethyl-5,6-dihydro-4H-pyrrolo[1,2-b]pyrazol-3-yl)phenyl)-N-(4-azaspiro[2.5]octan-6-yl)pyrazine-2-carboxamide |
| 52 | 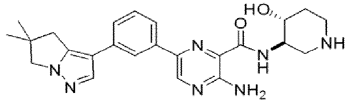 | 3-amino-6-(3-(5,5-dimethyl-5,6-dihydro-4H-pyrrolo[1,2-b]pyrazol-3-yl)phenyl)-N-((3R,4R)-4-hydroxypiperidin-3-yl)pyrazine-2-carboxamide |
| 53 | 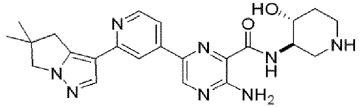 | 3-amino-6-(2-(5,5-dimethyl-5,6-dihydro-4H-pyrrolo[1,2-b]pyrazol-3-yl)pyridin-4-yl)-N-((3R,4R)-4-hydroxypiperidin-3-yl)pyrazine-2-carboxamide |
| 54 | 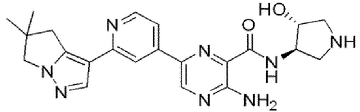 | 3-amino-6-(2-(5,5-dimethyl-5,6-dihydro-4H-pyrrolo[1,2-b]pyrazol-3-yl)pyridin-4-yl)-N-((3R,4R)-4-hydroxypyrrolidin-3-yl)pyrazine-2-carboxamide |
| 55 | 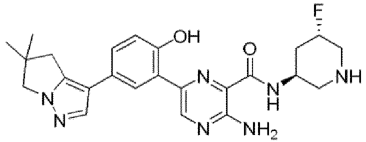 | 3-amino-6-(5-(5,5-dimethyl-5,6-dihydro-4H-pyrrolo[1,2-b]pyrazol-3-yl)-2-hydroxyphenyl)-N-((3 S,5 S)-5-fluoropiperidin-3-yl)pyrazine-2-carboxamide |
| 56 | 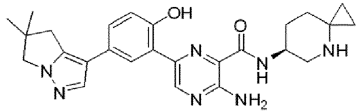 | (S)-3-amino-6-(5-(5,5-dimethyl-5,6-dihydro-4H-pyrrolo[1,2-b]pyrazol-3-yl)-2-hydroxyphenyl)-N-(4-azaspiro[2.5]octan-6-yl)pyrazine-2-carboxamide |
| 57 | 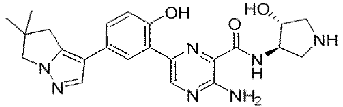 | 3-amino-6-(5-(5,5-dimethyl-5,6-dihydro-4H-pyrrolo[1,2-b]pyrazol-3-yl)-2-hydroxyphenyl)-N-((3R,4R)-4-hydroxypyrrolidin-3-yl)pyrazine-2-carboxamide |
| 58 | 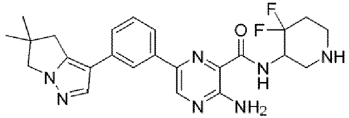 | 3-amino-N-(4,4-difluoropiperidin-3-yl)-6-(3-(5,5-dimethyl-5,6-dihydro-4H-pyrrolo[1,2-b]pyrazol-3-yl)phenyl)pyrazine-2-carboxamide |
| 59 | 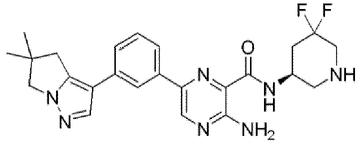 | (S)-3-amino-N-(5,5-difluoropiperidin-3-yl)-6-(3-(5,5-dimethyl-5,6-dihydro-4H-pyrrolo[1,2-b])pyrazol-3-yl)phenyl)pyrazine-2-carboxamide |
| 60 | 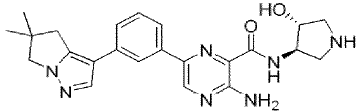 | 3-amino-6-(3-(5,5-dimethyl-5,6-dihydro-4H-pyrrolo[1,2-b]pyrazol-3-yl)phenyl)-N-((3R,4R)-4-hydroxypyrrolidin-3-yl)pyrazine-2-carboxamide |
| 61 | 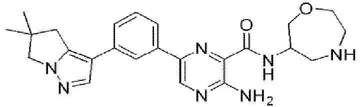 | 3-amino-6-(3-(5,5-dimethyl-5,6-dihydro-4H-pyrrolo[1,2-b]pyrazol-3-yl)phenyl)-N-(1,4-oxazepan-6-yl)pyrazine-2-carboxamide |
| 62 | 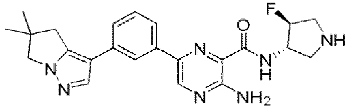 | 3-amino-6-(3-(5,5-dimethyl-5,6-dihydro-4H-pyrrolo[1,2-b]pyrazol-3-yl)phenyl)-N-((3 S,4S)-4-fluoropyrrolidin-3-yl)pyrazine-2-carboxamide |
| 63 | 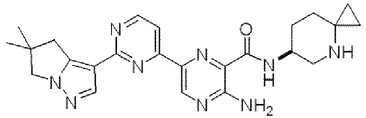 | (S)-3-amino-6-(2-(5,5-dimethyl-5,6-dihydro-4H-pyrrolo[1,2-b]pyrazol-3-yl)pyrimidin-4-yl)-N-(4-azaspiro[2.5]octan-6-yl)pyrazine-2-carboxamide |
| 64 | 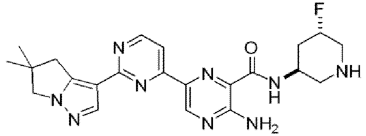 | 3-amino-6-(2-(5,5-dimethyl-5,6-dihydro-4H-pyrrolo[1,2-b]pyrazol-3-yl)pyrimidin-4-yl)-N-((3 S,5 S)-5-fluoropiperidin-3-yl)pyrazine-2-carboxamide |
| 65 | 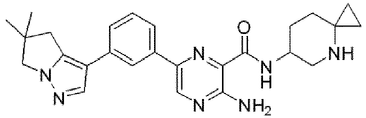 | 3-amino-6-(3-(5,5-dimethyl-5,6-dihydro-4H-pyrrolo[1,2-b]pyrazol-3-yl)phenyl)-N-(4-azaspiro[2.5]octan-6-yl)pyrazine-2-carboxamide |
| 66 | 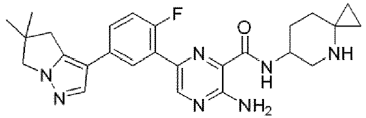 | 3-amino-6-(5-(5,5-dimethyl-5,6-dihydro-4H-pyrrolo[1,2-b]pyrazol-3-yl)-2-fluorophenyl)-N-(4-azaspiro[2.5]octan-6-yl)pyrazine-2-carboxamide |

**[Table 5]**

| Example No. | LC/MS (ESI) m/z: [M+H]⁺ | NMR |
|---|---|---|
| 43 | 450 | ¹H NMR (400 MHz, DMSO-d₆) δ 8.61 (d, *J=* 2.4 Hz, 1H), 8.52 (d, *J=* 7.8 Hz, 1H), 7.96 (dd, *J =* 7.4, 2.4 Hz, 1H), 7.87 (s, 1H), 7.48 (m, 1H), 7.20 (m, 1H), 4.27 (m, 1H), 3.91 (s, 2H), 3.50 (dd, *J =* 12.2, 4.2 Hz, 1H), 3.32 (m, 1H), 2.95 (m, 4H), 2.05 (m, 2H), 1.85 (m, 2H), 1.33 (s, 3H), 1.32 (s, 3H). |
| 44 | 468 | ¹H NMR (400 MHz, DMSO-d₆) δ 8.59 (m, 2H), 7.94 (m, 1H), 7.87 (s, 1H), 7.47 (m, 1H), 7.20 (m, 1H), 5.23 (m, 1H), 4.61 (m, 1H), 3.90 (s, 2H), 3.55 (m, 2H), 3.11 (m, 2H), 2.94 (s, 2H), 2.45 (m, 1H), 2.08 (m, 1H), 1.33 (s, 3H), 1.32 (s, 3H). |
| 45 | 450 | ¹H NMR (400 MHz, DMSO-*D*₆) δ 9.15 (d, *J* = 73.6 Hz, 1H), 8.94 - 8.77 (m, 2H), 8.10 (q, *J =* 1.6 Hz, 1H), 7.99 (s, 1H), 7.91 (dq, *J =* 7.2, 1.5 Hz, 1H), 7.60 (s, 2H), 7.47 - 7.38 (m, 2H), 5.25 (dd, *J =* 45.2, 28.9 Hz, 1H), 4.58 - 4.39 (m, 1H), 3.85 (s, 2H), 3.50 (d, *J =* 11.9 Hz, 1H), 3.30 (d, *J* = 12.1 Hz, 1H), 3.10 (t, *J* = 22.2 Hz, 2H), 2.97 (d, *J* = 4.1 Hz, 2H), 2.85 (s, 1H), 2.32 - 1.99 (m, 2H), 1.25 (s, 6H). |
| 46 | 476 | ¹H NMR (400 MHz, METHANOL-*D*₄) δ 8.61 (d, *J* = 2.4 Hz, 1H), 8.54 (d, *J =* 7.7 Hz, 1H), 7.97 (dd, *J =* 7.4, 2.4 Hz, 1H), 7.87 (s, 1H), 7.49 (ddd, *J* = 8.6, 4.6, 2.4 Hz, 1H), 7.21 (dd, *J =* 11.1, 8.6 Hz, 1H), 4.35 (td, *J =* 10.1, 5.6 Hz, 1H), 3.91 (s, 2H), 3.54 - 3.48 (m, 1H), 3.20 (t, *J =* 11.1 Hz, 1H), 2.96 (d, *J =* 1.6 Hz, 2H), 2.23 - 2.11 (m, 2H), 2.04 - 1.93 (m, 1H), 1.61 (dt, *J =* 15.0, 4.4 Hz, 1H), 1.08 - 0.81 (m, 4H). |
| 47 | 466 | ¹H NMR (400 MHz, DMSO-*D*₆) δ 8.73 (s, 1H), 8.68 - 8.55 (m, 3H), 7.94 (dd, *J =* 7.5, 2.4 Hz, 1H), 7.91 (s, 1H), 7.71 (s, 1H), 7.52 (ddd, *J =* 8.6, 4.7, 2.4 Hz, 1H), 7.29 (dd, *J =* 11.1, 8.5 Hz, 1H), 5.31 (s, 1H), 4.03 (ddt, *J =* 13.4, 9.0, 4.4 Hz, 1H), 3.84 (s, 2H), 3.75 (td, *J =* 9.8, 4.3 Hz, 1H), 3.28 (d, *J* = 14.1 Hz, 2H), 2.95 (d, *J* = 5.9 Hz, 2H), 2.82 (d, *J* = 11.6 Hz, 1H), 2.04 - 1.93 (m, 1H), 1.64 (q, *J* = 10.4 Hz, 1H), 1.23 (d, *J* = 2.6 Hz, 6H). |
| 48 | 451 | ¹H NMR (400 MHz, DMSO-*D*₆) δ 9.33 (s, 1H), 9.10 (s, 2H), 8.90 (d, *J* = 8.7 Hz, 1H), 8.57 (dd, *J =* 5.5, 0.7 Hz, 1H), 8.20 (t, *J =* 1.2 Hz, 1H), 8.17 (s, 1H), 8.04 - 7.89 (m, 2H), 5.22 (d, *J* = 45.1 Hz, 1H), 4.48 (dt, *J* = 8.2, 4.1 Hz, 1H), 3.89 (s, 2H), 3.54 (t, *J =* 12.7 Hz, 1H), 3.32 (d, *J =* 11.0 Hz, 1H), 3.19 (s, 1H), 3.13 - 3.00 (m, 4H), 2.28 (d, *J =* 13.8 Hz, 1H), 2.10 (dt, *J =* 44.5, 13.2 Hz, 1H), 1.25 (s, 6H). |
| 49 | 433 | ¹H NMR (400 MHz, DMSO-*D*₆) δ 9.10 (s, 1H), 8.79 (d, *J =* 8.5 Hz, 2H), 8.66 (d, *J =* 11.2 Hz, 1H), 8.57 (d, *J =* 5.5 Hz, 1H), 8.22 - 8.15 (m, 2H), 8.04 - 7.87 (m, 2H), 4.19 (s, 1H), 3.90 (s, 2H), 3.28 (dd, *J* = 28.9, 12.2 Hz, 2H), 3.13 - 2.90 (m, 3H), 2.76 (d, *J =* 11.4 Hz, 1H), 1.89 (d, *J* = 15.6 Hz, 2H), 1.73 (td, *J =* 11.9, 6.5 Hz, 2H), 1.25 (d, *J =* 2.4 Hz, 6H). |
| 50 | 459 | ¹H NMR (400 MHz, DMSO-*D*₆) δ 9.34 (s, 1H), 9.10 (s, 1H), 8.82 (dd, *J* = 21.2, 9.8 Hz, 2H), 8.58 (d, *J* = 5.5 Hz, 1H), 8.23 - 8.14 (m, 2H), 7.99 (t, *J =* 17.7 Hz, 2H), 4.35 - 4.25 (m, 2H), 3.90 (s, 3H), 3.35 (d, *J =* 11.8 Hz, 1H), 3.17 - 2.99 (m, 3H), 2.12 (q, *J =* 8.0, 4.8 Hz, 1H), 1.94 (d, *J* = 7.8 Hz, 2H), 1.41 (s, 1H), 1.25 (d, *J =* 2.6 Hz, 6H), 1.04 - 0.65 (m, 4H). |
| 51 | 458 | ¹H NMR (400 MHz, DMSO-*D*₆) δ 9.24 (d, *J =* 11.7 Hz, 1H), 8.91 (s, 1H), 8.83 - 8.73 (m, 1H), 8.63 (s, 1H), 8.14 - 8.09 (m, 1H), 7.99 (s, 1H), 7.91 (dt, *J* = 7.6*,* 1.6 Hz, 1H), 7.62 (s, 1H), 7.49 - 7.39 (m, 2H), 4.29 (s, 1H), 3.86 (s, 2H), 3.13 (d, *J* = 10.6 Hz, 1H), 3.04 - 2.92 (m, 2H), 2.64 - 2.58 (m, 1H), 2.10 (s, 1H), 1.93 (s, 2H), 1.40 (d, *J =* 14.0 Hz, 1H), 1.25 (d, *J =* 2.3 Hz, 6H), 0.98 (dd, *J =* 10.6, 5.3 Hz, 1H), 0.90 (d, *J =* 5.8 Hz, 1H), 0.83 - 0.77 (m, 1H), 0.70 (dd, *J =* 9.8, 5.3 Hz, 1H). |
| 52 | 448 | ¹H NMR (400 MHz, DMSO-*D*₆) δ 8.91 (s, 1H), 8.74 (d, *J =* 10.7 Hz, 1H), 8.63 (d, *J =* 10.5 Hz, 1H), 8.57 (d, *J =* 8.5 Hz, 1H), 8.09 (d, *J =* 1.9 Hz, 1H), 7.99 (s, 1H), 7.90 (dt, *J =* 7.4, 1.6 Hz, 1H), 7.62 (s, 1H), 7.48 - 7.39 (m, 2H), 4.06 - 4.03 (m, 1H), 3.85 (s, 2H), 3.80 (d, *J* = 4.3 Hz, 1H), 3.31 (t, *J* = 13.3 Hz, 2H), 3.00 (dd, *J =* 15.1, 5.3 Hz, 3H), 2.89 - 2.79 (m, 1H), 2.07 - 1.97 (m, 1H), 1.68 (d, *J* = 14.2 Hz, 1H), 1.25 (d, *J* = 2.6 Hz, 6H). |
| 53 | 449 | ¹H NMR (400 MHz, DMSO-*D*₆) δ 9.10 (s, 1H), 8.81 (s, 1H), 8.68 (d, *J* = 8.6 Hz, 2H), 8.58 (d, *J =* 5.5 Hz, 1H), 8.21 (d, *J =* 1.7 Hz, 1H), 8.17 (s, 1H), 8.02 (dd, *J =* 5.6, 1.6 Hz, 1H), 4.07 - 4.01 (m, 1H), 3.90 (s, 2H), 3.84 - 3.79 (m, 1H), 3.32 (d, *J =* 12.4 Hz, 2H), 3.08 - 2.94 (m, 3H), 2.85 (d, *J =* 11.6 Hz, 1H), 2.08 - 2.00 (m, 1H), 1.65 (d, *J =* 12.7 Hz, 1H), 1.25 (d, *J =* 3.0 Hz, 6H). |
| 54 | 435 | ¹H NMR (400 MHz, DMSO-*D*₆) δ 9.22 (s, 2H), 9.11 (s, 1H), 8.88 (d, *J* = 7.1 Hz, 1H), 8.57 (d, *J =* 5.6 Hz, 1H), 8.23 - 8.17 (m, 2H), 8.06 (dd, *J =* 5.7, 1.7 Hz, 1H), 7.96 (s, 1H), 4.44 (dt, *J =* 5.3, 2.9 Hz, 1H), 4.36 (dt, *J* = 6.9, 3.4 Hz, 1H), 3.90 (s, 2H), 3.63 - 3.52 (m, 1H), 3.49 - 3.40 (m, 1H), 3.35 (dq, *J =* 11.9, 6.1, 5.4 Hz, 1H), 3.13 (t, *J* = 7.5 Hz, 1H), 3.06 (s, 2H), 1.25 (s, 6H). |
| 55 | 466 | ¹H NMR (400 MHz, METHANOL-*D*₄) δ 8.81 (s, 1H), 7.83 - 7.74 (m, 2H), 7.31 (dd, *J =* 8.4, 2.3 Hz, 1H), 6.92 (d, *J =* 8.4 Hz, 1H), 5.20 (d, *J* = 44.8 Hz, 1H), 4.60 (ddd, *J =* 12.1, 7.8, 4.3 Hz, 1H), 3.88 (s, 2H), 3.65 - 3.50 (m, 2H), 3.27 - 3.14 (m, 1H), 3.04 (t, *J =* 11.9 Hz, 1H), 2.91 (s, 2H), 2.44 (s, 1H), 2.17 - 1.99 (m, 1H), 1.31 (s, 6H). |
| 56 | 474 | ¹H NMR (400 MHz, METHANOL-*D*₄) δ 8.85 (s, 1H), 7.83 - 7.76 (m, 2H), 7.33 (dd, *J =* 8.4, 2.2 Hz, 1H), 6.93 (d, *J =* 8.4 Hz, 1H), 4.33 (s, 1H), 3.88 (s, 2H), 3.51 (d, *J =* 11.9 Hz, 1H), 3.21 (t, *J =* 10.9 Hz, 1H), 2.93 (s, 2H), 2.17 - 2.09 (m, 2H), 2.03 - 1.92 (m, 1H), 1.63 (d, *J =* 13.6 Hz, 1H), 1.32 (s, 7H), 1.09 - 0.95 (m, 2H), 0.89 (ddd, *J =* 25.2, 9.7, 5.8 Hz, 2H). |
| 57 | 450 | ¹H NMR (400 MHz, METHANOL-*D*₄) δ 8.78 (s, 1H), 7.82 - 7.69 (m, 2H), 7.32 (dd, *J =* 8.4, 2.3 Hz, 1H), 6.92 (d, *J =* 8.4 Hz, 1H), 4.86 (s, 1H), 4.53 (dd, *J =* 4.4, 2.2 Hz, 1H), 4.37 (d, *J =* 7.0 Hz, 1H), 3.88 (s, 2H), 3.76 (dd, *J* = 12.5, 6.9 Hz, 1H), 3.59 - 3.49 (m, 2H), 2.91 (s, 2H), 1.32 (s, 7H). |
| 58 | 468 | ¹H NMR (400 MHz, DMSO-*D*₆) δ 8.93 (s, 1H), 8.44 (d, *J* = 9.6 Hz, 1H), 8.05 (s, 1H), 7.96 (s, 1H), 7.82-7.80 (m, 1H), 7.62 (s, 2H), 7.54-7.52 (m, 1H), 7.46-7.42 (m, 1H), 4.44 (m, 1H) 3.88 (s, 2H), 3.22-3.04 (m, 4H), 2.74-2.65 (m, 2H), 2.08 (m, 3H), 1.27 (m, 6H). |
| 59 | 468 | ¹H NMR (400 MHz, DMSO-*D*₆) δ 8.91 (s, 1H), 8.67 (d, *J =* 8.8 Hz, 1H), 8.12 (s, 1H), 8.00 (s, 1H), 7.87 (d, *J* = 8.0 Hz, 1H), 7.62 (bs, 2H), 7.52 - 7.50 (m, 1H), 7.47 - 7.43 (m, 1H), 4.20 - 4.16 (m, 1H), 3.89 (s, 2H), 3.03 - 2.93 (m, 4H), 2.82 - 2.70 (m, 2H), 2.68 - 2.65 (m, 1H), 2.35 - 2.32 (m, 1H), 2.28 - 2.24 (m, 1H), 1.29 (s, 6H). |
| 60 | 434 | ¹H NMR (400 MHz, DMSO-*D*₆) δ 8.89 (s, 1H), 8.53 (d, *J =* 8 Hz, 1H), 8.11 (s, 1H), 8.00 (s, 1H), 7.88 (d, *J =* 8 Hz, 1H), 7.75-7.60 (m, 2H), 7.52 (d, *J =* 8 Hz, 1H), 7.45 (t, *J =* 8 Hz, 1H), 4.15-4.10 (m, 2H), 3.89 (s, 2H), 3.20-3.10 (m, 1H), 3.09-3.05 (m, 1H), 3.03 (s, 2H), 2.72-2.70 (m, 2H), 2.64-2.60 (m, 2H), 1.30 (s, 6H). |
| 61 | 448 | ¹H NMR (400 MHz, DMSO-*D*₆) δ 8.90 (s, 1H), 8.75 - 8.72 (m, 1H), 8.10 (s, 1H), 7.98 (s, 1H), 7.83 (d, *J =* 7.6 Hz, 1H), 7.63 (bs, 2H), 7.51 - 7.44 (m, 2H), 4.20 - 4.15 (m, 1H), 3.99 - 3.88 (m, 3H), 3.75 - 3.66 (m, 3H), 3.06 - 3.00 (m, 3H), 2.95 - 2.82 (m, 4H), 1.29 (s, 6H). |
| 62 | 436 | ¹H NMR (400 MHz, DMSO-*D*₆) δ 8.89 (s, 1H), 8.78 (d, *J =* 4 Hz, 1H), 8.10 (s, 1H), 7.99 (s, 1H), 7.89 (d, *J* = 8 Hz, 1H), 7.60 (bs, 2H), 7.49 (d, *J =* 8 Hz, 1H), 7.43 (t, *J* = 8 Hz, 1H), 5.24 (s, 1H), 5.11 (s, 1H), 4.43-4.35 (m, 1H), 3.87 (s, 2H), 3.18-3.00 (m, 4H), 2.81-2.77 (m, 2H), 1.26-2.21 (m, 6H). |
| 63 | 460 | ¹H NMR (400 MHz, DMSO-*D*₆) δ 9.28 (s, 2H), 8.84 - 8.78 (m, 2H), 8.70 (d, *J* = 11.5 Hz, 1H), 8.21 (d, *J* = 5.3 Hz, 1H), 8.13 (s, 1H), 4.31 (s, 1H), 3.90 (s, 2H), 3.32 (s, 1H), 3.15 (t, *J* = 10.9 Hz, 1H), 3.03 (s, 2H), 2.11 (dd, *J =* 14.8, 7.9 Hz, 1H), 1.96 - 1.88 (m, 2H), 1.44 - 1.33 (m, 1H), 1.27 (s, 6H), 0.96 (ddd, *J* = 31.4, 10.4, 5.0 Hz, 2H), 0.86 - 0.70 (m, 2H). |
| 64 | 452 | ¹H NMR (400 MHz, DMSO-*D*₆) δ 9.35 (s, 1H), 9.26 (s, 1H), 9.09 (s, 1H), 8.87 (d, *J=* 8.8 Hz, 1H), 8.77 (d, *J=* 5.3 Hz, 1H), 8.18 (d, *J=* 5.3 Hz, 1H), 8.12 (s, 1H), 5.22 (d, *J =* 45.1 Hz, 1H), 4.48 (dd, *J =* 8.3, 4.1 Hz, 2H), 3.90 (s, 2H), 3.54 (t, *J =* 12.7 Hz, 1H), 3.35 - 3.06 (m, 3H), 3.03 (s, 2H), 2.18 (dd, *J* = 25.6, 12.8 Hz, 1H), 1.26 (s, 6H). |
| 65 | 458 | ¹H NMR (400 MHz, DMSO-*D*₆) δ 9.30 (t, *J =* 10.8 Hz, 1H), 8.91 (s, 1H), 8.76 (t, *J =* 11.9 Hz, 2H), 8.11 (t, *J =* 1.8 Hz, 1H), 7.99 (s, 1H), 7.92 (dt, *J* = 7.5, 1.6 Hz, 1H), 7.63 (s, 1H), 7.49 - 7.40 (m, 2H), 4.29 (s, 1H), 3.86 (s, 2H), 3.38 - 3.30 (m, 1H), 3.13 (q, *J* = 10.8 Hz, 1H), 2.99 (q, *J* = 15.7 Hz, 2H), 2.11 (dt, *J* = 14.8, 7.8 Hz, 1H), 1.93 (dd, *J* = 7.9, 3.9 Hz, 2H), 1.39 (d, *J =* 13.9 Hz, 1H), 1.24 (d, *J =* 2.3 Hz, 6H), 1.03 - 0.94 (m, 1H), 0.90 (dt, *J =* 10.0, 5.1 Hz, 1H), 0.85 - 0.77 (m, 1H), 0.75 - 0.64 (m, 1H). |
| 66 | 476 | ¹H NMR (400 MHz, METHANOL-*D*₄) δ 9.29 (d, *J =* 2.4 Hz, 1H), 9.22 (d, *J =* 7.7 Hz, 1H), 8.65 (dd, *J =* 7.4, 2.4 Hz, 1H), 8.55 (s, 1H), 8.16 (ddd, *J* = 8.6, 4.6, 2.4 Hz, 1H), 7.88 (dd, *J* = 11.1, 8.5 Hz, 1H), 5.10 - 4.94 (m, 1H), 4.58 (s, 2H), 4.19 (ddd, *J =* 12.0, 4.2, 1.4 Hz, 1H), 3.88 (dd, *J* = 12.0, 10.1 Hz, 1H), 3.64 (d, *J =* 1.6 Hz, 2H), 2.91 - 2.75 (m, 2H), 2.71 - 2.58 (m, 1H), 2.28 (dt, *J* = 15.0, 4.4 Hz, 1H), 2.00 (d, *J* = 1.9 Hz, 7H), 1.76 - 1.48 (m, 4H). |

**[Table 6]**

| Example No. | Starting material used |
|---|---|
| 43 | Step 1: 2,4-dibromo-1-fluorobenzene |
| 44 | Step 1: 2,4-dibromo-1-fluorobenzene |
| | Step 4: tert-butyl (3S,5S)-3-amino-5-fluoropiperidine-1-carboxylate |
| 45 | Step 4: tert-butyl (3S,5S)-3-amino-5-fluoropiperidine-1-carboxylate |
| 46 | Step 1: 2,4-dibromo-1-fluorobenzene |
| | Step 4: Intermediate A |
| 47 | Step 1: 2,4-dibromo-1-fluorobenzene |
| | Step 4: tert-butyl trans-3-amino-4-hydroxy-piperidine-1-carboxylate |
| 48 | Step 1: 4-bromo-2-chloropyridine |
| | Step 4: tert-butyl (3S,5S)-3-amino-5-fluoropiperidine-1-carboxylate |
| 49 | Step 1: 4-bromo-2-chloropyridine |
| 50 | Step 1: 4-bromo-2-chloropyridine |
| | Step 4: Intermediate A |
| 51 | Step 4: Intermediate A |
| 52 | Step 4: tert-butyl trans-3-amino-4-hydroxy-piperidine-1-carboxylate |
| 53 | Step 1: 4-bromo-2-chloropyridine |
| | Step 4: tert-butyl trans-3-amino-4-hydroxy-piperidine-1-carboxylate |
| 54 | Step 1: 4-bromo-2-chloropyridine |
| | Step 4: tert-butyl (3R,4R)-3-amino-4-hydroxypyrrolidine-1-carboxylate |
| 55 | Step 1: 2-bromo-4-chlorophenol |
| | Step 4: tert-butyl (3S,5S)-3-amino-5-fluoropiperidine-1-carboxylate |
| 56 | Step 1: 2-bromo-4-chlorophenol |
| | Step 4: Intermediate A |
| 57 | Step 1: 2-bromo-4-chlorophenol |
| | Step 4: tert-butyl (3R,4R)-3-amino-4-hydroxypyrrolidine-1-carboxylate |
| 58 | Step 4: tert-butyl 3-amino-4,4-difluoropiperidine-1-carboxylate |
| 59 | Step 4: tert-butyl (S)-5-amino-3,3-difluoropiperidine-1-carboxylate |
| 60 | Step 4: tert-butyl (3R,4R)-3-amino-4-hydroxypyrrolidine-1-carboxylate |
| 61 | Step 4: tert-butyl 6-amino-1,4-oxazepane-4-carboxylate |
| 62 | Step 4: tert-butyl (3S,4S)-3-amino-4-fluoropyrrolidine-1-carboxylate |
| 63 | Step 1: 2,4-dibromopyrimidine |
| | Step 4: Intermediate A |
| 64 | Step 1: 2,4-dibromopyrimidine |
| | Step 4: tert-butyl (3S,5S)-3-amino-5-fluoropiperidine-1-carboxylate |
| 65 | Step 4: tert-butyl 6-amino-4-azaspiro[2.5]octane-carboxylate |
| 66 | Step 1: 2,4-dibromo-1-fluorobenzene |
| | Step 4: tert-butyl 6-amino-4-azaspiro[2.5]octane-carboxylate |

### Experimental Example 1: Evaluation of Chk1 and Chk2 inhibitory activity of compounds

The compounds of Examples 1 to 66 were diluted at various concentrations and transferred to a 384-well plate by 5 µl, and 10 µl of a mixed solution (2X) of Chk1 kinase (Thermo, P3040) or Chk2 kinase (Thermo, PV3367) and a substrate (Thermo, PV3508) was added to wells treated with the compounds. 5 µl of an ATP solution at a 4X concentration was added to the test well and reacted at room temperature for 1 hour. 20 µl of LanthaScreen Terbium-CREB p-Ser 133 antibody (Thermo, PV3542) was added to the reaction well and incubated at room temperature for 30 minutes. Conversion data was measured by an EnVision 2014 multi label reader (PerkinElmer). The activity for each concentration was determined by the ratio (FRET ratio = FR) of the value obtained by dividing the value at 520 nm by the value at 490 nm among the emission wavelength values for each wavelength (Em520/Em490). The results obtained for each concentration of the compounds were the average values obtained in two wells, and the IC₅₀ values of the compounds were calculated using PRIZM software for analysis of the results. The measured IC₅₀ values are shown in Table 7 below. IC₅₀ in Table 7 was graded as follows.

Grade A: IC₅₀ ≤ 10 nM, Grade B: 100 nM ≥ IC₅₀ > 10 nM, Grade C: 1000 nM ≥ IC₅₀ > 100 nM, Grade D: IC₅₀ > 1000 nM

From the results of Table 7, it was confirmed that the compound of the present invention has excellent inhibitory activity against Chk1 and/or chk2, and particularly selectively inhibits chk2.

**[Table 7]**

| Example No. | Kinase inhibitory activity (IC₅₀) | | Example No. | Kinase inhibitory activity (IC₅₀) | |
|---|---|---|---|---|---|
| | Chk1 | Chk2 | | Chk1 | Chk2 |
| 1 | | B | 34 | B | A |
| 2 | C | B | 35 | D | B |
| 3 | | A | 36 | D | D |
| 4 | D | B | 37 | D | B |
| 5 | | A | 38 | D | D |
| 6 | | A | 39 | C | A |
| 7 | | A | 40 | B | A |
| 8 | | B | 41 | B | A |
| 9 | | B | 42 | B | A |
| 10 | | B | 43 | C | A |
| 11 | | B | 44 | A | B |
| 12 | | D | 45 | C | A |
| 13 | | C | 46 | D | B |
| 14 | C | B | 47 | | B |
| 15 | C | A | 48 | | A |
| 16 | B | A | 49 | | A |
| 17 | B | A | 50 | | A |
| 18 | D | B | 51 | | A |
| 19 | C | B | 52 | | B |
| 20 | B | B | 53 | | B |
| 21 | B | A | 54 | | A |
| 22 | | D | 55 | | A |
| 23 | | A | 56 | | A |
| 24 | | A | 57 | | B |
| 25 | D | B | 58 | | A |
| 26 | C | B | 59 | D | B |
| 27 | B | B | 60 | | A |
| 28 | D | A | 61 | | A |
| 29 | B | A | 62 | | B |
| 30 | B | A | 63 | | A |
| 31 | C | B | 64 | D | A |
| 32 | D | D | 65 | B | B |
| 33 | D | C | 66 | D | B |

### Experimental Example 2: cancer cell growth inhibition test

The human breast cancer cell line MCF-7 was treated with trypsin and harvested. The number of cells was counted, and about 3000 cells / 80 µl per well were inoculated into a 96 well plate (Corning) and then incubated in a 37 °C incubator for 24 hours. Thereafter, the example compounds were diluted in a culture solution at various concentrations, and each well was treated with 20 µl and stored in a cell culture incubator for 72 hours. In the case of the group administered in combination, the example compounds and cisplatin or doxorubicin were diluted in a culture solution at various concentrations, and each well was treated with 20 µl. After 72 hours, each well was treated with 30 µl of CellTiter-Glo solution (Promega), and the luminescence was read on a Victor3 multi label reader (PerkinElmer).

Relative viability (%) = (value of each test well / value of well treated with DMSO alone) X 100

The results obtained for each concentration for each example compound were the average values obtained from three wells, and PRIZM software was used for analysis of the results.

The results of measuring the cell proliferation inhibitory activity of the example compounds in the cell-based assay are shown in Table 8. IC₅₀ in Table 8 was graded as follows.

Grade A: IC₅₀ ≤ 10 µM, Grade B: 100 µM ≥ IC₅₀ > 10 µM, Grade C: IC₅₀ > 100 µM

From the results of Table 8, it was confirmed that the compound of the present invention exhibits excellent cell proliferation inhibitory activity.

**[Table 8]**

| Example No. | Celltiter glo analysis (IC₅₀) | Example No. | Celltiter glo analysis (IC₅₀) |
|---|---|---|---|
| 1 | B | 34 | B |
| 2 | B | 35 | B |
| 3 | B | 36 | B |
| 4 | B | 37 | C |
| 5 | C | 38 | B |
| 6 | B | 39 | B |
| 7 | B | 40 | C |
| 8 | B | 41 | C |
| 9 | B | 42 | B |
| 10 | B | 43 | B |
| 11 | B | 44 | A |
| 12 | A | 45 | B |
| 13 | A | 46 | B |
| 14 | C | 47 | B |
| 15 | A | 48 | B |
| 16 | B | 49 | B |
| 17 | B | 50 | B |
| 18 | B | 51 | B |
| 19 | B | 52 | B |
| 20 | B | 53 | C |
| 21 | B | 54 | C |
| 22 | B | 55 | B |
| 23 | B | 56 | B |
| 24 | B | 57 | C |
| 25 | C | 58 | B |
| 26 | C | 59 | A |
| 27 | C | 60 | C |
| 28 | C | 61 | C |
| 29 | B | 62 | B |
| 30 | C | 63 | B |
| 31 | B | 64 | B |
| 32 | B | 65 | C |
| 33 | B | 66 | A |

On the other hand, the results of measuring the cell proliferation inhibitory activity when administered in combination with cisplatin or doxorubicin are shown in Table 9. As shown in Table 9, the compound of the present invention exhibited synergistic cell proliferation inhibitory activity when administered in combination with cisplatin or doxorubicin.

**[Table 9]**

| Compound | Administered alone (IC₅₀, µM) | Administered in combination with cisplatin (IC₅₀, µM) | Administered in combination with doxorubicin (IC₅₀, µM) |
|---|---|---|---|
| cisplatin | 26 | | |
| doxorubicin | 1.5 | | |
| Example 4 | 48.1 | 3.44 | 0.67 |
| Example 13 | 8.59 | 2.93 | 0.91 |
| Example 15 | 11.8 | 2.27 | 3.05 |
| Example 32 | 33.1 | 4.58 | 0.91 |

### Experimental Example 3: Pharmacokinetics test using mice

6 to 8 week-old male Balb/C mice weighing 22-29 g were acclimatized for about 1 week, and then 12 mice for each group were divided into an intravenous administration group and an oral administration group and used in the experiment. The mice were fasted before administration of the compound and were allowed to drink water ad libitum. Feed was provided 2 hours after administration of the compound.

A preparation for intravenous injection (0.2 mg/mL) was prepared by dissolving 1.264 mg of the compound of Example 9 in 6.257 mL of DMSO : a mixed solution of solutol and ethanol (1:1) : physiological saline (5:5:90 %v/v). A preparation for oral administration (1 mg/mL) was prepared by suspending 5.916 mg of the compound of Example 9 in 5.857 mL of 0.5% methyl cellulose (0.5:99.5 %v/v) containing 0.1 Tween 80.

The compound of Example 9 was administered at a dose of 1 mg/kg for intravenous administration and at a dose of 10 mg/kg for oral administration using an oral zonde.

In the case of intravenous administration, blood was collected at 5 minutes, 15 minutes, 30 minutes, 1 hour, 2 hours, 4 hours, 8 hours and 24 hours after administration of the compound. In the case of oral administration, blood was collected at 15 minutes, 30 minutes, 1 hour, 2 hours, 4 hours, 8 hours, 10 hours and 24 hours after administration of the compound. Plasma was separated from the collected blood, and the content of compounds in blood was measured by LC-MS/MS method, and PK parameters were calculated using Phoenix software ver.8.1.

The amount of change over time in the average blood concentration of the compound is shown in Fig. 1, and the calculated PK parameters are shown in Table 10 below.

**[Table 10]**

| **PK parameter** | | **IV (1 mg/kg)** | **PO (10 mg/kg)** |
|---|---|---|---|
| t_{1/2} | (h) | 1.36 | 1.92 |
| C_{0/}Cₘₐₓ | (ng/mL) | 231 | 748.6 |
| AUC₀₋ₜ | (ng·h/mL) | 267 | 1731 |
| AUC_{0-∞} | (ng·h/mL) | 298 | 1767 |
| CL | *(mL*/*min*/*kg)* | 56.2 | - |
| V_{d} | (L/kg) | 6.59 | - |
| Vₛₛ | (L/kg) | 5.22 | - |
| Tₘₐₓ | (h) | - | 0.5 |
| Time points considered for terminal slope calculation | | 1- 4 h | 4-10 h |
| Oral bioavailability (%F) | | | 59 |

In the above description, for the purpose of explanation, numerous specific details have been set forth in order to provide a thorough understanding of the embodiments. However, it will be apparent to one of ordinary skill in the art that one or more other embodiments can be practiced without some of these specific details. It should also be understood that throughout the present specification, references to "one embodiment," "an embodiment," an embodiment having an ordinal designation, or the like, mean that a particular feature, structure, or property may be included in the practice of the present invention. It is further understood that in the present invention, various features are sometimes grouped together in a single embodiment, drawing, or description thereof for the purpose of simplifying the description and facilitating understanding of various inventive aspects, and one or more features or specific details from one embodiment can be practiced along with one or more features or specific details from other embodiments where appropriate in the practice of the present invention.

Although the present invention has been described with respect to what are considered exemplary embodiments, the present invention is not limited to the described embodiments, but is intended to cover all modifications, alternatives and equivalents that may be included within the scope of the present invention as set forth in the claims.

## Claims

1. A compound of Formula A below, or a stereoisomer, hydrate, solvate or pharmaceutically acceptable salt thereof: 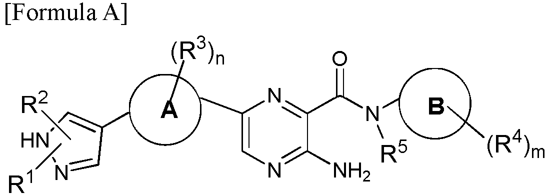 in which,
ring A is a C₆-C₁₀ aryl or a 5 to 10-membered heteroaryl containing 1 to 3 nitrogen atoms;
ring B is a C₃-C₁₀ monocyclic or bicyclic cycloalkyl or a monocyclic or bicyclic 3 to 10-membered heterocycloalkyl, wherein the heterocycloalkyl contains 1 to 3 heteroatoms selected from N, O and S;
R¹ and R² are each independently halogen, -OH, -CN, amino, nitro, oxo, C₁-C₈ alkyl, C₁-C₈ alkoxy, a straight chain or branched chain C₁-C₈ alkyl optionally substituted with one or more R', or a C₃-C₈ cycloalkyl optionally substituted with one or more R', or
when R¹ and R² are attached to adjacent ring elements, they may be taken together with the carbon atom and the nitrogen atom to which they are attached to form a pyrrolidine ring, wherein the pyrrolidine ring is optionally substituted with one or more R';
R' is one or more substituents independently selected from the group consisting of halogen, -OH, -CN, amino, nitro, oxo, C₁-C₈ alkyl and C₁-C₈ alkoxy;
R³ is selected from the group consisting of halogen, -OH, -CN, amino, nitro, oxo, C₁-C₈ alkyl, C₃-C₈ cycloalkyl and C₁-C₈ alkoxy;
R⁴ is selected from the group consisting of halogen, -OH, -CN, amino, nitro, oxo, C₁-C₈ alkyl, C₁-C₈ alkoxy, -CO-(C₁-C₈ alkyl), -CO-(C₂-C₈ alkenyl), -COOH, and -COO-C₁-C₈ alkyl, wherein said C₁-C₈ alkyl, C₁-C₈ alkoxy and C₂-C₈ alkenyl may be optionally substituted with one or more halogen, -OH or -CN;
R⁵ is H or a straight chain or branched chain C₁-C₈ alkyl;
n is an integer from 0 to 2, and wherein when n is 2, R³ may each be bound to the same or different ring atoms; and
m is an integer from 0 to 3, and wherein when m is 2 or more, R⁴ may each be bound to the same or different ring atoms.

2. The compound, or stereoisomer, hydrate, solvate or pharmaceutically acceptable salt thereof according to claim 1, wherein ring A is phenyl, pyridinyl or pyrimidinyl.

3. The compound, or stereoisomer, hydrate, solvate or pharmaceutically acceptable salt thereof according to claim 1, wherein ring B is a monocyclic or spirocyclic 3 to 10-membered heterocycloalkyl.

4. The compound, or stereoisomer, hydrate, solvate or pharmaceutically acceptable salt thereof according to claim 3, wherein the monocyclic or spirocyclic 3 to 10-membered heterocycloalkyl optionally contains, in addition to one N, 1 or 2 heteroatoms selected from N, O and S.

5. The compound, or stereoisomer, hydrate, solvate or pharmaceutically acceptable salt thereof according to claim 1, wherein the compound of Formula A is a compound of Formula I below: 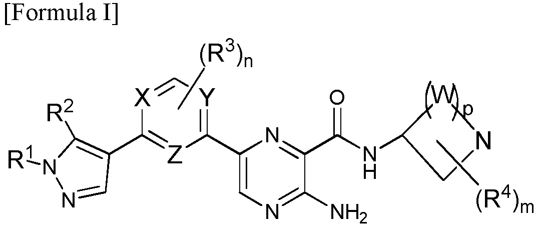 in which,
X, Y and Z are each independently C or N;
R¹ is a straight chain or branched chain C₁-C₈ alkyl optionally substituted with one or more R' or a C₃-C₈ cycloalkyl optionally substituted with one or more R'; and
R² is H or a straight chain or branched chain C₁-C₈ alkyl; or
R¹ and R² may be taken together with the nitrogen atom and the carbon atom to which they are each attached to form a pyrrolidine ring, wherein the pyrrolidine ring is optionally substituted with one or more R';
R' is one or more substituents independently selected from the group consisting of halogen, -OH, -CN, amino, nitro, oxo, C₁-C₈ alkyl and C₁-C₈ alkoxy;
R³ is selected from the group consisting of halogen, -OH, -CN, amino, nitro, oxo, C₁-C₈ alkyl, C₃-C₈ cycloalkyl and C₁-C₈ alkoxy;
R⁴ is selected from the group consisting of halogen, -OH, -CN, amino, nitro, oxo, C₁-C₈ alkyl, C₁-C₈ alkoxy, -CO-C₁-C₈ alkyl, -CO-C₂-C₈ alkenyl, -COOH, and -COO-C₁-C₈ alkyl, wherein said C₁-C₈ alkyl, C₁-C₈ alkoxy and C₂-C₈ alkenyl may be optionally substituted with one or more halogen, -OH or -CN;
n is an integer from 0 to 2, and wherein when n is 2, two R³ may each be bound to the same or different ring atoms;
m is an integer from 0 to 2, and wherein when m is 2, two R⁴ may each be bound to the same or different ring atoms;
p is an integer from 1 to 5; and
W is C, and wherein when p is 2 or more, one of W may be optionally replaced by a heteroatom selected from N, O or S, or one of W may be taken together with an additional carbon atom to form a C₃-C₅ cycloalkyl ring.

6. The compound, or stereoisomer, hydrate, solvate or pharmaceutically acceptable salt thereof according to claim 5, wherein
X and Z are N, and Y is C;
Y and Z are N, and X is C;
X is N, and Y and Z are C;
Y is N, and X and Z are C; or
X, Y and Z are all C.

7. The compound, or stereoisomer, hydrate, solvate or pharmaceutically acceptable salt thereof according to claim 5, wherein
R¹ is a straight chain or branched chain C₁-C₅ alkyl optionally substituted with one or more halogen, -OH or -CN, or a C₃-C₅ cycloalkyl optionally substituted with one or more halogen, -OH or -CN; and
R² is H.

8. The compound, or stereoisomer, hydrate, solvate or pharmaceutically acceptable salt thereof according to claim 7, wherein
R¹ is -CH₂-CF₃, -CH₂-CN, -CH₃, -CH₂-C(CH₃)₂OH, or cyclopropyl; and
R² is H.

9. The compound, or stereoisomer, hydrate, solvate or pharmaceutically acceptable salt thereof according to claim 5, wherein
R¹ and R² are taken together with the nitrogen atom and the carbon atom to which they are each attached to form a pyrrolidine ring, wherein the pyrrolidine ring is optionally substituted with one or more C₁-C₅ alkyl, or 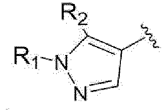 has a structure of

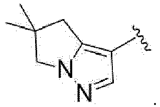

10. The compound, or stereoisomer, hydrate, solvate or pharmaceutically acceptable salt thereof according to claim 5, wherein
R³ is selected from the group consisting of halogen, -OH, -CN, C₁-C₅ alkyl and C₁-C₅ alkoxy; and
R⁴ is selected from the group consisting of halogen, -OH, -CN, C₁-C₅ alkyl and C₁-C₅ alkoxy.

11. The compound, or stereoisomer, hydrate, solvate or pharmaceutically acceptable salt thereof according to claim 5, wherein
p is an integer from 2 to 4; and
W is all C, or one of W is replaced by O, or one of W is taken together with an additional carbon atom to form a cyclopropyl ring.

12. The compound, or stereoisomer, hydrate, solvate or pharmaceutically acceptable salt thereof according to claim 11, wherein 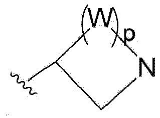 has a structure selected from: 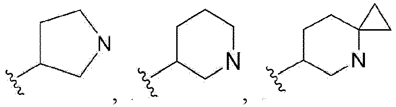 and

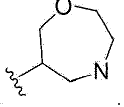

13. The compound, or stereoisomer, hydrate, solvate or pharmaceutically acceptable salt thereof according to claim 1, wherein the compound is any one selected from the following compounds:

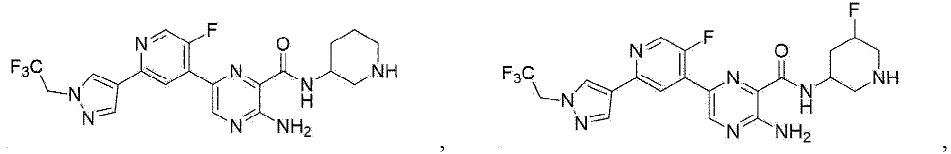

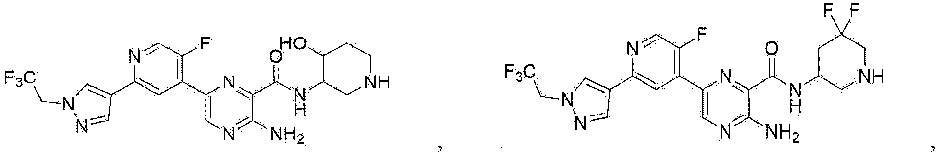

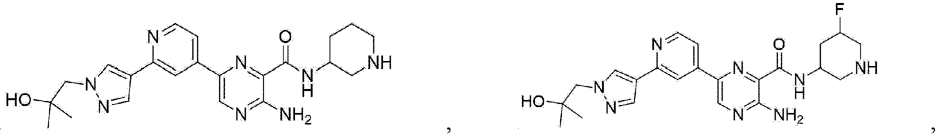

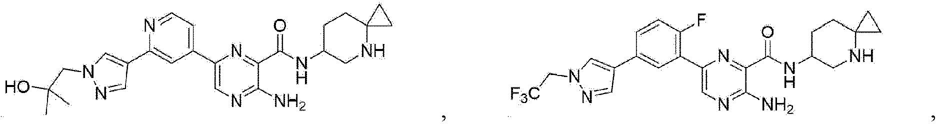

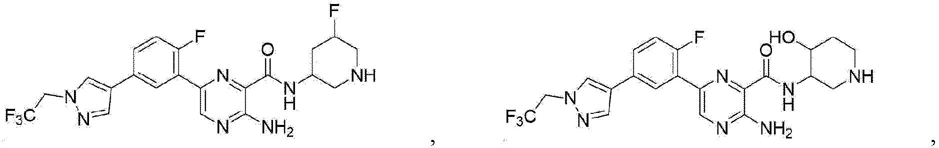

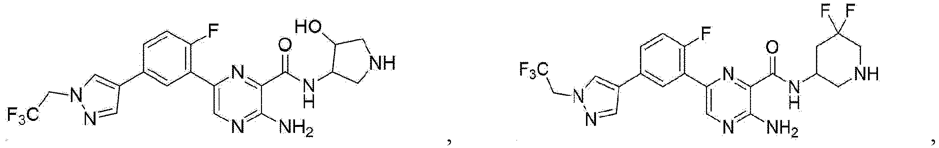

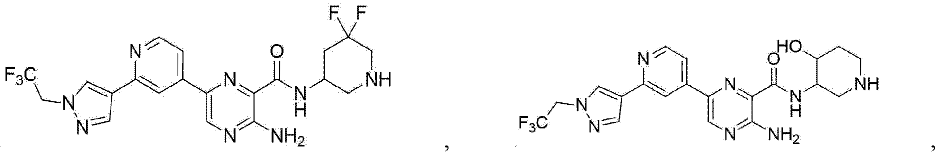

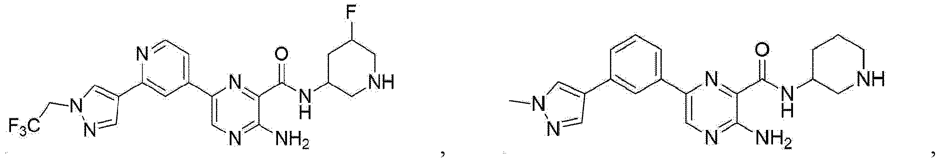

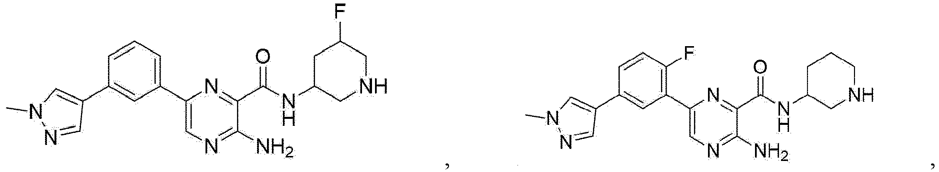

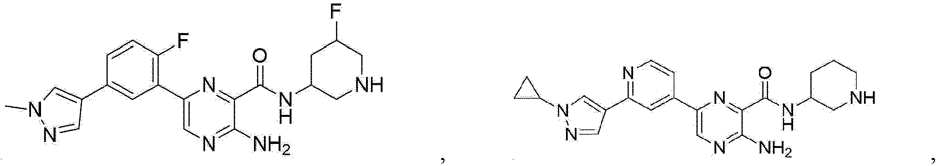

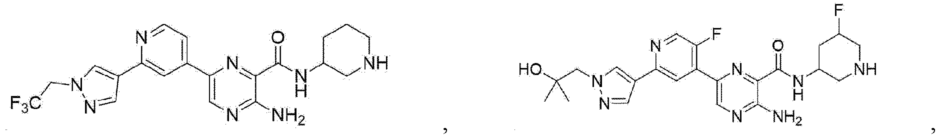

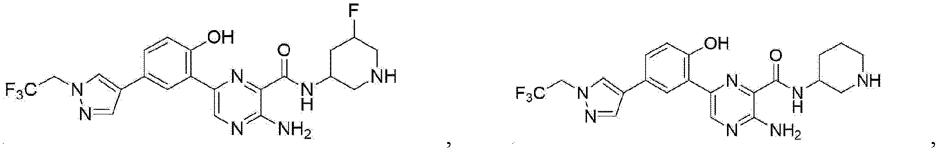

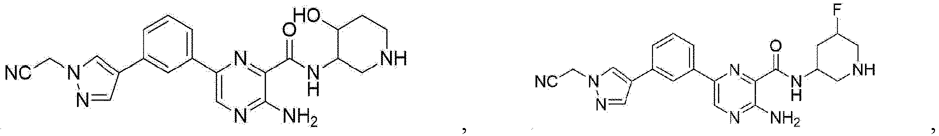

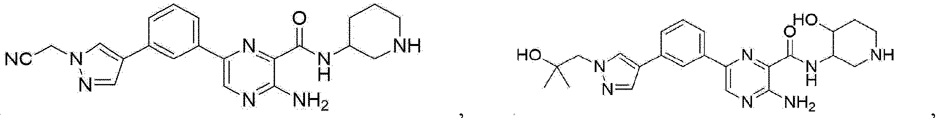

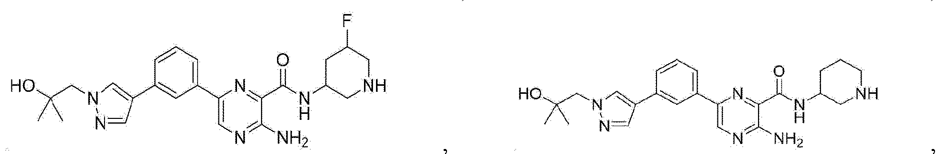

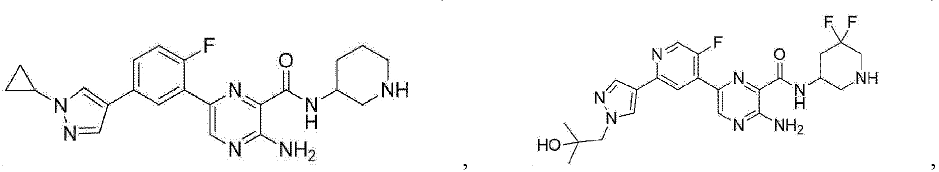

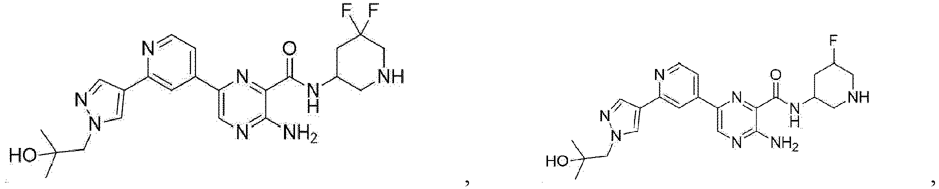

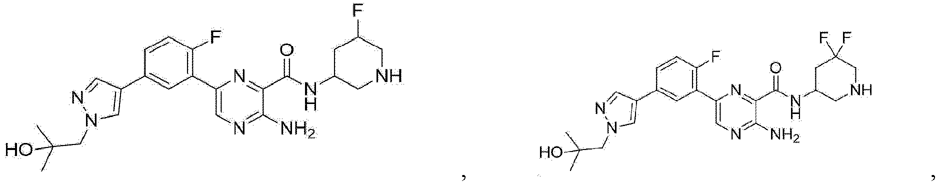

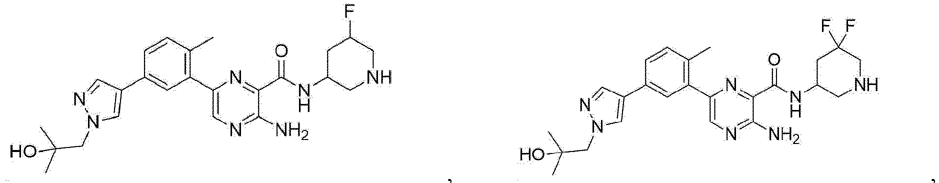

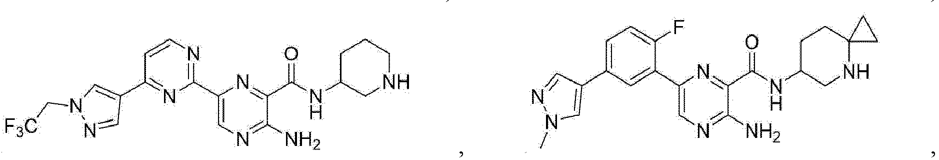

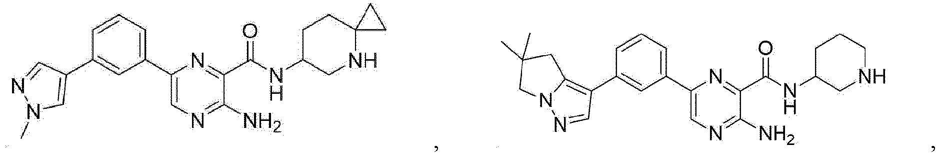

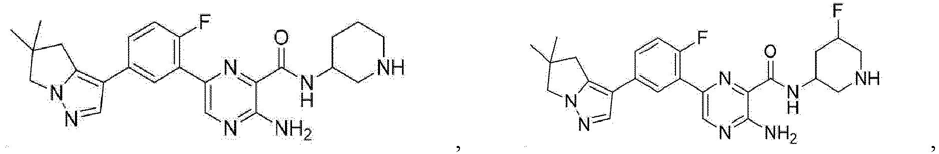

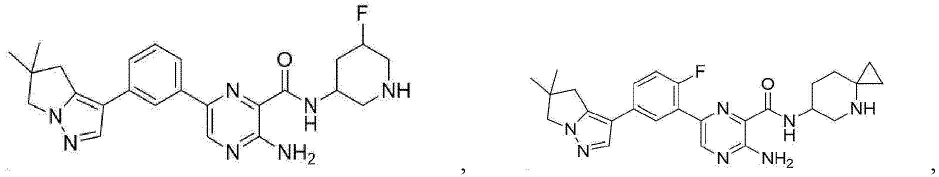

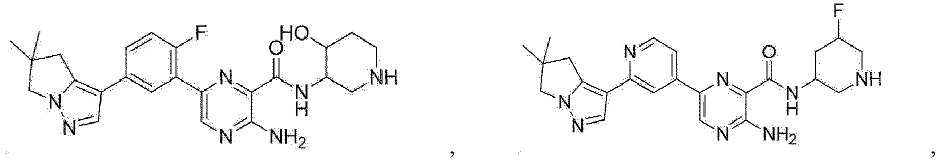

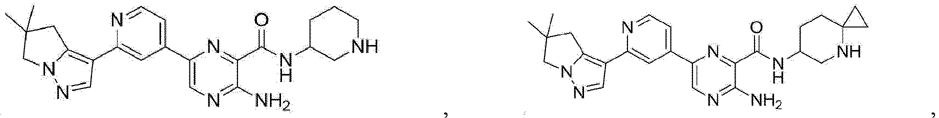

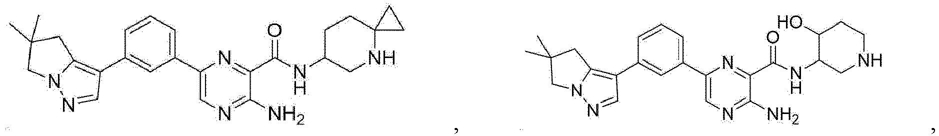

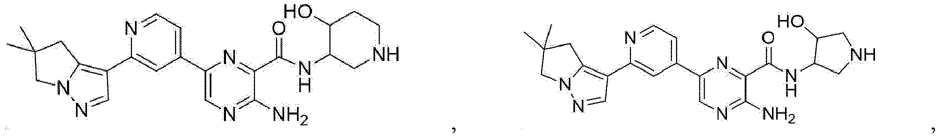

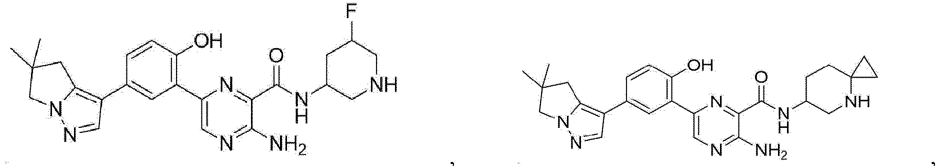

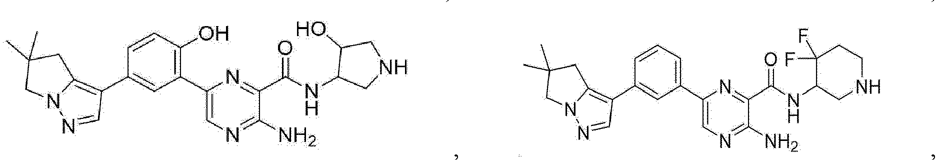

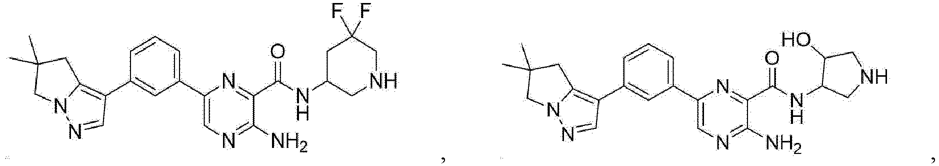

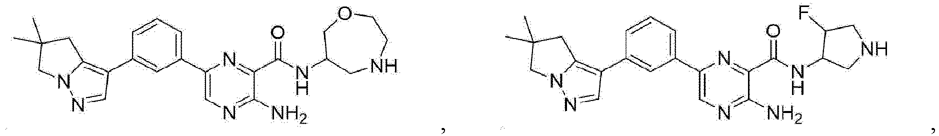

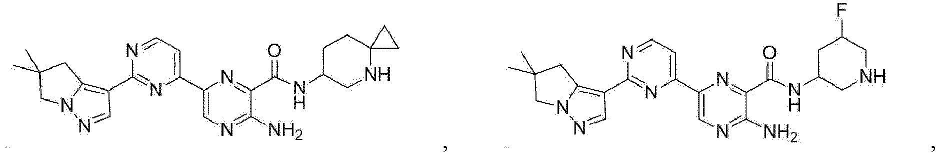

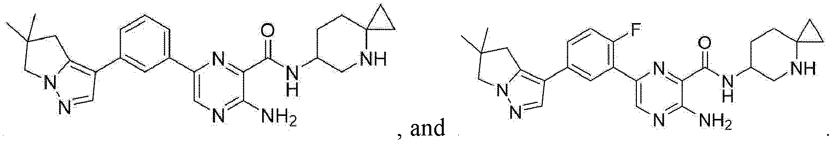

14. A pharmaceutical composition for preventing or treating a disease mediated by checkpoint kinase 2 activation, comprising the compound, or stereoisomer, hydrate, solvate or pharmaceutically acceptable salt thereof according to any one of claims 1 to 13 as an active ingredient.

15. The pharmaceutical composition according to claim 14, wherein the disease mediated by checkpoint kinase 2 activation is cancer.

16. The pharmaceutical composition according to claim 15, wherein the cancer is selected from the group consisting of ovarian cancer, cervical cancer, endometrial cancer, uterine sarcoma, vulvar cancer, breast cancer, skin cancer, head and neck cancer, pancreatic cancer, lung cancer, large intestine cancer, colorectal cancer, gastric cancer, prostate cancer, bladder cancer, urethral cancer, liver cancer, kidney cancer, skin cancer, cerebrospinal tumor, brain cancer, thymoma, mesothelioma, bronchial cancer, nasopharyngeal cancer, laryngeal cancer, esophageal cancer, biliary tract cancer, testicular cancer, germ cell tumor, thyroid cancer, parathyroid cancer, lymphoma, myelodysplastic syndrome (MDS), myelofibrosis, acute myeloid leukemia (AML), acute lymphocytic leukemia (ALL), chronic myeloid leukemia (CML), chronic lymphocytic leukemia (CLL), multiple myeloma, endocrine cancer, sarcoma, ataxia telangiectasia, childhood neuroblastoma and Li-Fraumeni syndrome.

17. The pharmaceutical composition according to claim 15, wherein the pharmaceutical composition is administered simultaneously or sequentially with at least one agent having anticancer activity.

18. The pharmaceutical composition according to claim 17, wherein the agent having anticancer activity is a DNA damaging agent selected from radiation, cisplatin, oxaliplatin, carboplatin, adriamycin or doxorubicin, daunorubicin, irinotecan, gemcitabine, temozolomide, capecitabine, topotecan, camptothecin, cytarabine, 5-fluorouracil, cyclophosphamide, etoposide or etoposide phosphate, teniposide, daunorubicin and pemetrexed.

19. The pharmaceutical composition according to claim 17, wherein the agent having anticancer activity is a PARP inhibitor, or an agent targeting a protein selected from the group consisting of EGFR, VEGFR, CD20, CD38, RNAK-L, BTK, Bcr-abl, PDGFR/FGFR, MEK/RAF/KRAS, HER2/Neu, ubiquitin, JAK, ALK, TGFβRI, proteasome, Bcl-2, C-Met, VR1, VR2, VR3, c-kit, AXL, RET, Braf, DNMT, CDK4/6 and STING; an immune checkpoint inhibitor; a cell therapeutic agent; an antibody therapeutic agent; or an anticancer virus therapeutic agent.

20. A method for preventing or treating cancer, comprising administering the compound, or stereoisomer, hydrate, solvate or pharmaceutically acceptable salt thereof according to any one of claims 1 to 13 to a subject.

21. A method of inhibiting checkpoint kinase 2 activation, comprising administering the compound, or stereoisomer, hydrate, solvate or pharmaceutically acceptable salt thereof according to any one of claims 1 to 13 to a subject.

22. A use of the compound, or stereoisomer, hydrate, solvate or pharmaceutically acceptable salt thereof according to any one of claims 1 to 13 for the manufacture of a medicament for preventing or treating cancer.
